# EUROPÄISCHE PATENTSCHRIFT

(11) **EP 3 033 405 B1**
(45) Veröffentlichungstag und Bekanntmachung des Hinweises auf die Patenterteilung: **09.05.2018**
(21) Anmeldenummer: 14742471.7
(22) Anmeldetag: 24.07.2014
(51) Int. Cl.: C09K 11/06, H05B 33/20, H01L 51/54, H01L 51/50

(54) **MATERIALIEN FÜR ELEKTRONISCHE VORRICHTUNGEN**
MATERIALS FOR ELECTRONIC DEVICES
MATÉRIAUX DESTINÉS À DES DISPOSITIFS ÉLECTRONIQUES

(30) Priorität: 15.08.2013 EP 13004061
(43) Veröffentlichungstag der Anmeldung: 22.06.2016
(73) Patentinhaber: Merck Patent GmbH, 64293 Darmstadt (DE)
(72) Erfinder: PFISTER, Jochen, 64665 Alsbach-Haehnlein (DE); VOGES, Frank, 67098 Bad Duerkheim (DE); MONTENEGRO, Elvira, 69469 Weinheim (DE); MUJICA-FERNAUD, Teresa, 64283 Darmstadt (DE)
(86) Internationale Anmeldenummer: PCT/EP2014/002031
(87) Internationale Veröffentlichungsnummer: WO 2015/022051

(56) Entgegenhaltungen:
- WO-A1-2006/108497
- WO-A1-2006/122630
- WO-A1-2013/100467
- WO-A1-2014/129846

## Beschreibung

Die vorliegende Anmeldung betrifft eine Verbindung mit Spirobifluoren-Grundgerüst und daran ankondensierter Benzofluoren-Einheit. Die Verbindung eignet sich zur Verwendung in elektronischen Vorrichtungen, insbesondere in organischen Elektrolumineszenzvorrichtungen (OLEDs).

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs verstanden.

Der Aufbau von OLEDs, in denen organische Verbindungen als funktionelle Materialien eingesetzt werden, ist beispielsweise in US 4539507, US 5151629, EP 0676461 und WO 98/27136 beschrieben. Allgemein werden unter der Bezeichnung OLEDs elektronische Vorrichtungen verstanden, welche eine oder mehrere Schichten enthaltend organische Verbindungen aufweisen und unter Anlegen von elektrischer Spannung Licht emittieren.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Schichten mit lochtransportierender Funktion, wie beispielsweise lochinjizierende Schichten, Lochtransportschichten, Elektronenblockierschichten und auch emittierende Schichten. Zur Verwendung in diesen Schichten werden kontinuierlich neue Materialien mit lochtransportierenden Eigenschaften gesucht.

Es ist im Stand der Technik bekannt, Triarylamine als Materialien mit lochtransportierenden Eigenschaften in den oben genannten Schichten einzusetzen. Diese können Mono-Triarylamine darstellen, wie beispielsweise in JP 1995/053955, WO 2006/123667 und JP 2010/222268 beschrieben, oder Bis- oder andere Oligoamine darstellen, wie beispielsweise in US 7504163 oder US 2005/0184657 beschrieben. Bekannte Beispiele für Triarylamin-Verbindungen als Materialien mit lochtransportierenden Eigenschaften für OLEDs sind unter anderem Tris-p-biphenyl-amin, N,N'-Di-1-naphthyl-N,N'-diphenyl-1,1'-biphenyl-4,4'-diamin (NPB) und 4,4',4"-Tris-(3-methylphenylphenylamino)triphenylamin (MTDATA).
Weiterhin im Stand der Technik bekannt ist die Verwendung von Spirobifluoren-Arylaminoverbindungen in OLEDs, unter anderem als Lochtransportmaterialien (WO 2012/034627 und WO 2013/120577). Weiterhin bekannt für diese Verwendung sind Spirobifluoren-Derivate, die eine Benzofuran-Einheit ankondensiert an das Spirobifluoren-Grundgerüst aufweisen, und die eine oder mehrere Arylaminogruppen in 2-Position am Spirobifluoren gebunden aufweisen (WO 2013/100467).
Im Rahmen von Untersuchungen zu neuartigen Materialien zur Verwendung in OLEDs wurde nun überraschend gefunden, dass sich Verbindungen, die eine Benzofuran-Einheit ankondensiert an ein Spirobifluoren-Grundgerüst aufweisen, und eine in bestimmter Position am Spirobifluoren gebundene Arylamin- oder Carbazolgruppe aufweisen, hervorragend zur Verwendung in OLEDs eignen, insbesondere als Materialien mit lochtransportierender Funktion.

Die gefundenen Verbindungen weisen eine oder mehrere Eigenschaften gewählt aus sehr guten lochleitenden Eigenschaften, sehr guten elektronenblockierenden Eigenschaften, hoher Glasübergangstemperatur, hoher Oxidationsstabilität, guter Löslichkeit und hoher Temperaturstabilität auf.
Gegenstand der vorliegenden Erfindung ist daher eine Verbindung der Formel (I-1) - (I-6) gemäß Anspruch 1.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von den oben unter Aryl- und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Es ist bevorzugt, dass A eine Gruppe der Formel (A-1) oder (A-3) ist, besonders bevorzugt eine Gruppe der Formel (A-1).

Es ist bevorzugt, dass Ar¹ bei jedem Auftreten gleich oder verschieden gewählt ist aus einer Einfachbindung oder einer divalenten Gruppe gewählt aus wahlweise mit Resten R² substituiertem Benzol, Biphenyl, Terphenyl, Fluoren, Spirobifluoren, Indenofluoren, Carbazol, Dibenzofuran, Dibenzothiophen oder einer Kombination von zwei oder mehr dieser Gruppen, wobei jedoch nicht mehr als 30 aromatische Ringatome in Ar¹ vorliegen dürfen.

Gruppen Ar¹ sind bevorzugt gewählt aus Gruppen der folgenden Formeln: 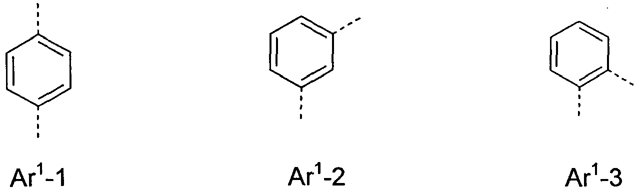 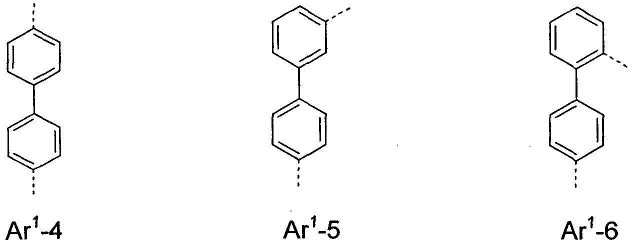 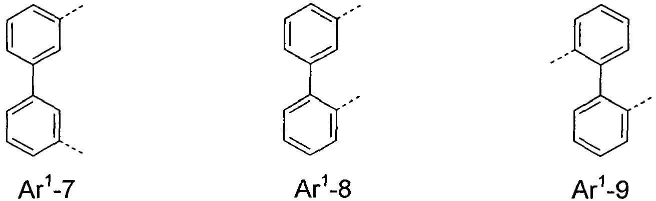 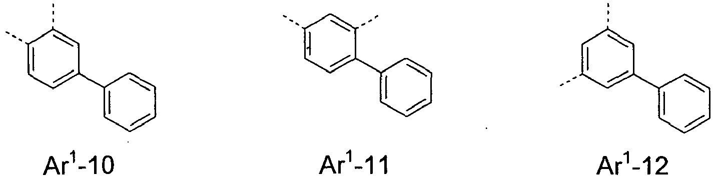 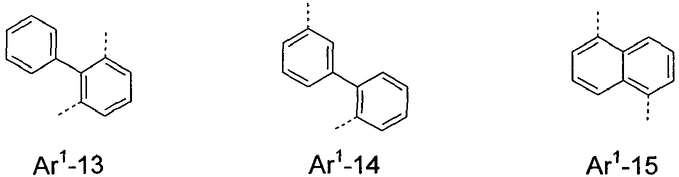 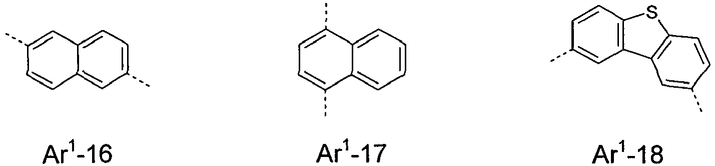 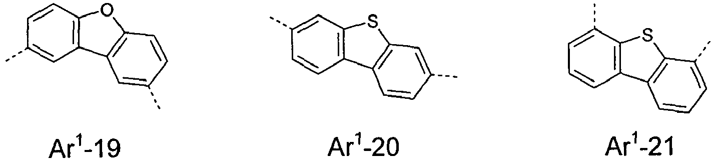 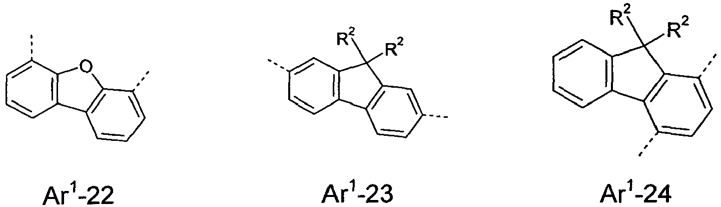 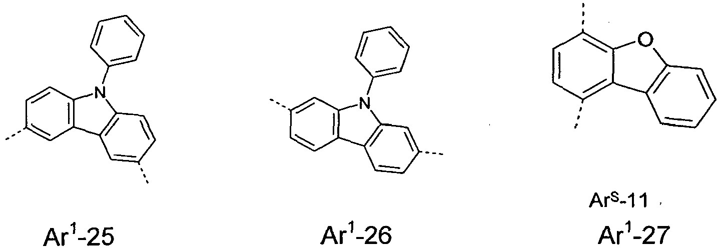 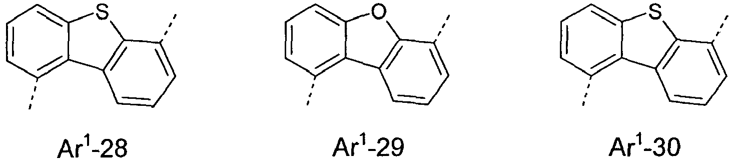 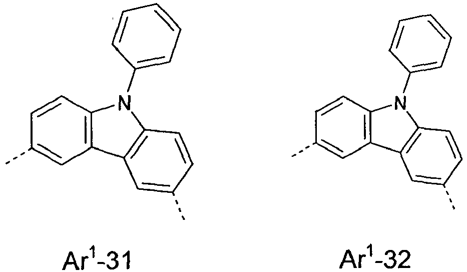 wobei die gestrichelten Bindungen die Bindungen an den Rest der Formel darstellen und die Gruppen an den freien Positionen durch einen oder mehrere Reste R² substituiert sein können, bevorzugt an den freien Positionen aber unsubstituiert sind.

Dabei steht R² in den Gruppen der Formeln (Ar¹-23) und (Ar¹-24) bevorzugt gleich oder verschieden für eine Alkylgruppe mit 1 bis 10 C-Atomen, insbesondere für Methyl, oder eine Phenylgruppe, die mit einem oder mehreren Resten R³ substituiert sein kann und bevorzugt unsubstituiert ist. Zwei Alkylgruppen R² können dabei auch unter Ausbildung einer Spiro-Gruppe einen Ring bilden, bevorzugt einen Cyclohexylring oder einen Cyclopentylring.

Es ist bevorzugt, dass Ar² bei jedem Auftreten gleich oder verschieden gewählt ist aus einem aromatischen oder heteroaromatischen Ringsystem mit 6 bis 25 aromatischen Ringatomen, das mit einem oder mehreren Resten R² substituiert sein kann. Besonders bevorzugt sind Phenyl, Biphenyl, Terphenyl, Fluorenyl, Spirobifluorenyl, Indenofluorenyl, Naphthyl, Phenanthrenyl, Furanyl, Benzofuranyl, Dibenzofuranyl, Thiophenyl, Benzothiophenyl, Dibenzothiophenyl, Carbazolyl, Indolocarbazolyl und Indenocarbazolyl, welche mit einem oder mehreren Resten R² substituiert sein können.

Gruppen Ar² sind bevorzugt gleich oder verschieden bei jedem Auftreten gewählt aus Gruppen der folgenden Formeln: 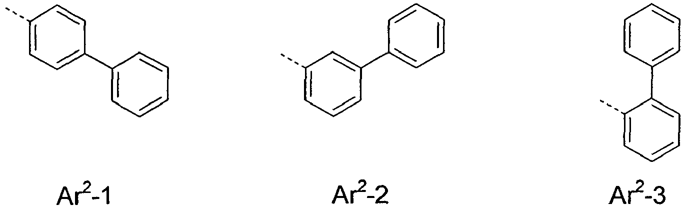 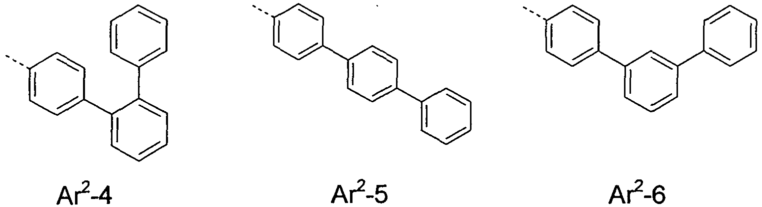 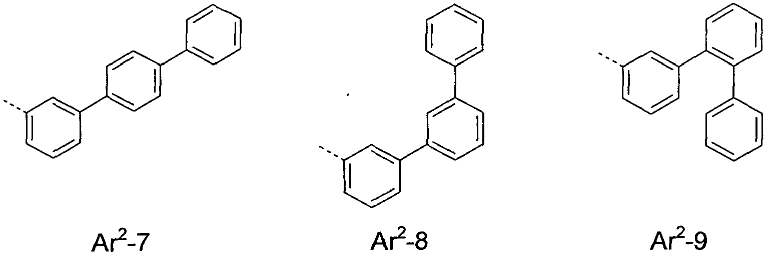 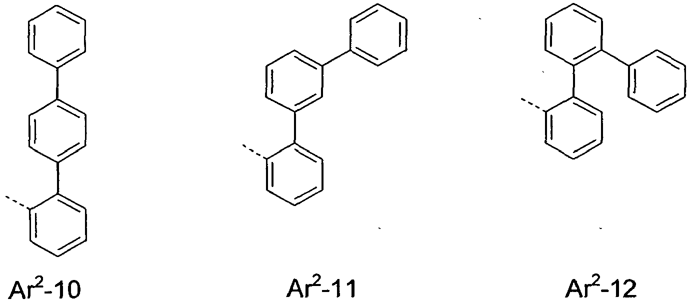 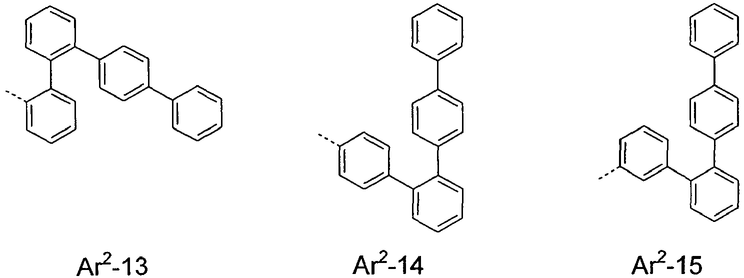 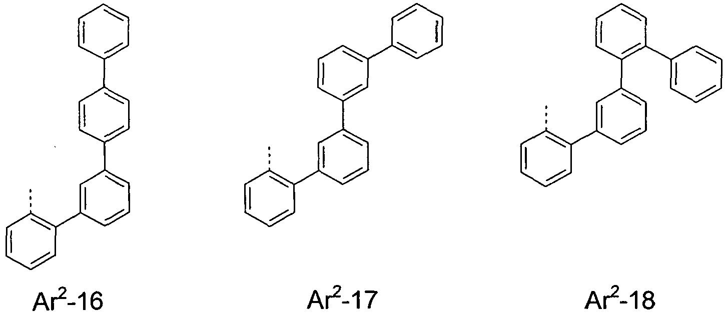 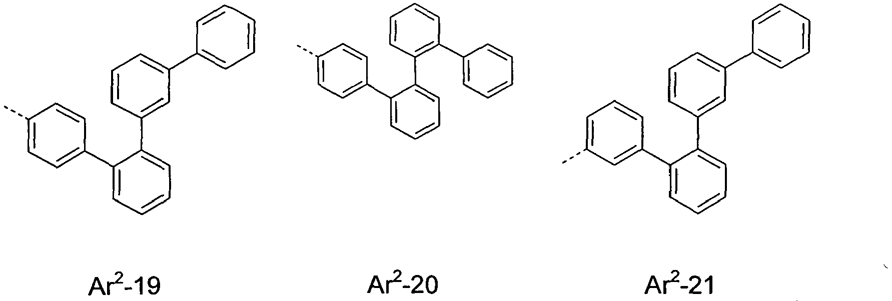 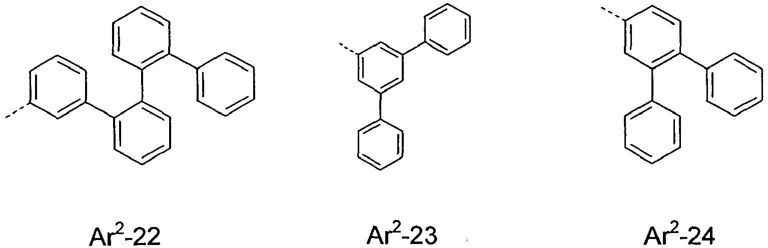 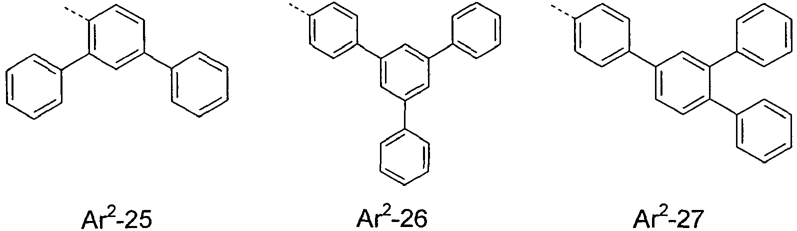 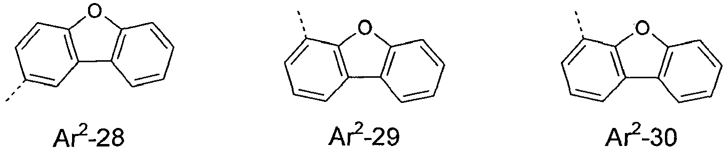 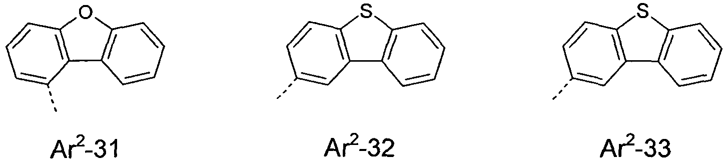 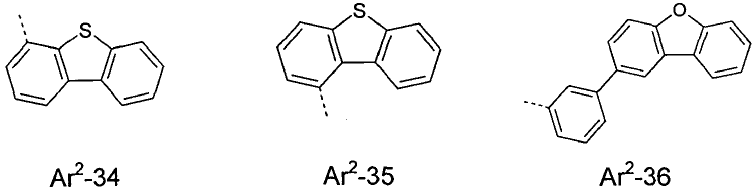 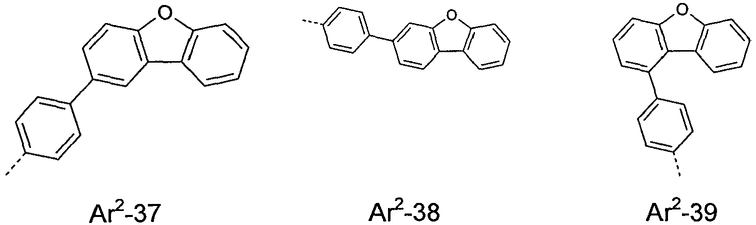 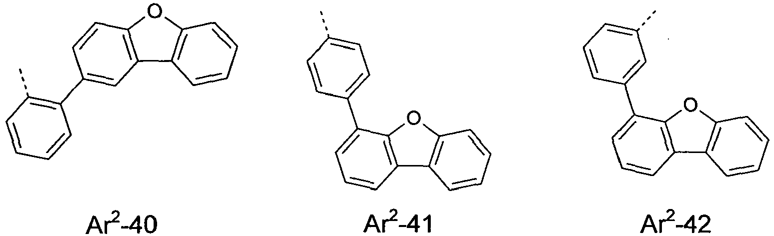 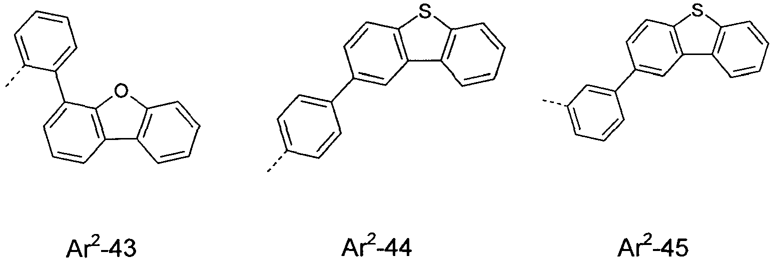 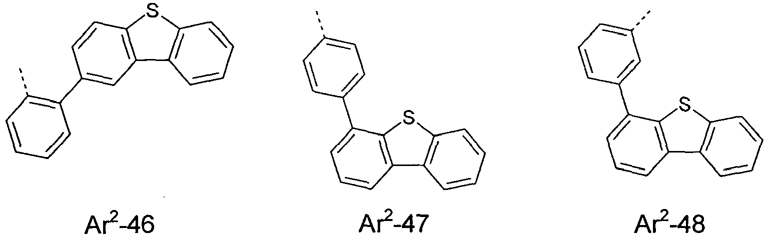 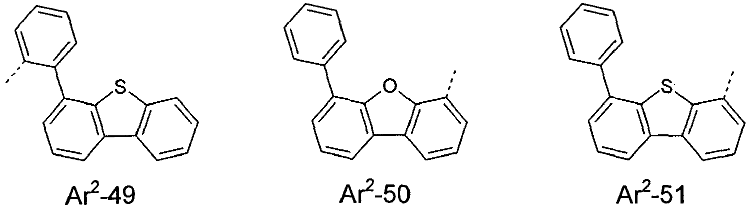 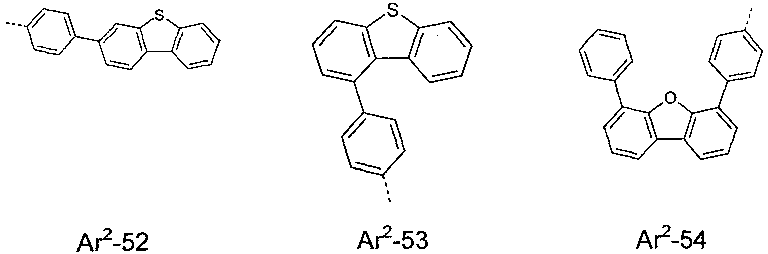 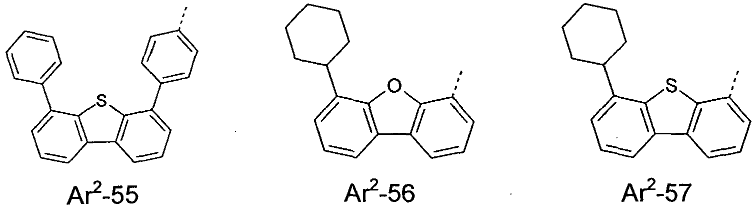 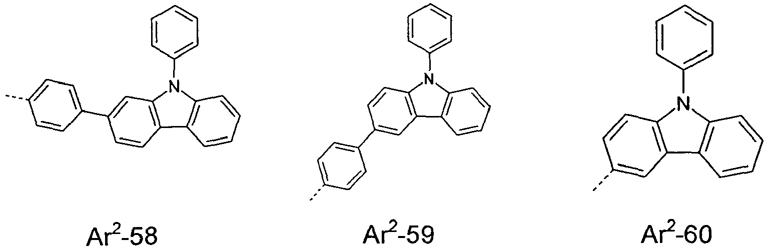 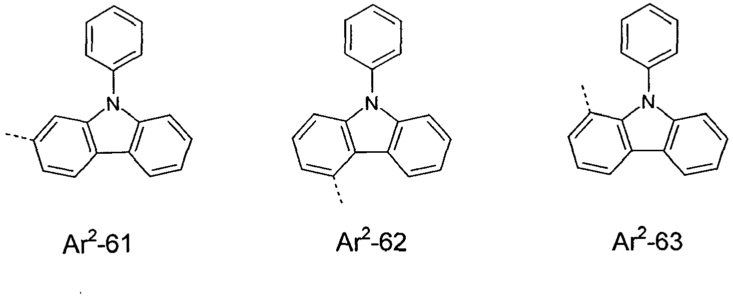 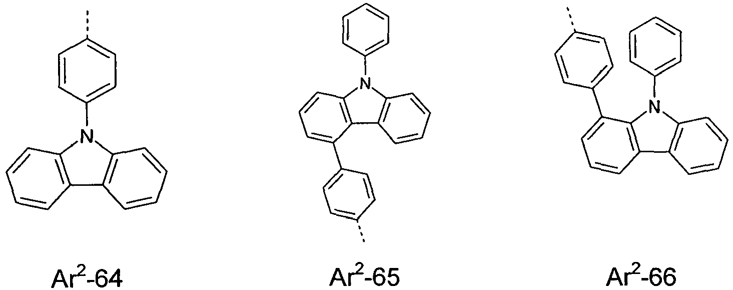 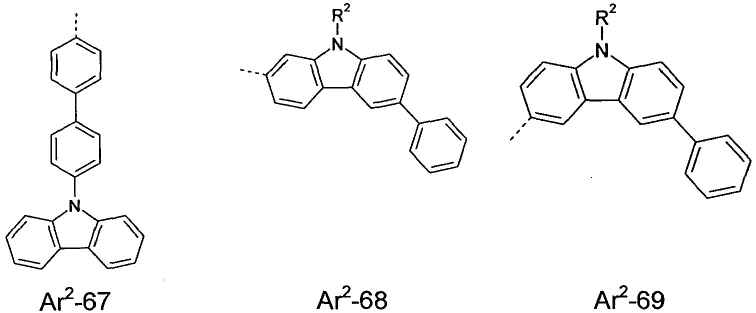 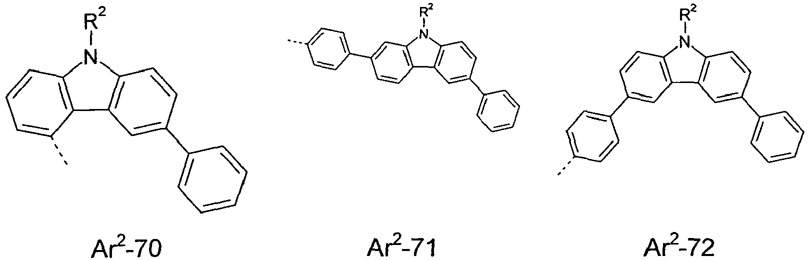 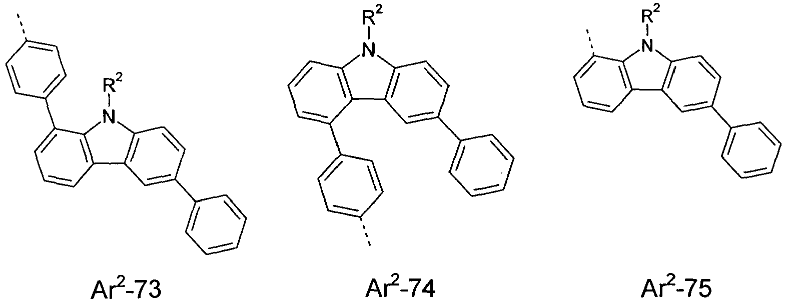 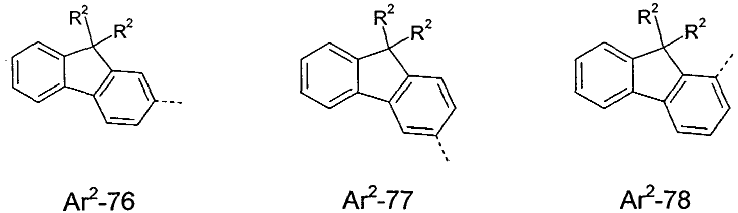 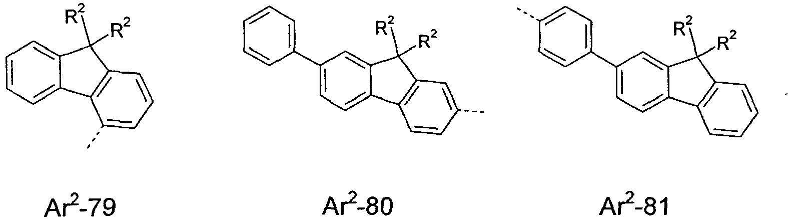 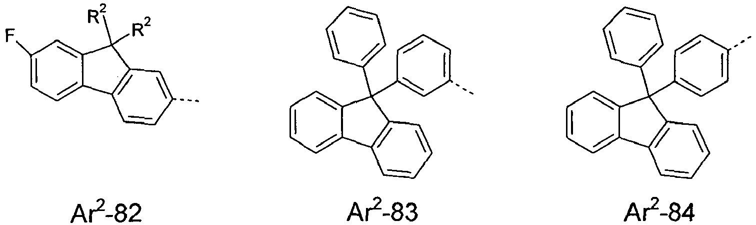 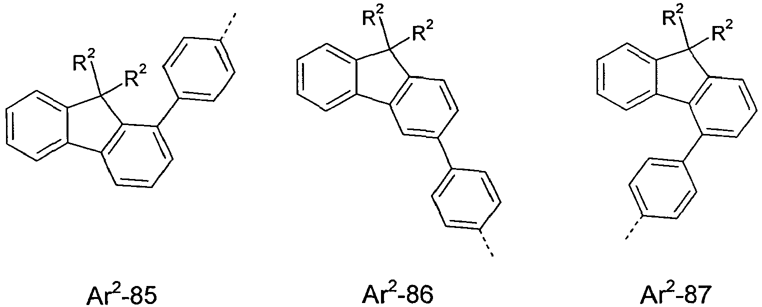 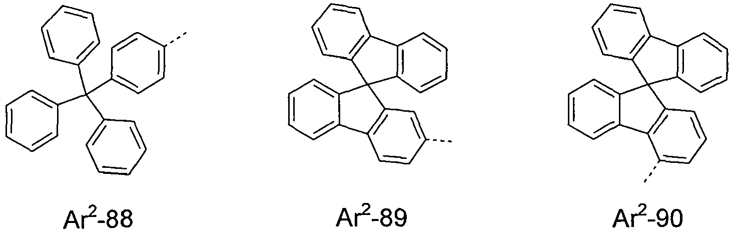 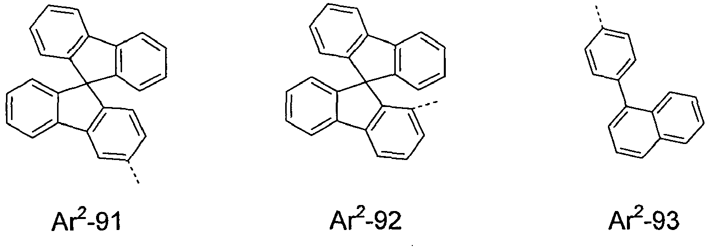 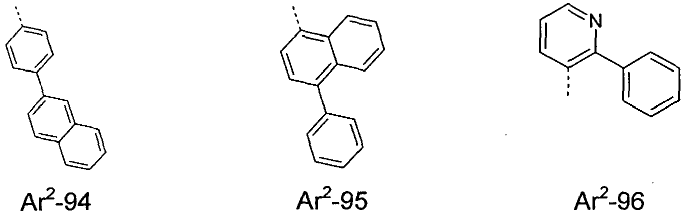 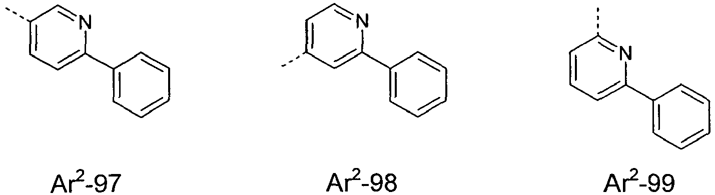 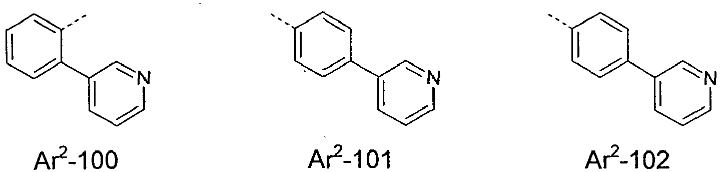 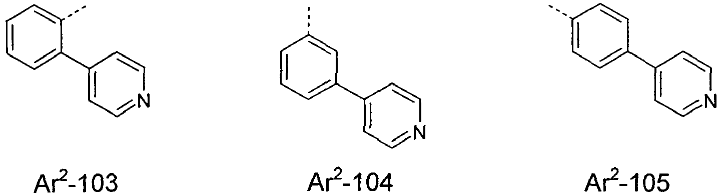 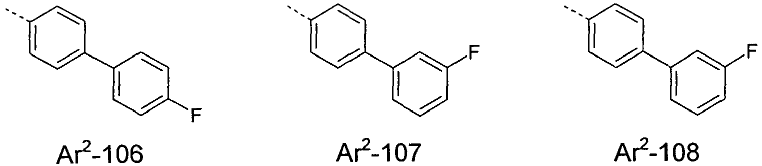 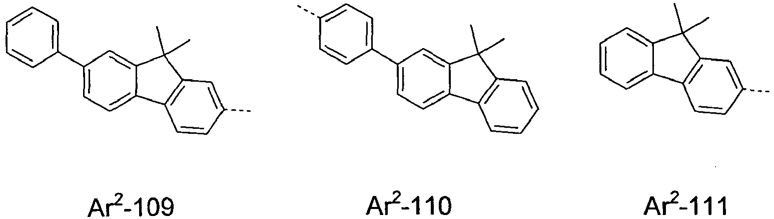 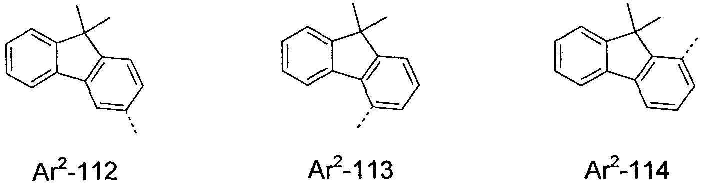 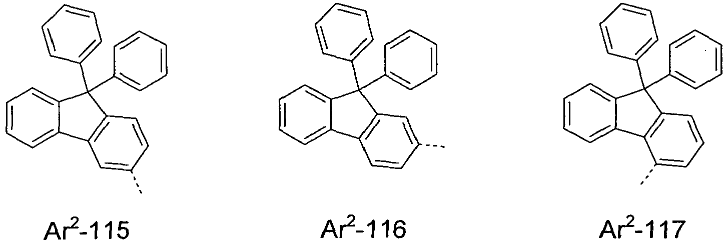 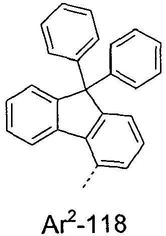 wobei die gestrichelte Bindung die Bindung an den Stickstoff darstellt und die Gruppen an den freien Positionen durch einen oder mehrere Reste R² substituiert sein können, bevorzugt an den freien Positionen aber unsubstituiert sind.

Dabei steht R² in den Gruppen der Formeln (Ar²-68) bis (Ar²-82) und (Ar²-85) bis (Ar²-87) bevorzugt gleich oder verschieden für eine Alkylgruppe mit 1 bis 10 C-Atomen, insbesondere für Methyl, oder eine Phenylgruppe, die mit einem oder mehreren Resten R³ substituiert sein kann und bevorzugt unsubstituiert ist. Zwei Alkylgruppen R² können dabei auch unter Ausbildung einer Spiro-Gruppe einen Ring bilden, bevorzugt einen Cyclohexylring oder einen Cyclopentylring.

Bevorzugte Ausführungsformen der Gruppen Ar³ entsprechen denjenigen für Gruppen Ar².

Es ist bevorzugt, dass X bei jedem Auftreten gleich oder verschieden gewählt ist aus einer Einfachbindung oder einer Gruppe gewählt aus C(R²)₂, C=O, O, S und NR², besonders bevorzugt ist X eine Einfachbindung.

R⁰ ist bevorzugt bei jedem Auftreten gleich oder verschieden H, D, F, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R³ substituiert sein können, oder ein aromatisches oder heteroaromatisches Ringsystem mit 5 bis 15 aromatischen Ringatomen. Besonders bevorzugt ist R⁰ bei jedem Auftreten gleich oder verschieden H, F, eine geradkettige Alkylgruppe mit 1 bis 10 C-Atomen oder eine verzweigte oder cyclische Alkylgruppe mit 3 bis 10 C-Atomen, wobei die oben genannten Gruppen jeweils mit einem oder mehreren Resten R³ substituiert sein können.

Bevorzugt ist R¹ und R² bei jedem Auftreten gleich oder verschieden H, D, F, CN, Si(R³)₃, N(R³)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch - C≡C-, -R³C=CR³-, Si(R³)₂, C=O, C=NR³, -NR³-, -O-, -S-, -C(=O)O- oder - C(=O)NR³- ersetzt sein können, oder ein aromatisches oder heteroaromatisches Ringsystem mit 5 bis 30 aromatischen Ringatomen, das jeweils mit einem oder mehreren Resten R³ substituiert sein kann, wobei zwei oder mehr Reste R¹ bzw. R² miteinander verknüpft sein können und einen Ring bilden können. Besonders bevorzugt ist R¹ und R² bei jedem Auftreten gleich oder verschieden H, D, F, CN, eine geradkettige Alkylgruppe mit 1 bis 10 C-Atomen oder eine verzweigte oder cyclische Alkylgruppe mit 3 bis 10 C-Atomen, wobei die oben genannten Gruppen jeweils mit einem oder mehreren Resten R³ substituiert sein können, oder ein aromatisches oder heteroaromatisches Ringsystem mit 6 bis 25 aromatischen Ringatomen, das jeweils mit einem oder mehreren Resten R³ substituiert sein kann.

Bevorzugt ist R¹ bei jedem Auftreten gleich oder verschieden H, F, CN, eine geradkettige Alkylgruppe mit 1 bis 10 C-Atomen oder eine verzweigte oder cyclische Alkylgruppe mit 3 bis 10 C-Atomen, wobei die oben genannten Gruppen jeweils mit einem oder mehreren Resten R³ substituiert sein können, oder ein aromatisches oder heteroaromatisches Ringsystem mit 6 bis 25 aromatischen Ringatomen, das jeweils mit einem oder mehreren Resten R³ substituiert sein kann. Besonders bevorzugte Gruppen R¹ sind H, F, CN, Methyl, tert-Butyl, Phenyl, Biphenyl, Dibenzofuran, Dibenzothiophen und Carbazol.

Wenn ein an das Grundgerüst von Formel (I) gebundener Rest R¹ nicht H darstellt, so ist er bevorzugt in einer der Positionen 4, 5, 4' und 5' des Spirobifluoren-Grundgerüsts gebunden, wobei die Nummerierung wie folgt ist:

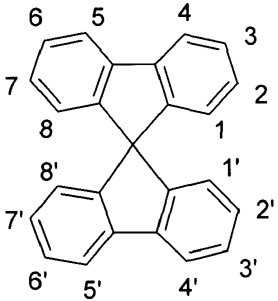

Bevorzugt ist R³ bei jedem Auftreten gleich oder verschieden H, D, F, CN, Si(R⁴)₃, N(Ar³)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, - R⁴C=CR⁴-, Si(R⁴)₂, C=O, C=NR⁴, -NR⁴-, -O-, -S-, -C(=O)O- oder - C(=O)NR⁴- ersetzt sein können, oder ein aromatisches oder heteroaromatisches Ringsystem mit 5 bis 30 aromatischen Ringatomen, das jeweils mit einem oder mehreren Resten R⁴ substituiert sein kann, wobei zwei oder mehr Reste R³ miteinander verknüpft sein können und einen Ring bilden können. Besonders bevorzugt ist R³ bei jedem Auftreten gleich oder verschieden H, D, F, CN, N(Ar³)₂, eine geradkettige Alkylgruppe mit 1 bis 10 C-Atomen oder eine verzweigte oder cyclische Alkylgruppe mit 3 bis 10 C-Atomen, wobei die oben genannten Gruppen jeweils mit einem oder mehreren Resten R⁴ substituiert sein können, oder ein aromatisches oder heteroaromatisches Ringsystem mit 6 bis 25 aromatischen Ringatomen, das jeweils mit einem oder mehreren Resten R⁴ substituiert sein kann.

Es gelten für Formeln (I-1) bis (I-6) insbesondere die bevorzugten Ausführungsformen von R⁰, Ar¹ und A.

Die folgende Tabelle zeigt Beispiele für Verbindungen gemäß Anspruch 1. Nicht anspruchsgemäße Verbindungen sind mit # gekennzeichnet.

| | | |
|---|---|---|
| 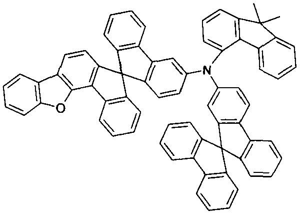 | 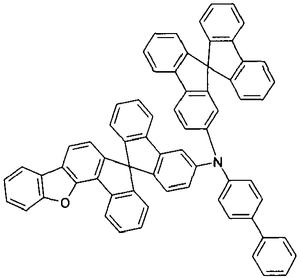 | 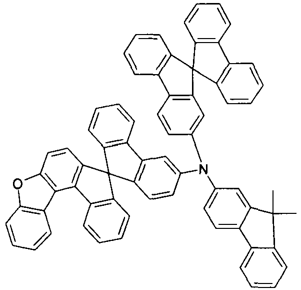 |
| 1 | 2 | 3 |
| 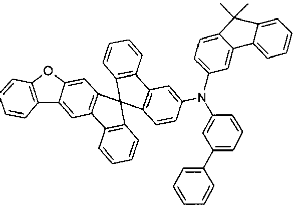 | 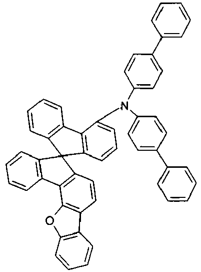 | 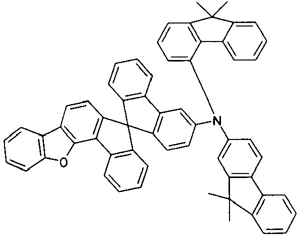 |
| 4# | 5 | 6 |
| 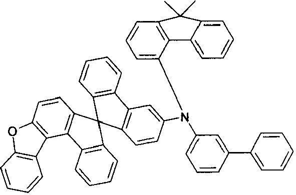 | 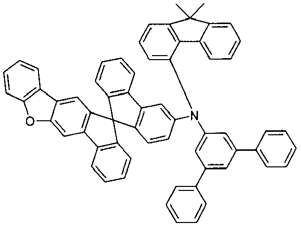 | 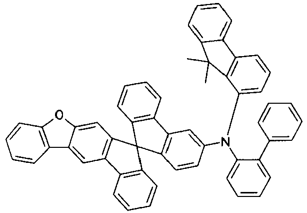 |
| 7 | 8# | 9# |
| 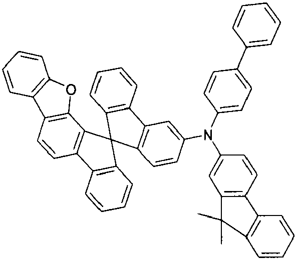 | 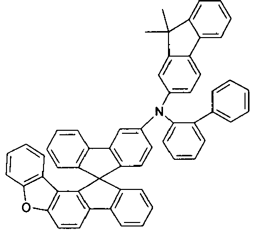 | 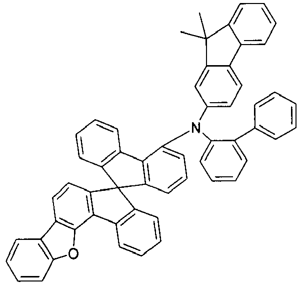 |
| 10# | 11# | 12 |
| 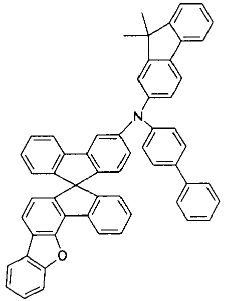 | 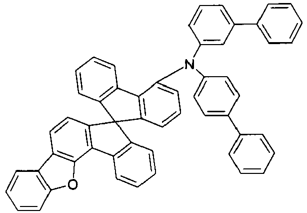 | 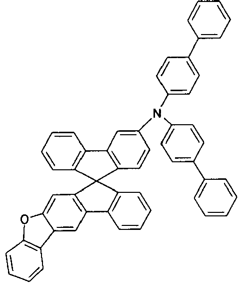 |
| 13 | 14 | 15# |
| 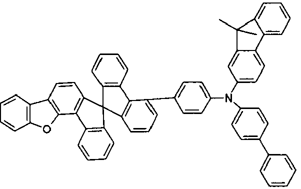 | 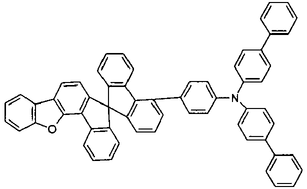 | 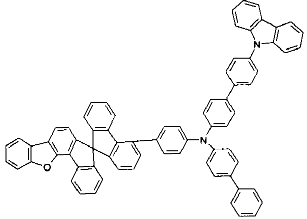 |
| 16 | 17 | 18 |
| 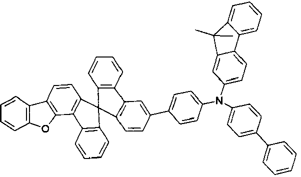 | 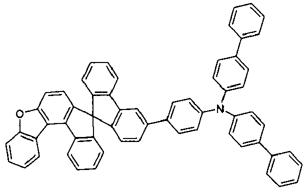 | 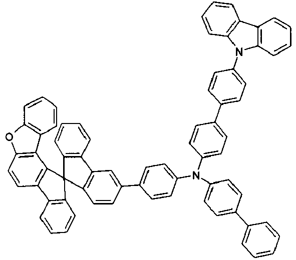 |
| 19 | 20 | 21# |
| 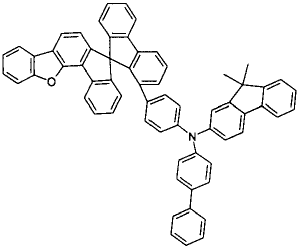 | 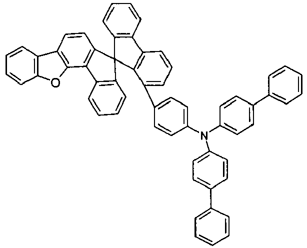 | 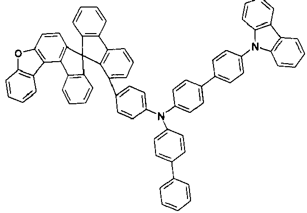 |
| 22 | 23 | 24 |
| 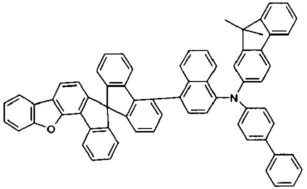 | 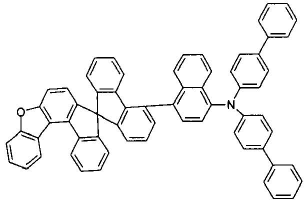 | 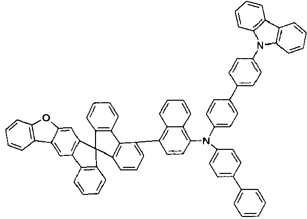 |
| 25 | 26 | 27# |
| 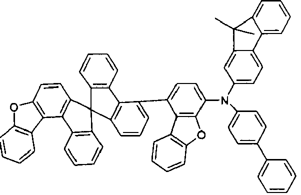 | 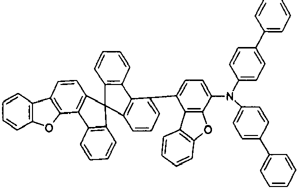 | 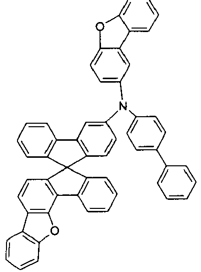 |
| 28 | 29 | 30 |
| 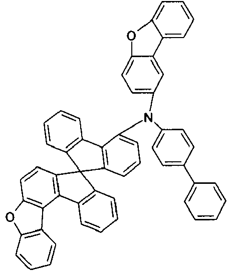 | 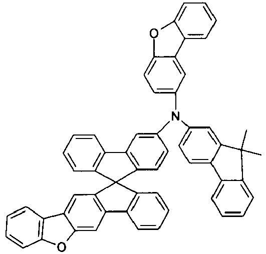 | 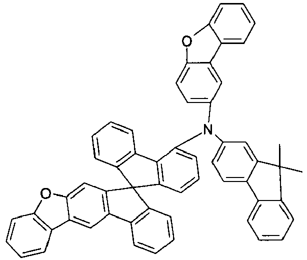 |
| 31 | 32# | 33# |
| 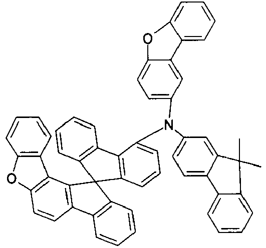 | 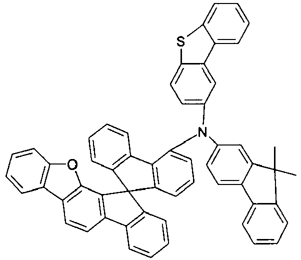 | 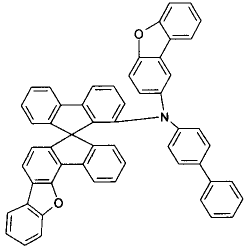 |
| 34# | 35# | 36 |
| 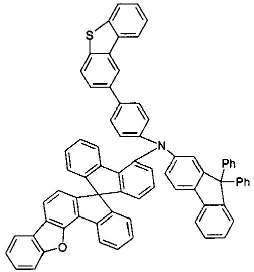 | 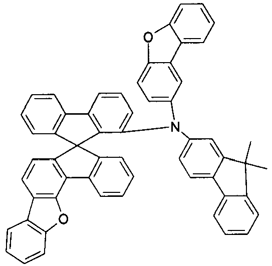 | 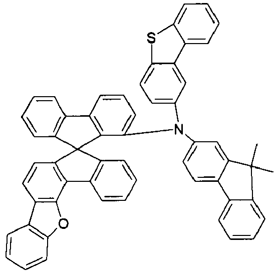 |
| 37 | 38 | 39 |
| 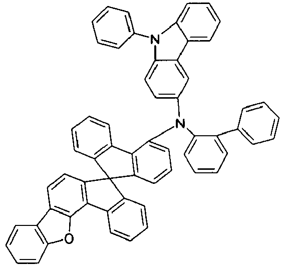 | 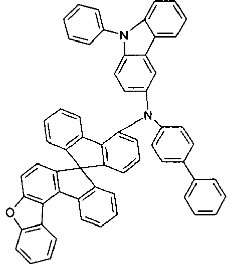 | 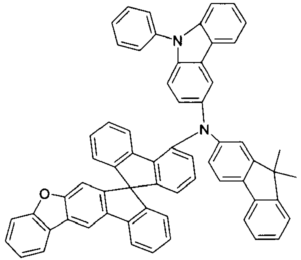 |
| 40 | 41 | 42# |
| 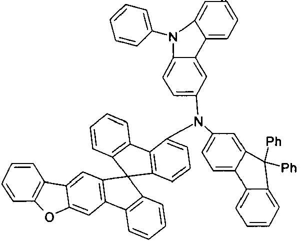 | 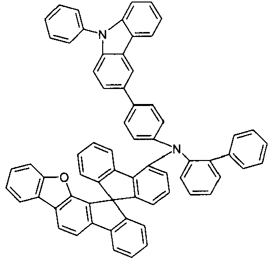 | 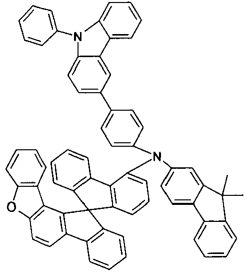 |
| 43# | 44# | 45# |
| 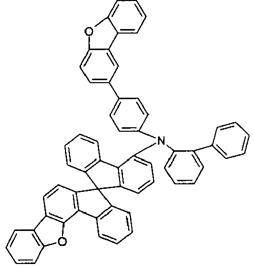 | 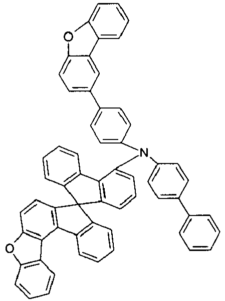 | 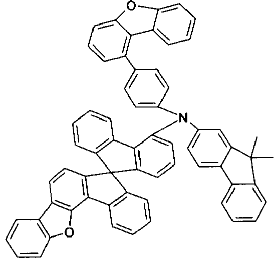 |
| 46 | 47 | 48 |
| 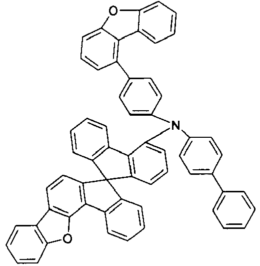 | 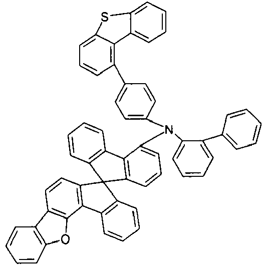 | 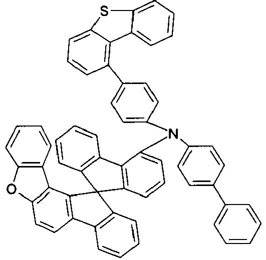 |
| 49 | 50 | 51# |
| 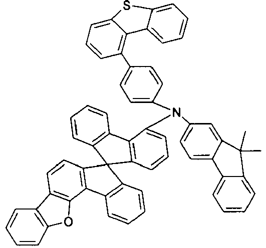 | 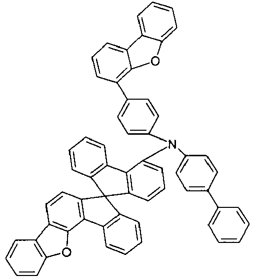 | 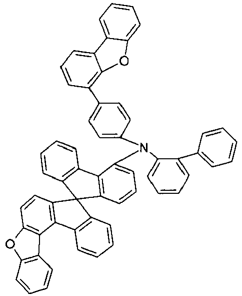 |
| 52 | 53 | 54 |
| 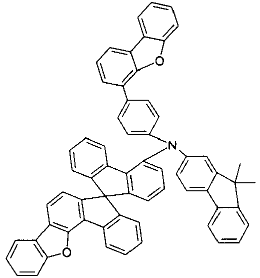 | 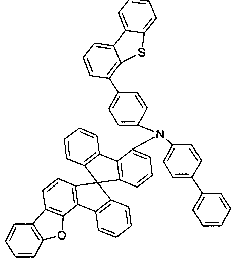 | 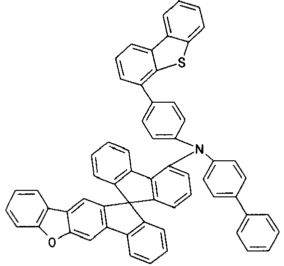 |
| 55 | 56 | 57# |
| 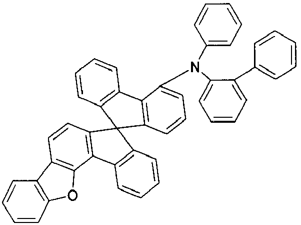 | 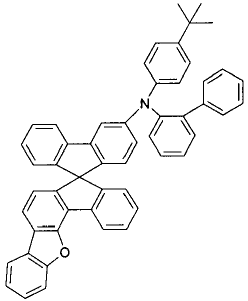 | 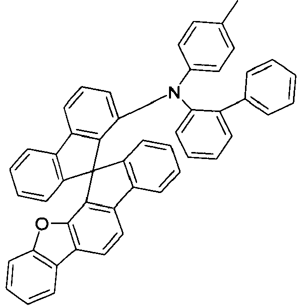 |
| 58 | 59 | 60# |
| 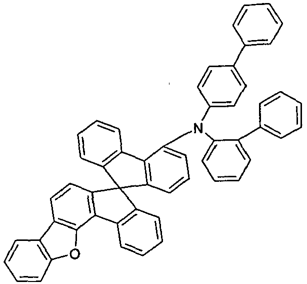 | 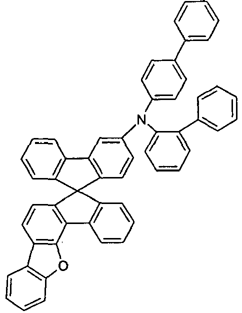 | 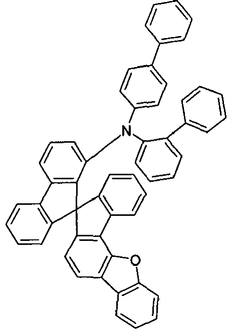 |
| 61 | 62 | 63 |
| 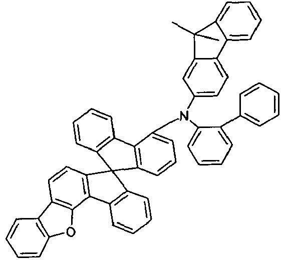 | 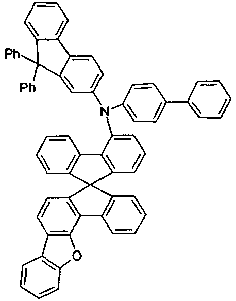 | 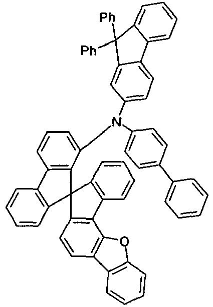 |
| 64 | 65 | 66 |
| 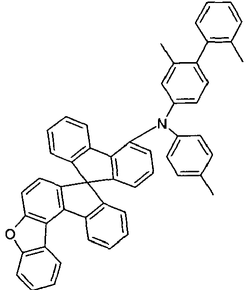 | 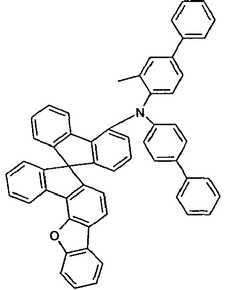 | 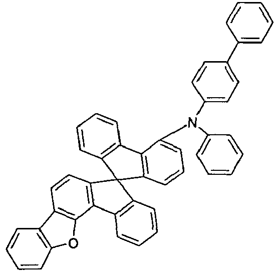 |
| 67 | 68 | 69 |
| 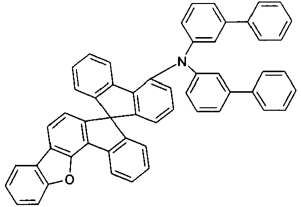 | 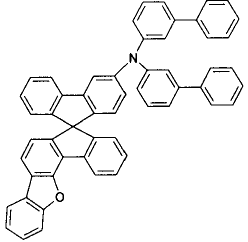 | 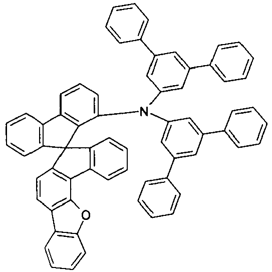 |
| 70 | 71 | 72 |
| 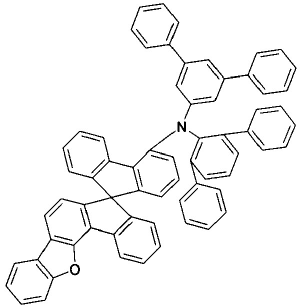 | 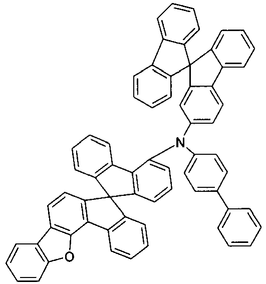 | 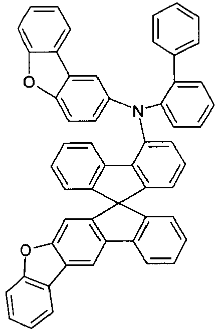 |
| 73 | 74 | 75# |
| 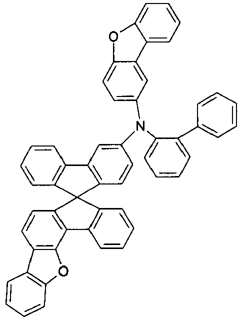 | 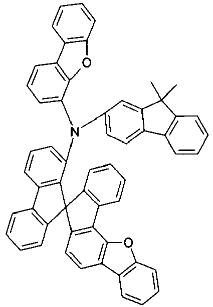 | 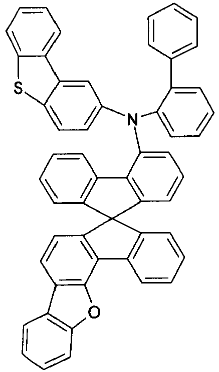 |
| 76 | 77 | 78 |
| 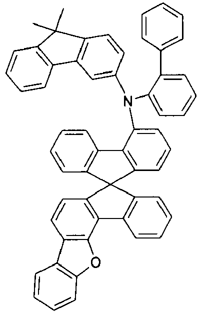 | 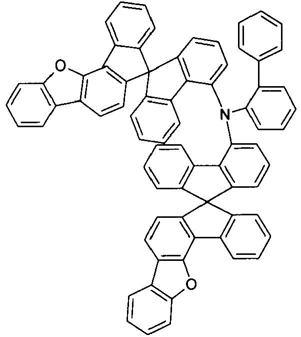 | 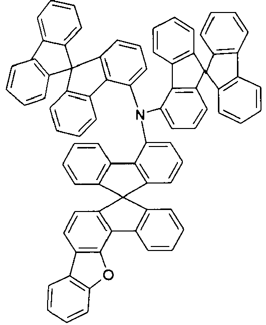 |
| 79 | 80 | 81 |
| 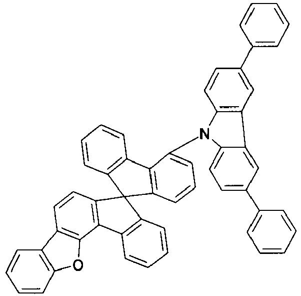 | 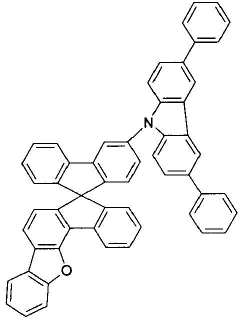 | 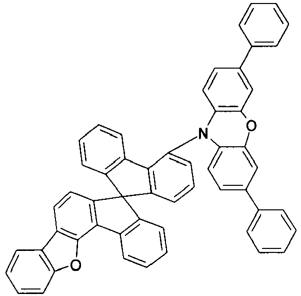 |
| 82 | 83 | 84 |
| 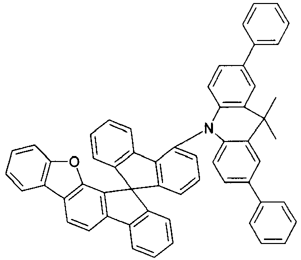 | 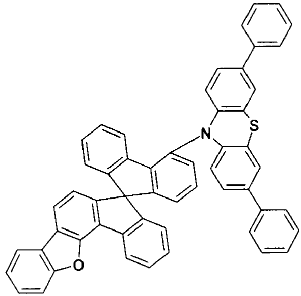 | 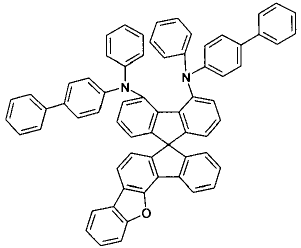 |
| 85# | 86 | 87 |
| 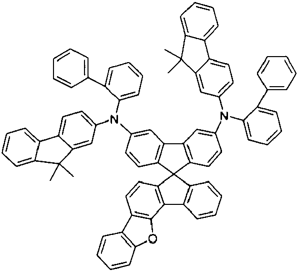 | 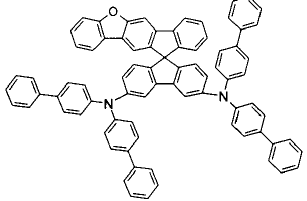 | 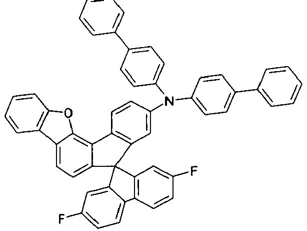 |
| 88 | 89# | 90 |
| 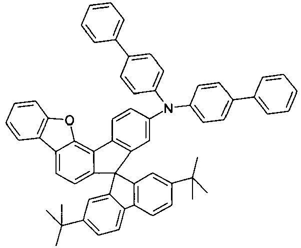 | 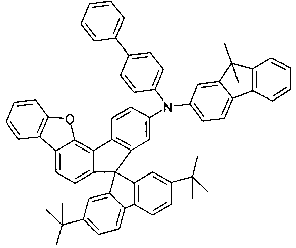 | 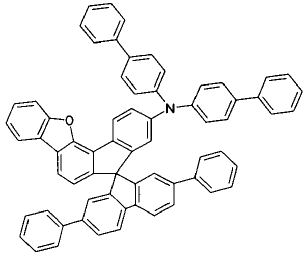 |
| 91 | 92 | 93 |
| 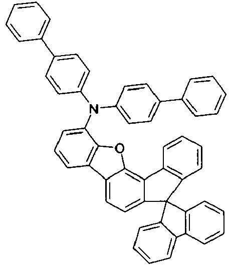 | 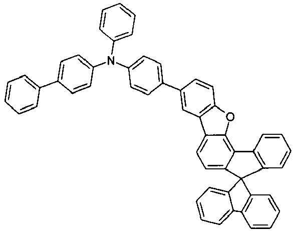 | 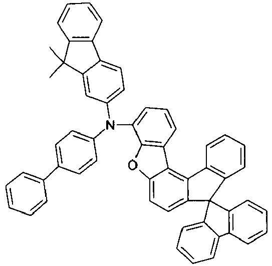 |
| 94 | 95 | 96 |
| 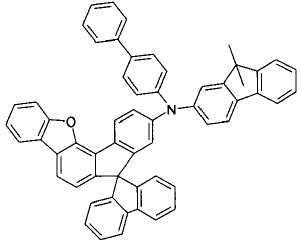 | 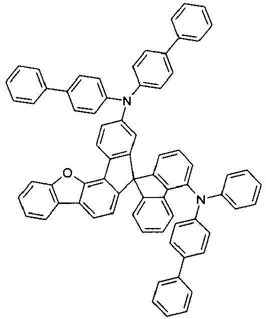 | 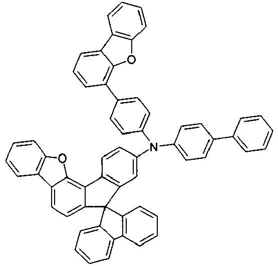 |
| 97 | 98 | 99 |
| 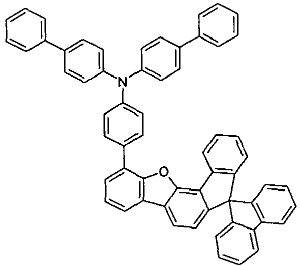 | 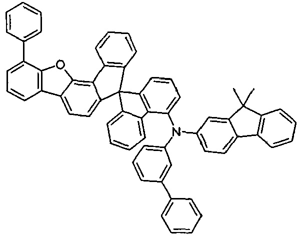 | 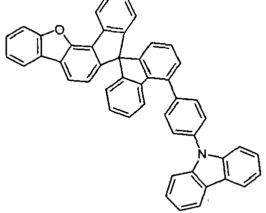 |
| 100 | 101 | 102 |
| 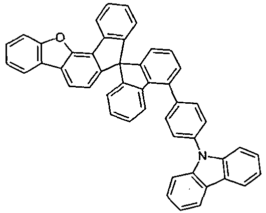 | 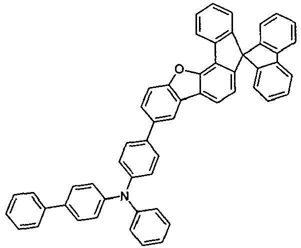 | 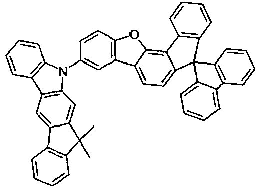 |
| 103 | 104 | 105 |
| 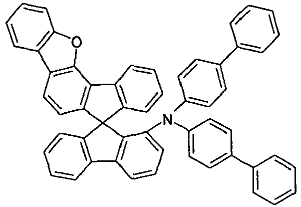 | 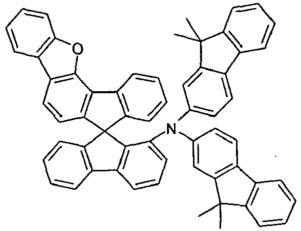 | 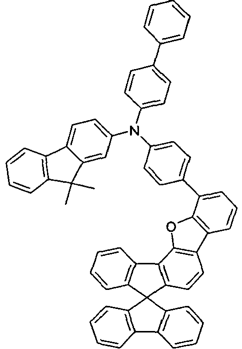 |
| 106 | 107 | 108 |
| 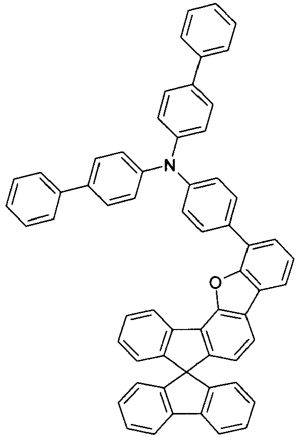 | 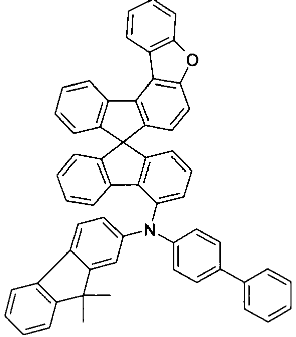 | 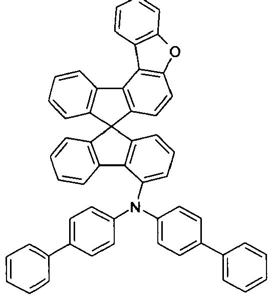 |
| 109 | 110 | 111 |
| 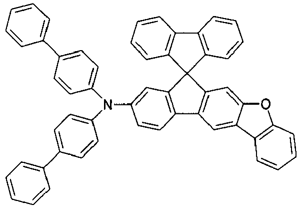 | 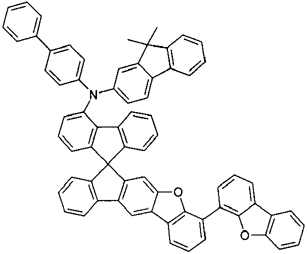 | 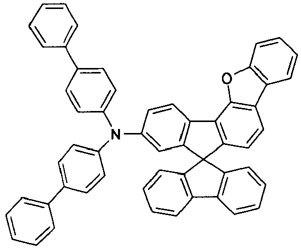 |
| 112# | 113# | 114 |
| 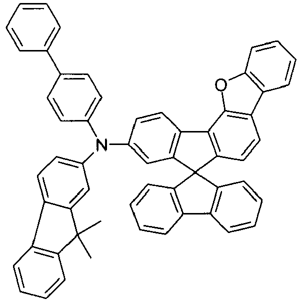 | 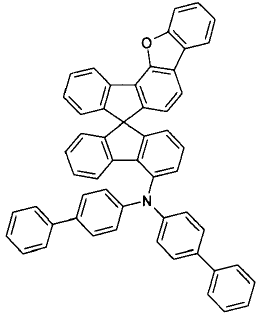 | 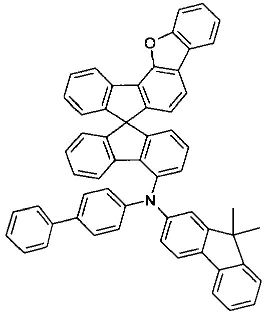 |
| 115 | 116 | 117 |
| 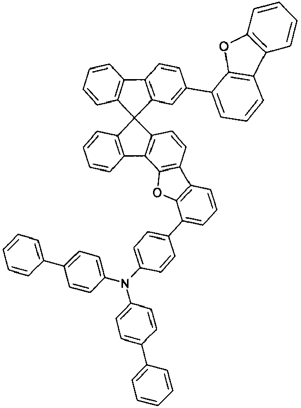 | 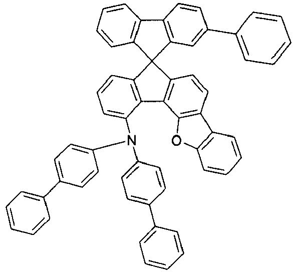 | 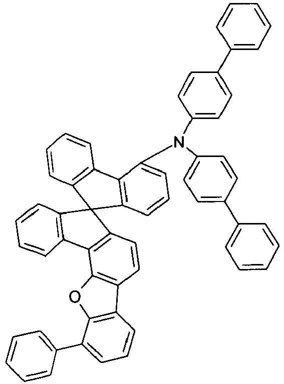 |
| 118 | 119 | 120 |
| 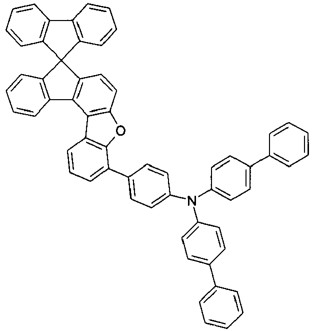 | 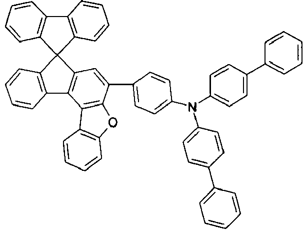 | 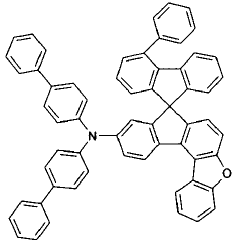 |
| 121 | 122# | 123 |
| 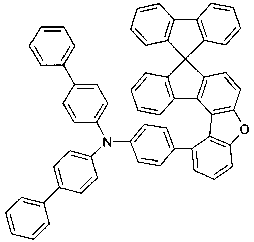 | 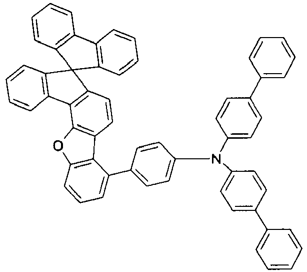 | 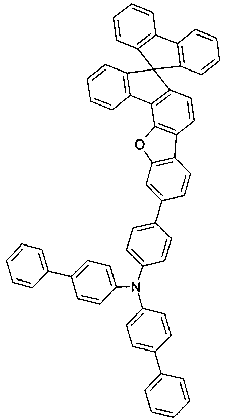 |
| 124 | 125 | 126 |
| 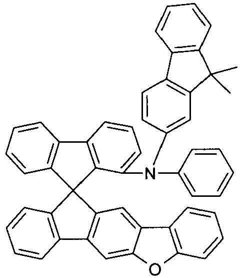 | 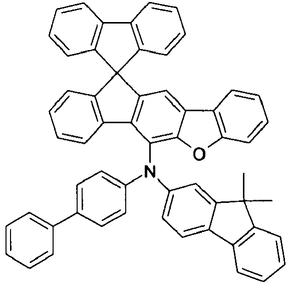 | 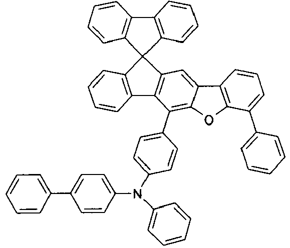 |
| 127# | 128# | 129# |
| 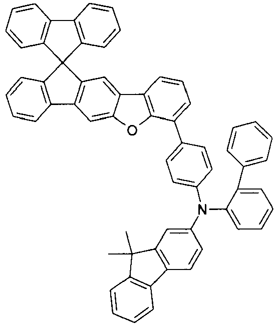 | 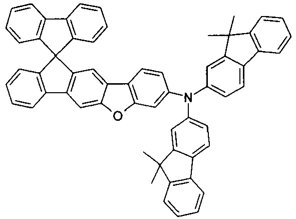 | 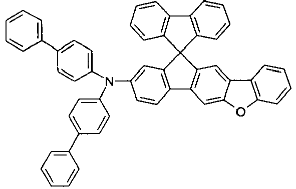 |
| 130# | 131# | 132# |
| 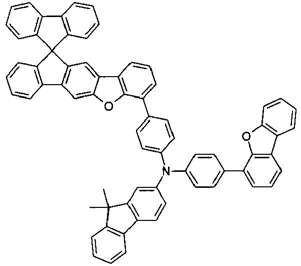 | 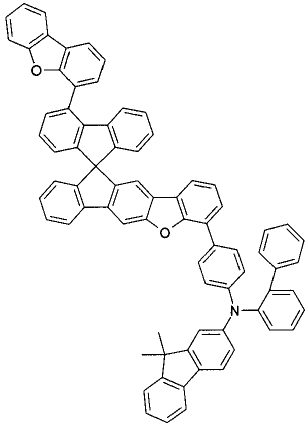 | 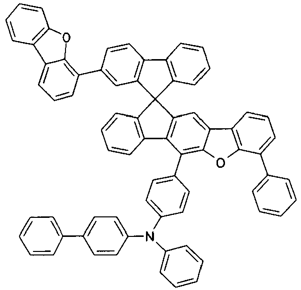 |
| 133# | 134# | 135# |
| 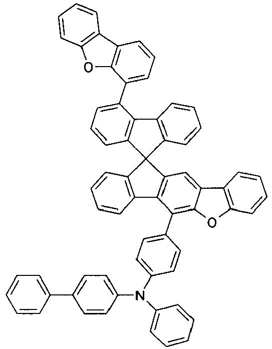 | 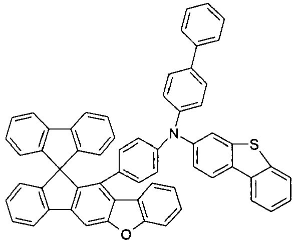 | 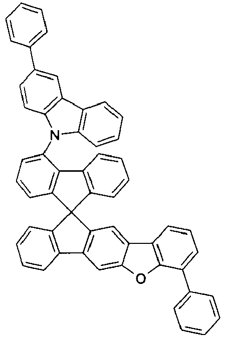 |
| 136# | 137# | 138# |
| 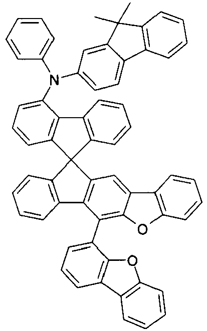 | 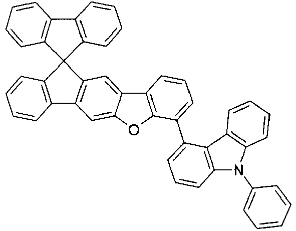 | 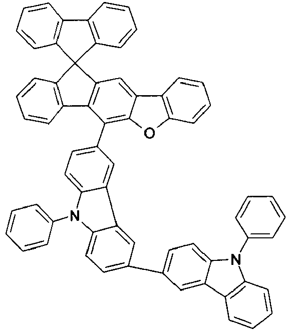 |
| 139# | 140# | 141# |
| 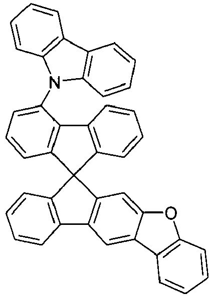 | 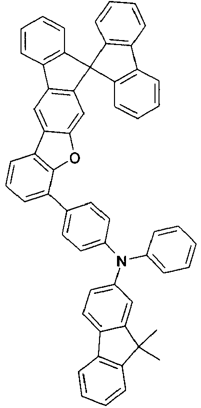 | 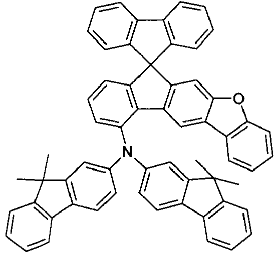 |
| 142# | 143# | 144# |
| 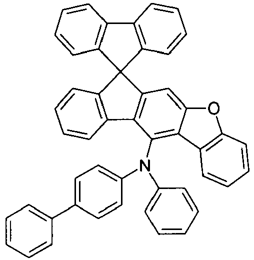 | 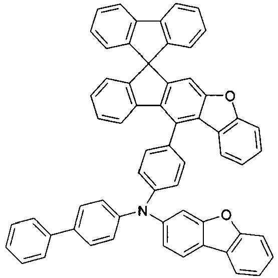 | 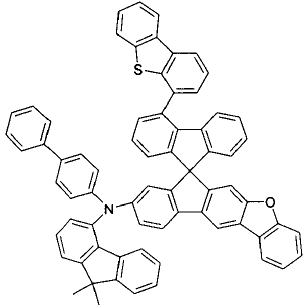 |
| 145# | 146# | 147# |
| 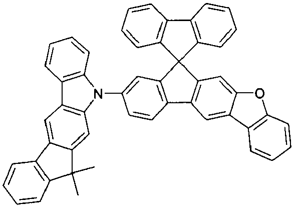 | 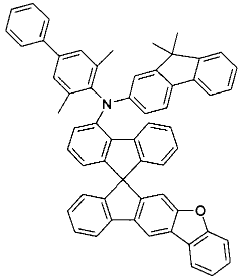 | 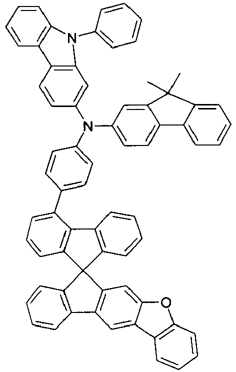 |
| 148# | 149# | 150# |
| 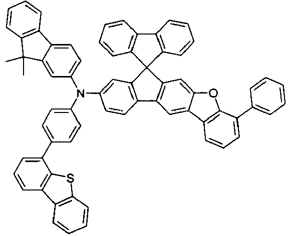 | 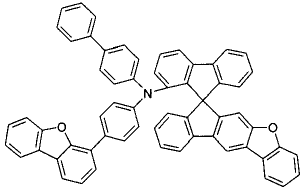 | 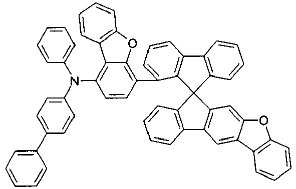 |
| 151# | 152# | 153# |
| 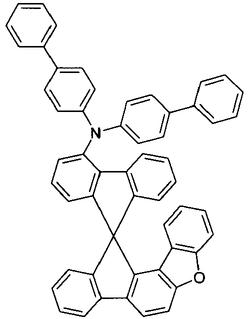 | 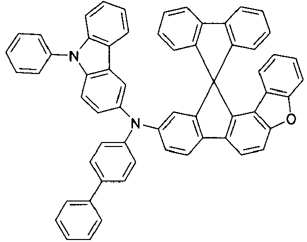 | 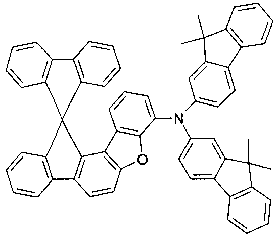 |
| 154# | 155# | 156# |
| 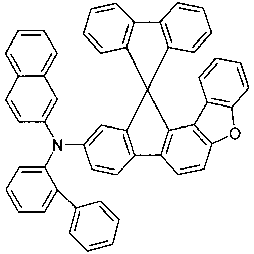 | 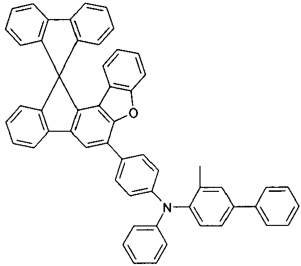 | 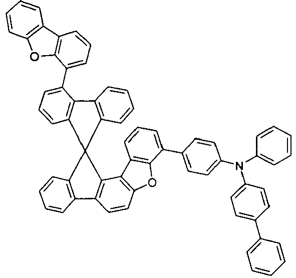 |
| 157# | 158# | 159# |
| 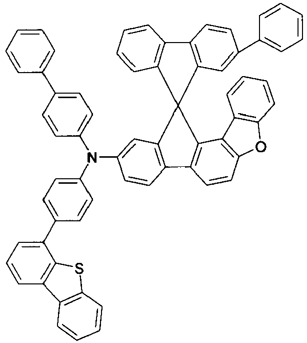 | 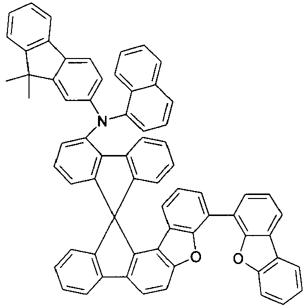 | 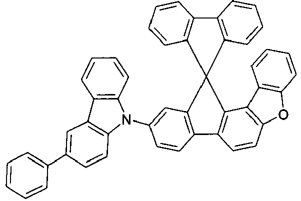 |
| 160# | 161# | 162# |
| 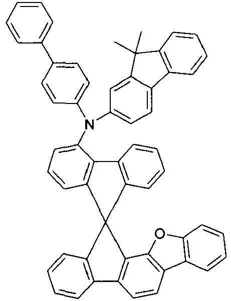 | 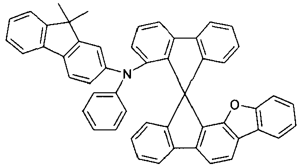 | 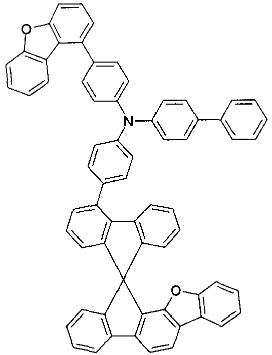 |
| 163# | 164# | 165# |
| 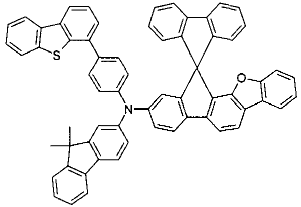 | 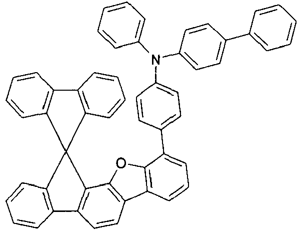 | 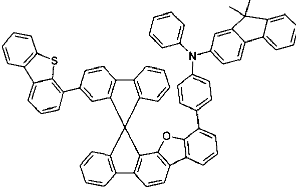 |
| 166# | 167# | 168# |
| 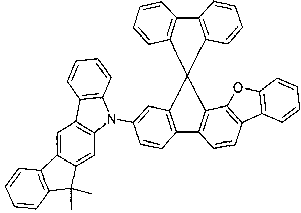 | 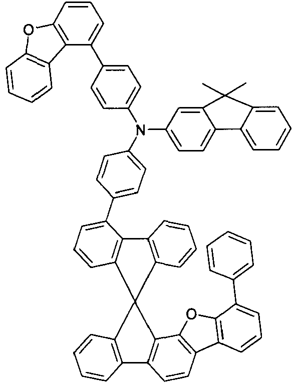 | 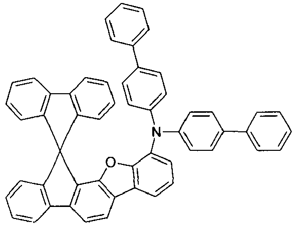 |
| 169# | 170# | 171# |

Die Synthese der Verbindungen gemäß Formel (I) kann mit im Stand der Technik bekannten Verfahren und Reaktionstypen, beispielsweise Halogenierung, metallorganische Addition, Buchwald-Kupplung und Suzuki-Kupplung durchgeführt werden.

Schemata 1, 2, 9 und 10 zeigen mögliche Synthesewege zur Herstellung der erfindungsgemäßen Verbindungen. Sie dienen zur Erläuterung der Erfindung für den Fachmann, und sind nicht einschränkend auszulegen. Der Fachmann kann im Rahmen seines allgemeinen Fachwissens die gezeigten Synthesewege abwandeln, oder völlig andere Wege entwickeln, falls dies vorteilhafter erscheint.

Schemata 1 bis 2 zeigen Verfahren zur Herstellung von Verbindungen der Formeln (II-5) bis (II-6), die Zwischenstufen bei der Herstellung von Verbindungen der Formel (I) darstellen.

Verbindungen der Formeln (II-5) bis (II-6) haben die folgenden Strukturen: 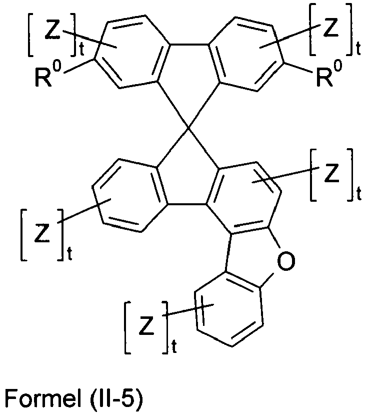 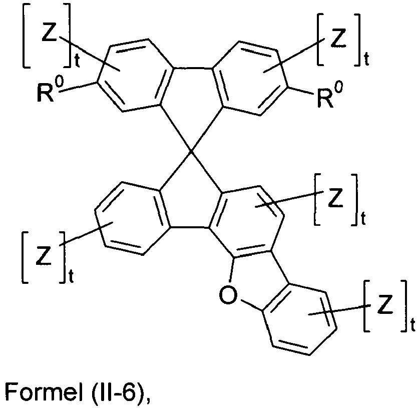 welche an einem oder mehreren freien Positionen mit einem Rest R¹ substituiert sein können, der wie oben definiert ist, und wobei R⁰ wie oben definiert ist und die weiteren Variablen wie folgt definiert sind:
- Z: ist bei jedem Auftreten gleich oder verschieden gewählt aus F, Cl, Br, I, B(OR³)₂, OSO₂R³, S(=O)R³ und S(=O)₂R³;
- t: ist bei jedem Auftreten gleich oder verschieden 0 oder 1, wobei mindestens ein Index t pro Formel gleich 1 ist.

Für die Verbindungen der Formeln (II-5) bis (II-6) gelten die oben für Formel (I) angegebenen Definitionen der Reste R⁰ und R¹ bis R³ ebenfalls als bevorzugt.

Es ist für Formeln (II-5) bis (II-6) bevorzugt, dass genau eine oder genau zwei Indices t gleich 1 sind.

Es ist für Formeln (II-5) bis (II-6) bevorzugt, dass Z bei jedem Auftreten gleich oder verschieden gewählt ist aus Cl, Br, I und B(OR³)₂.

Die Zwischenstufen gemäß den Formeln (II-5) bis (II-6) sind neu und als solche Gegenstand der vorliegenden Anmeldung.

In allen folgenden Syntheseschemata sind die Verbindungen unsubstituiert gezeigt. Dies schließt das Vorhandensein von beliebigen Substituenten in den Verfahren nicht aus.

In Schema 1 ist eine geeignete Synthese für die Zwischenstufe gemäß Formel (II-6) gezeigt.

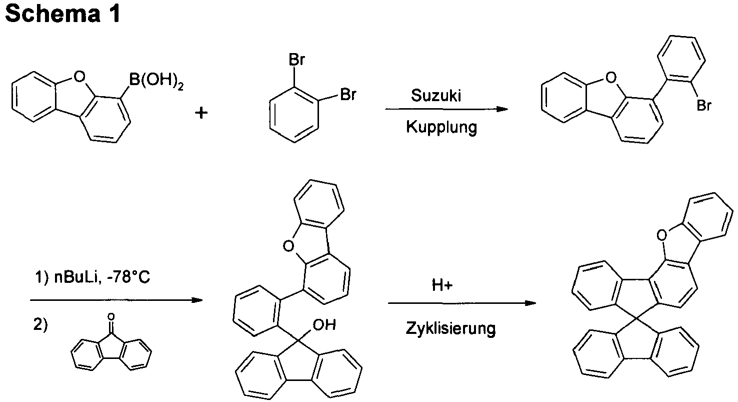

In Schema 2 ist eine geeignete Synthese für die Zwischenstufe gemäß Formel (II-5) gezeigt.

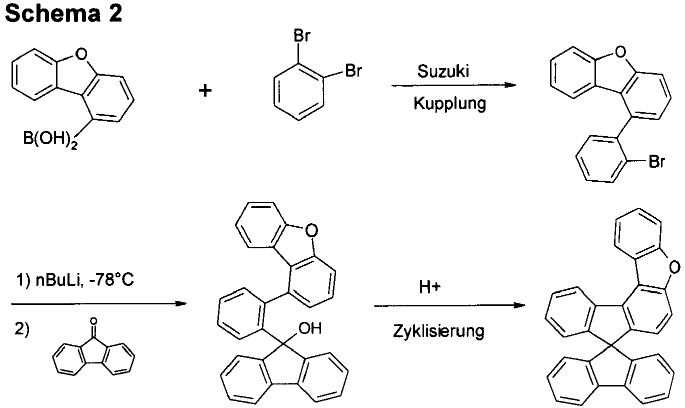

Die oben gezeigten Bausteine können an der Benzofluoren-Einheit mit einer reaktiven Gruppe, wie beispielsweise Halogen, versehen werden, wie das folgende Schema zeigt:

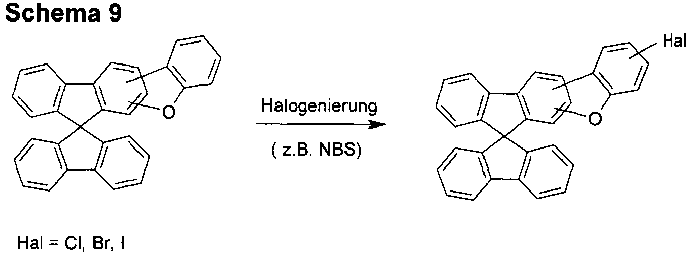

Die mit reaktiven Gruppen Z versehenen Zwischenstufen gemäß den Formeln (II-5) bis (II-6) sind vielseitige Bausteine, die zu Verbindungen der Formel (I) umgesetzt werden können, wie das folgende Schema zeigt: 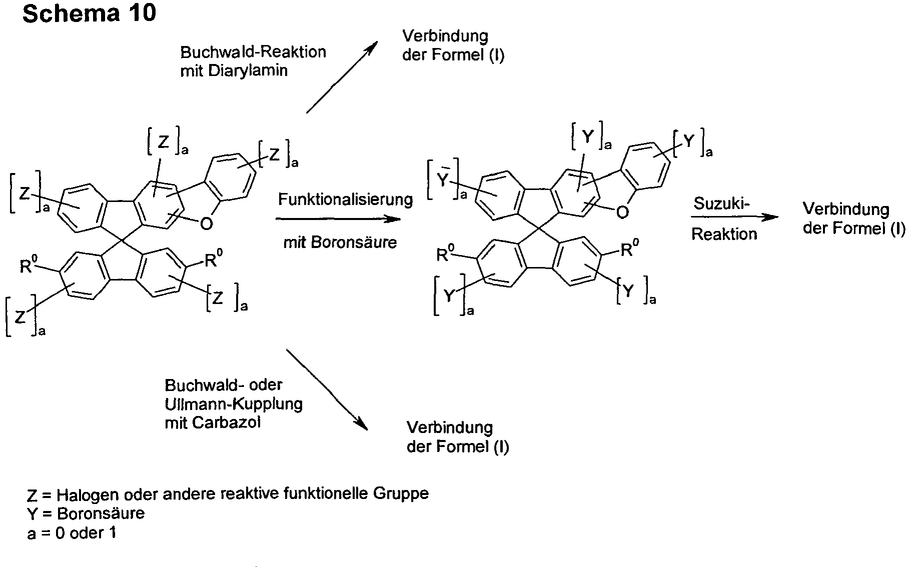 Gegenstand der vorliegenden Anmeldung ist daher auch ein Verfahren zur Herstellung von Verbindungen gemäß Formel (I), dadurch gekennzeichnet, dass zunächst das Spirobifluoren-Grundgerüst hergestellt wird, und in einem späteren Schritt über eine metallorganische Kupplungsreaktion eine Arylamino- oder eine Carbazolgruppe oder eine Aryl- oder Heteroarylgruppe, die mit einer Arylamino- oder Carbazolgruppe substituiert ist, eingeführt wird.

Die metallorganische Kupplungsreaktion ist dabei bevorzugt eine Buchwald-Kupplung oder eine Suzuki-Kupplung.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R⁰, R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer*)* und/oder organischen oder anorganischen p/n-Übergängen.

Die Abfolge der Schichten der organischen Elektrolumineszenzvorrichtung enthaltend die Verbindung der Formel (I) ist bevorzugt die folgende: Anode-Lochinjektionsschicht-Lochtransportschicht-wahlweise weitere Lochtransportschicht-wahlweise Elektronenblockierschicht-emittierende Schicht-Elektronentransportschicht-Elektroneninjektionsschicht-Kathode. Es müssen jedoch nicht alle der genannten Schichten vorhanden sein, und es können zusätzlich weitere Schichten vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen sind dabei bevorzugt in der Lochtransportschicht oder der Elektronenblockierschicht vorhanden. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (I) in einer elektronischen Vorrichtung enthaltend einen oder mehrere phosphoreszierende Emitter eingesetzt wird. Dabei kann die Verbindung in unterschiedlichen Schichten, bevorzugt in einer Lochtransportschicht, einer Elektronenblockierschicht, einer Lochinjektionsschicht oder in einer emittierenden Schicht, enthalten sein.

Vom Begriff phosphoreszierende Emitter sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Emitter angesehen.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen. Weitere Beispiele sind in einer folgenden Tabelle aufgeführt.

Die Verbindung gemäß Formel (I) kann aber auch erfindungsgemäß in einer elektronischen Vorrichtung enthaltend einen oder mehrere fluoreszierende Emitter eingesetzt werden.

In einer bevorzugten Ausführungsform der Erfindung werden die Verbindungen gemäß Formel (I) als Lochtransportmaterial eingesetzt. Die Verbindungen werden dann bevorzugt in einer Lochtransportschicht, einer Elektronenblockierschicht oder einer Lochinjektionsschicht eingesetzt.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden inbesondere die folgenden Verbindungen:

| | | |
|---|---|---|
| 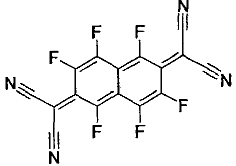 | 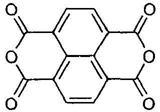 | 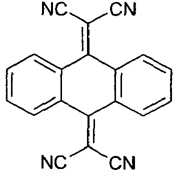 |
| (D-1) | (D-2) | (D-3) |
| 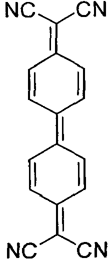 | 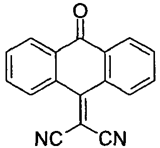 | 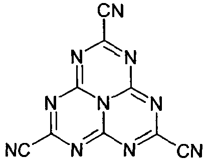 |
| (D-4) | (D-5) | (D-6) |
| 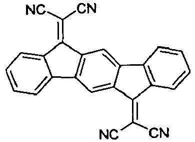 | 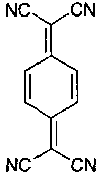 | 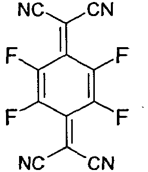 |
| (D-7) | (D-8) | (D-9) |
| 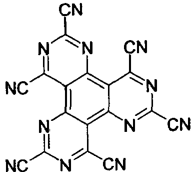 | 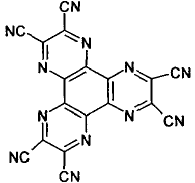 | 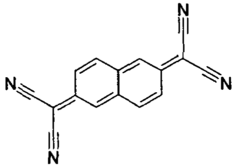 |
| (D-10) | (D-11) | (D-12) |

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

In einer weiteren Ausführungsform der vorliegenden Erfindung wird die Verbindung der Formel (I) als Matrixmaterial in Kombination mit einem oder mehreren Emittern, vorzugsweise phosphoreszierenden Emittern, eingesetzt.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Emitters zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Emitter enthalten. Auch in diesem Fall sind die Emitter im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil eines einzelnen Emitters.

Es ist bevorzugt, dass die Verbindungen gemäß Formel (I) als eine Komponente von Mixed-Matrix-Systemen verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) stellt dabei bevorzugt das Matrixmaterial mit lochtransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Emitter umfassen, bevorzugt einen oder mehrere phosphoreszierende Emitter. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Emitter oder den bevorzugten Matrixmaterialien für fluoreszierende Emitter, je nachdem welche Art von Emitter im mixed-Matrix-System eingesetzt wird.

Bevorzugte phosphoreszierende Emitter zur Verwendung in Mixed-Matrix-Systemen sind die obenstehend aufgeführten phosphoreszierenden Emitter.

Gemäß einer weiteren bevorzugten Ausführungsform der Erfindung kann die Verbindung der Formel (I) als fluoreszierender Emitter in einer emittierenden Schicht eingesetzt werden. Wenn die erfindungsgemäße Verbindung als fluoreszierender Emitter in einer emittierenden Schicht eingesetzt wird, wird sie bevorzugt in Kombination mit einem oder mehreren Matrixmaterialien eingesetzt. Bevorzugte Matrixmaterialien zur Verwendung in Kombination mit der Verbindung der Formel (I) als Emitter sind weiter unten angegeben.

Im Folgenden werden bevorzugte Ausführungsformen für die verschiedenen Funktionsmaterialien der elektronischen Vorrichtung aufgeführt.

Bevorzugte phosphoreszierende Emitter sind die oben genannten Verbindungen und die in der folgenden Tabelle gezeigten Verbindungen:

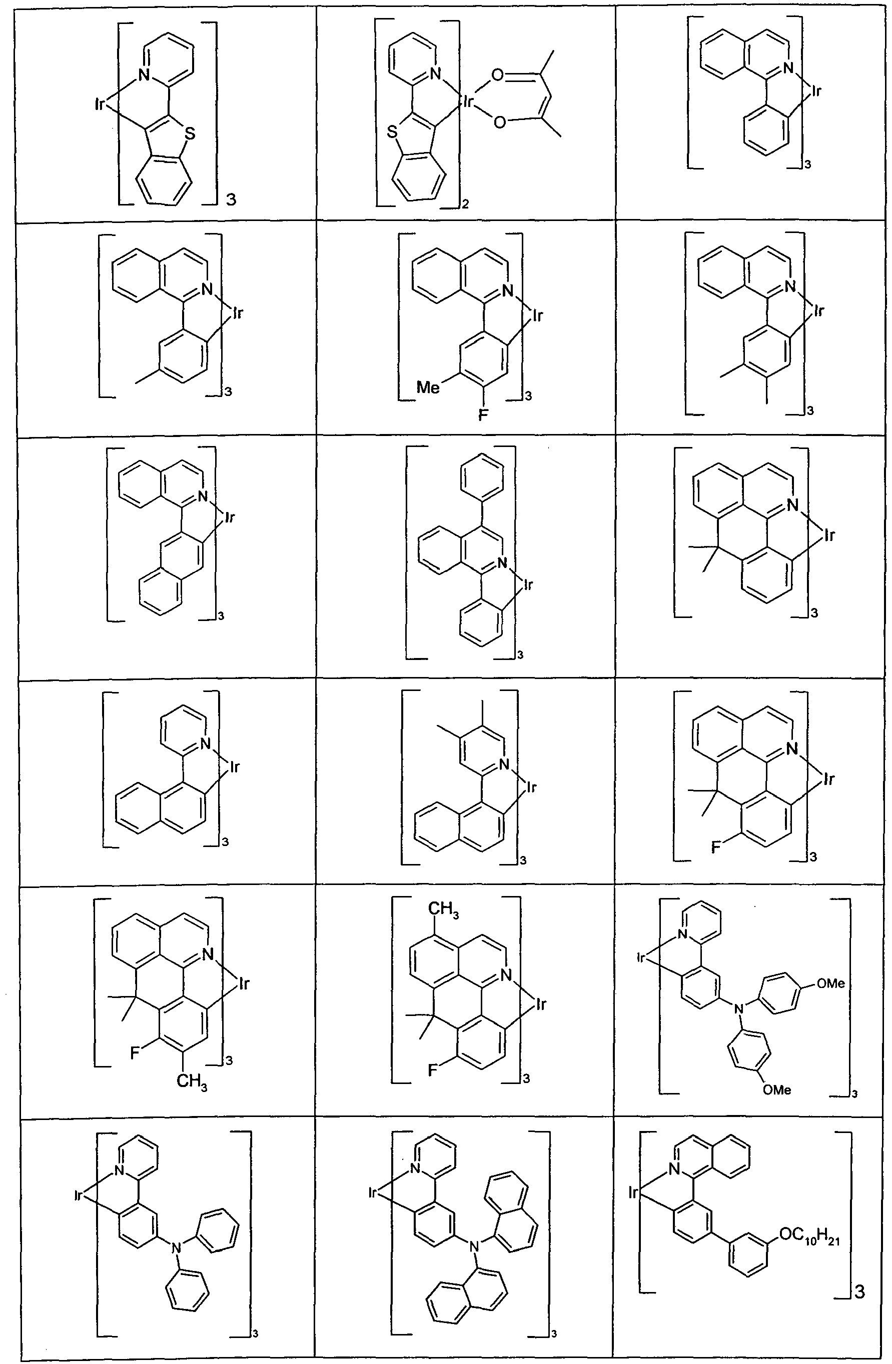

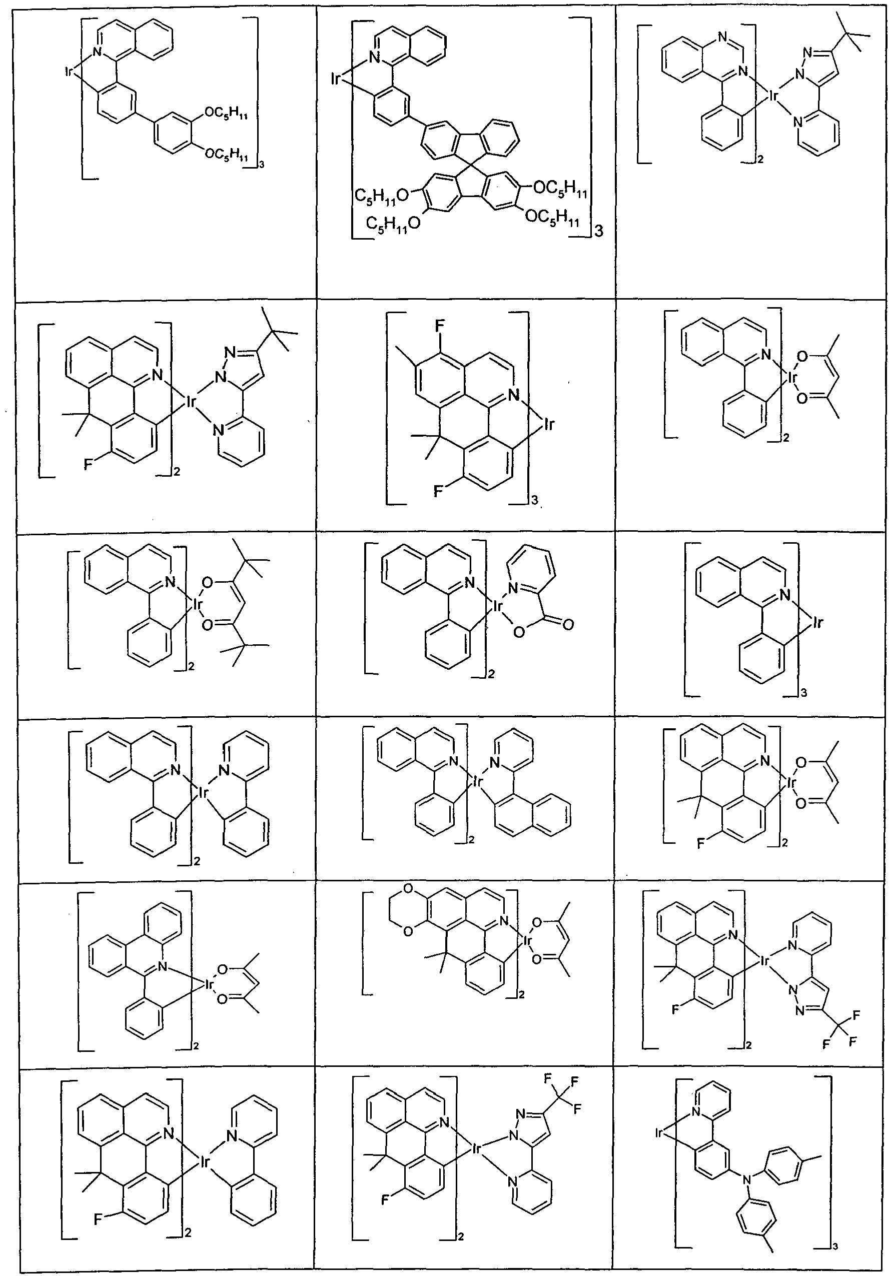

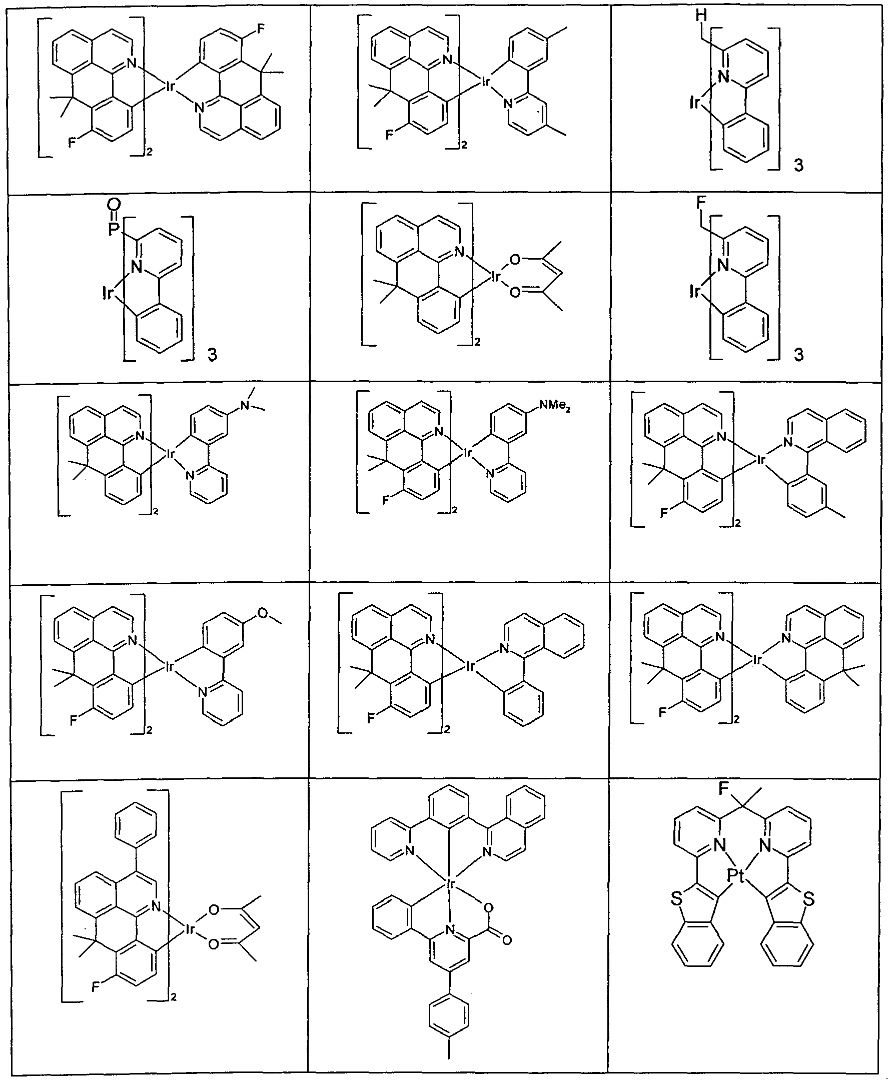

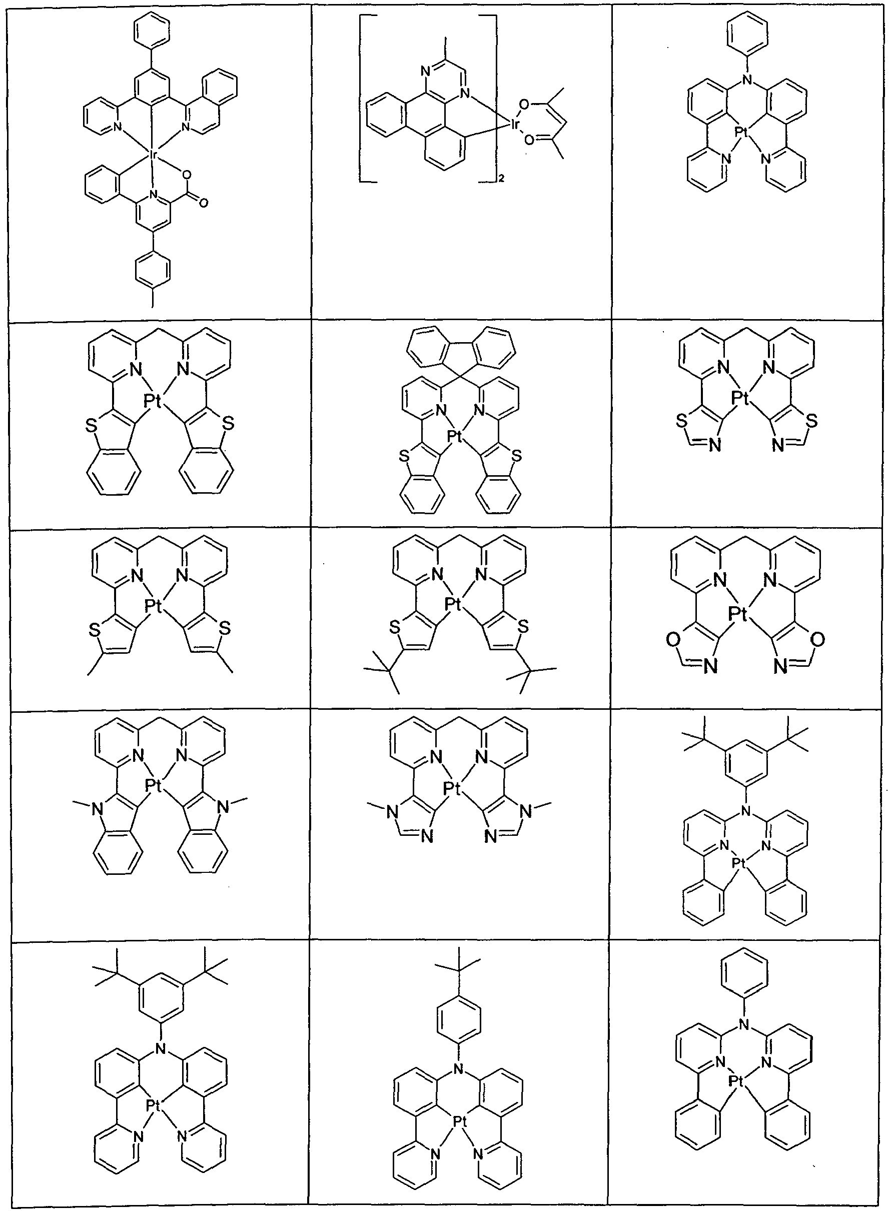

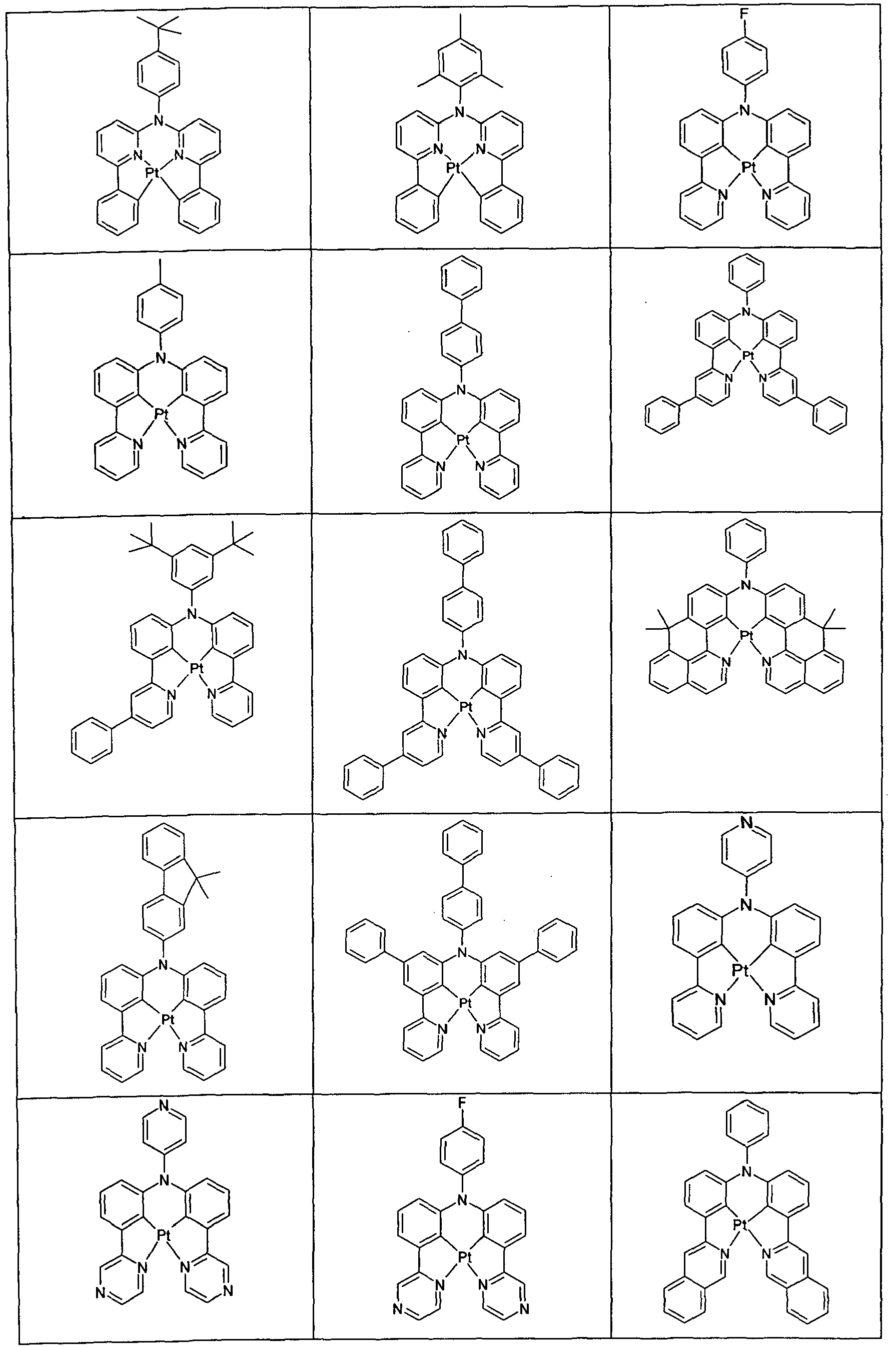

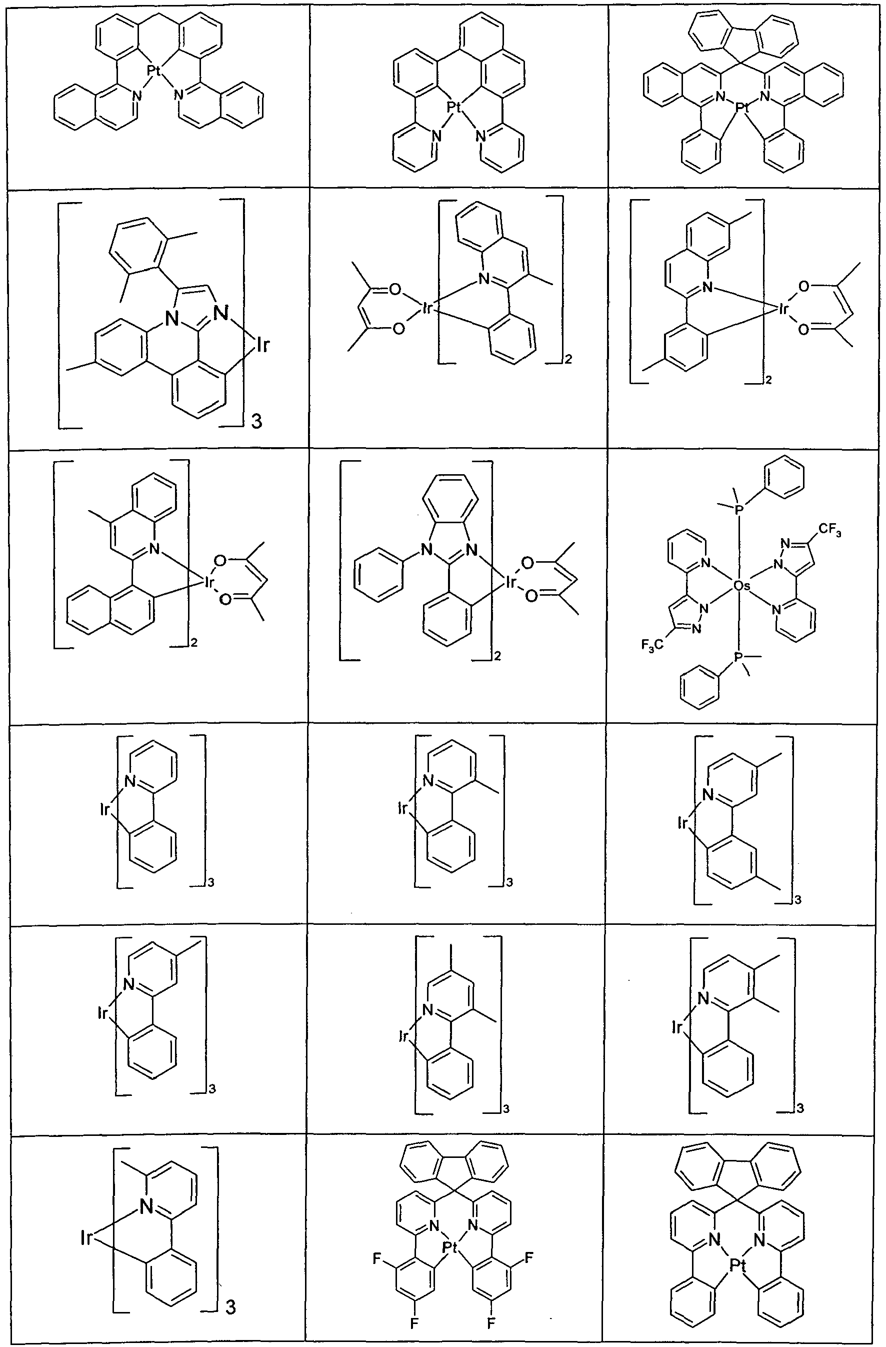

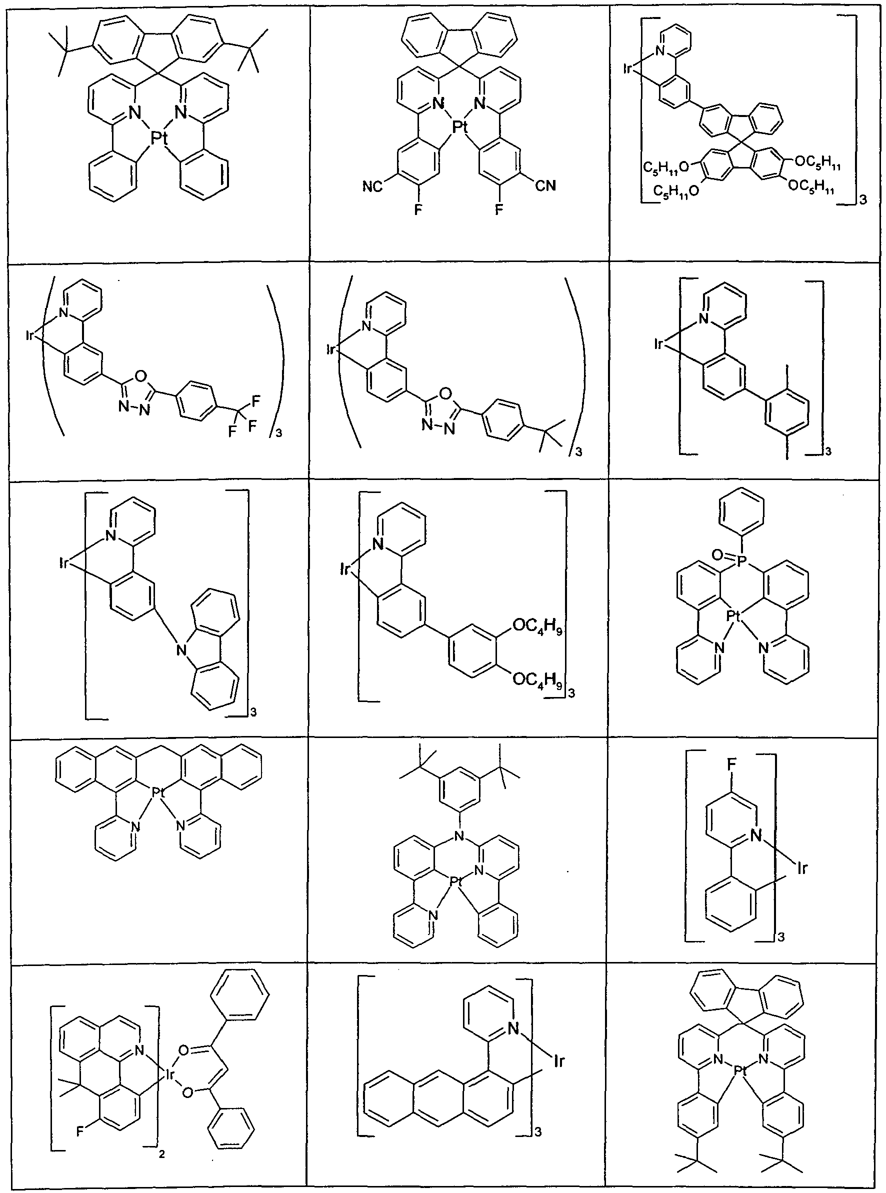

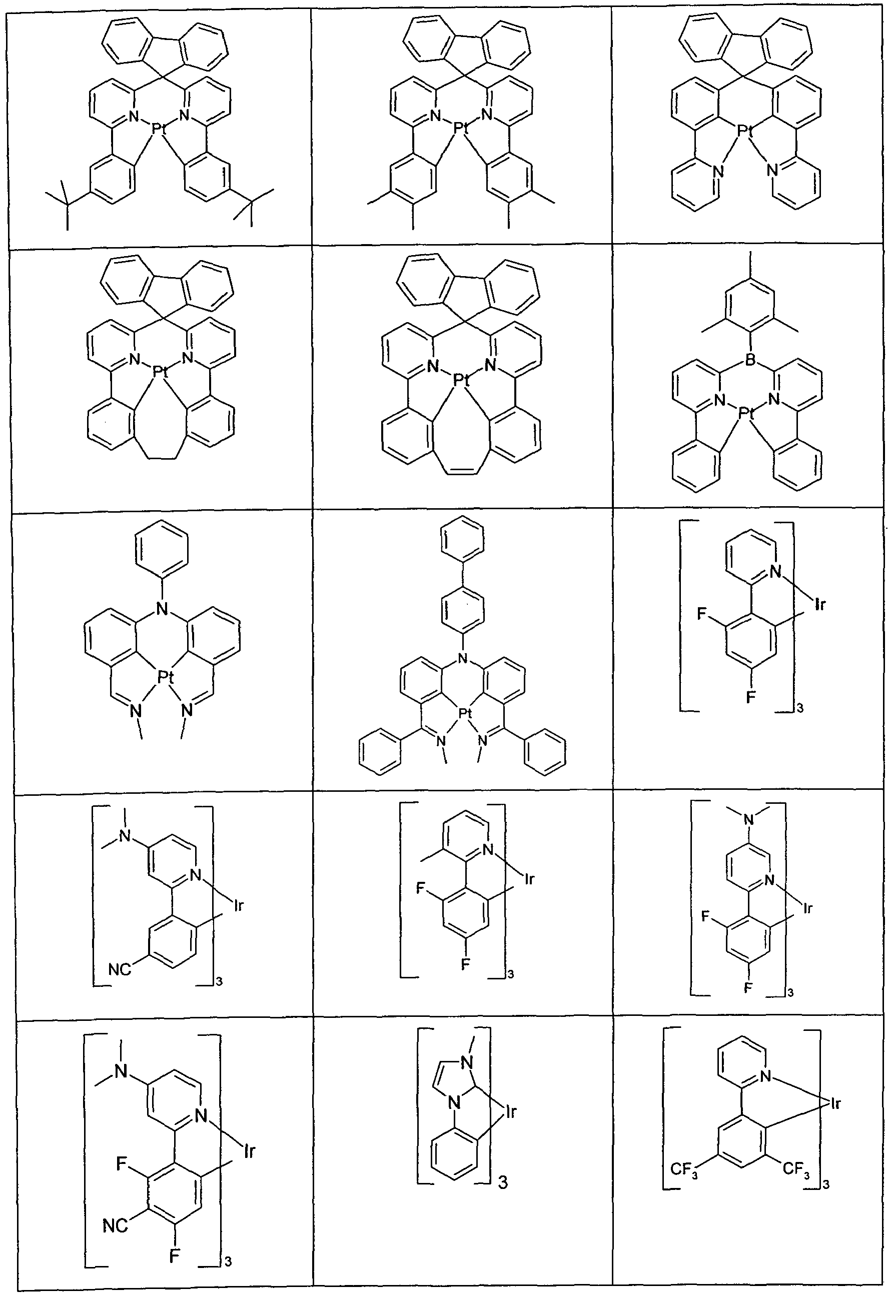

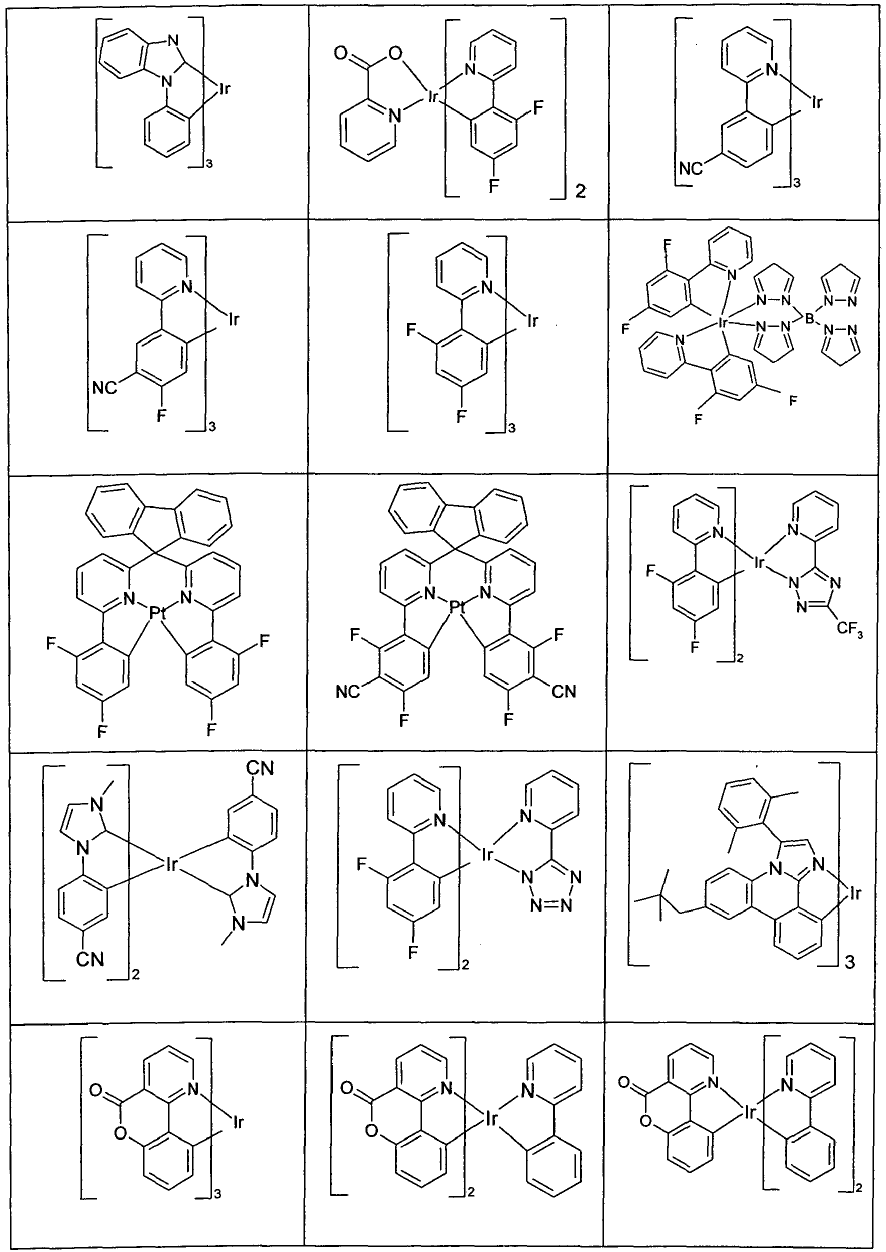

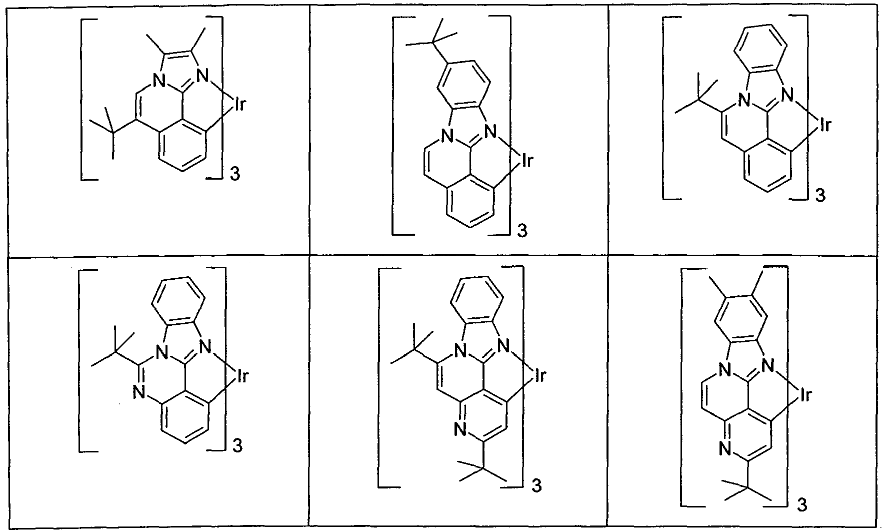

Bevorzugte fluoreszierende Emitter sind neben den Verbindungen der Formel (I) ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitter sind Indenofluorenamine bzw. -diamine, beispielsweise gemäß WO 2006/108497 oder WO 2006/122630,

Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und in WO 2013/185871 offenbarten Pyren-Arylamine. Ebenfalls bevorzugt sind die in WO 2014/037077offenbarten Benzoindenofluoren-Amine und die in EP 13000012.8 offenbarten Benzofluoren-Amine.

Als Matrixmaterialien, bevorzugt für fluoreszierende Emitter, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen.

Bevorzugte Matrixmaterialien für phosphoreszierende Emitter sind neben den Verbindungen der Formel (I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Außer den Verbindungen der Formel (I) sind bevorzugt als Lochtransportmaterialien, die in einer Lochtransport-, Lochinjektions- oder Elektronenblockierschicht in der erfindungsgemäßen Elektrolumineszenzvorrichtung verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938 und WO 2014/015935), Spiro-Dibenzopyran-Amine (z. B. gemäß WO 2013/083216) und Dihydroacridin-Derivate (z. B. gemäß WO 2012/150001).

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele (nicht anspruchsgemäße Beispiele sind mit # gekennzeichnet)

### A) Synthesebeispiele

### A-1) Beispiel 1: Synthese der Verbindungen (1-1) bis (1-13)

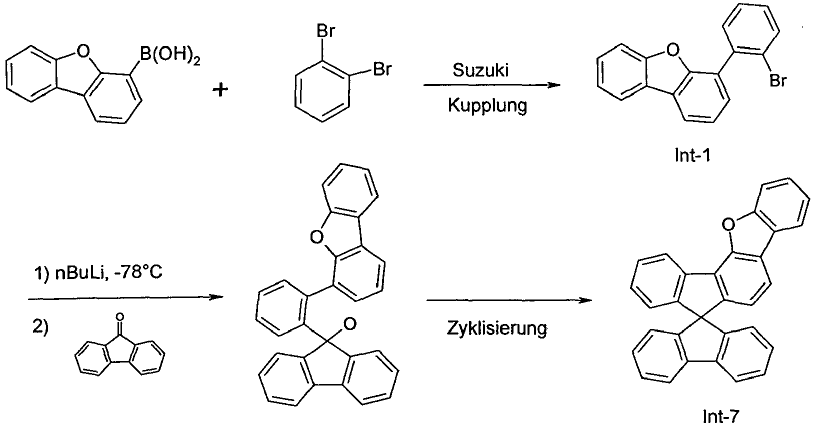

### Synthese von 4-(2-Bromo-phenyl)-dibenzofuran Int-1

100 g (462 mmol) Dibenzofuran-4-Boronsäure, 106 g (439 mmol) 1,2-Dibrombenzol und 10,7 g (9,2 mmol) Pd(Ph₃P)₄ werden in 980 mL Dioxan suspendiert. Zu dieser Suspension werden 979 mL Kaliumcarbonat Lösung 2 M langsam hinzugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockne eingeengt. Der Rückstand wird durch Chromatographie an Kieselgel gereinigt. Ausbeute: 87 g (270 mmol), 58% d. Th., Reinheit nach HPLC >98%.

### Synthese der Zwischenstufe Int-7

31 g (90 mmol) 4-(2-Bromo-phenyl)-dibenzofuran wird in 300 mL THF bei -78°C vorgelegt. Bei dieser Temperatur werden 40 mL BuLi (2M in Hexan) zugetropft. Nach 1 Stunde wird 16,9 g (94 mmol) Fluoren-9-on in 200 mL THF zugetropft. Der Ansatz wird über Nacht bei Raumtemperatur rühren gelassen, auf Eiswasser gegeben und mit Dichlormethan extrahiert. Die vereinigten organischen Phasen werden mit Wasser gewaschen und über Natriumsulfat getrocknet. Das Lösungsmittel wird im Vakuum entfernt und der Rückstand wird ohne weitere Aufreinigung mit 94 mL HCl und 1074 mL AcOH bei 100°C über Nacht unter Rückfluß erhitzt. Nach Erkalten saugt man vom ausgefallenen Feststoff ab, wäscht diesen einmal mit 100 ml Wasser, dreimal mit je 100 ml Ethanol und kristallisiert abschließend aus Heptan um. Ausbeute: 23.1 g (57 mmol), 58 %; Reinheit ca. 98 % nach ¹H-NMR.

Analog zu der beschriebenen Synthese von Verbindung Int-1 werden die folgenden Verbindungen hergestellt:

| | Edukt 1 | Edukt 2 | Produkt | Ausbeute |
|---|---|---|---|---|
| **Int-2** | 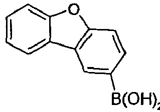 | 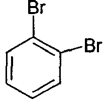 | 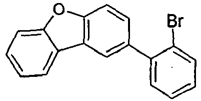 | 62% |
| **Int-3** | 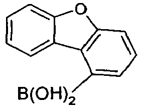 | 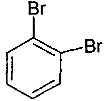 | 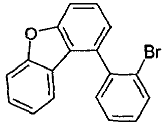 | 52% |
| **Int-4** | 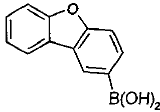 | 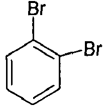 | 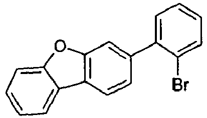 | 55% |
| **Int-5** | 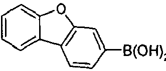 | 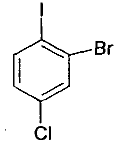 | 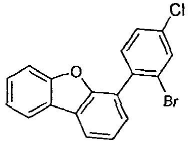 | 35% |
| **Int-6** | 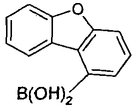 | 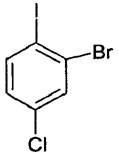 | 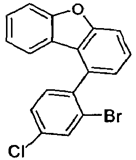 | 40% |
| **Int-6a** | 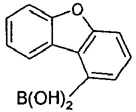 | 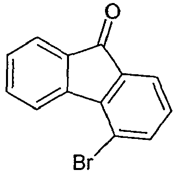 | 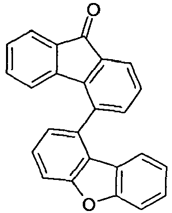 | 60% |
| **Int-6b** | 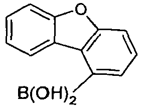 | 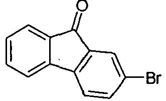 | 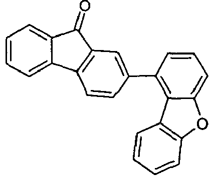 | 65% |
| **Int-6c** | 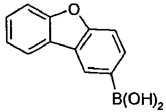 | 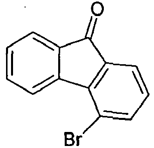 | 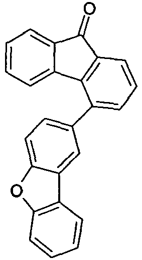 | 55% |
| **Int-6d** | 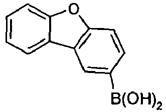 | 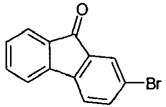 | 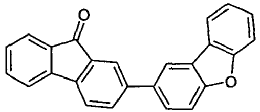 | 68% |
| **Int-6e** | 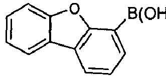 | 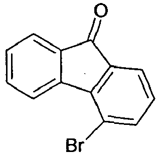 | 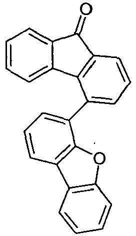 | 60% |
| **Int-6f** | 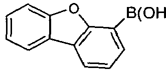 | 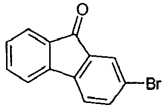 | 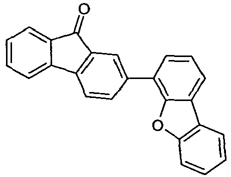 | 57% |
| **Int-6g** | 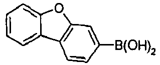 | 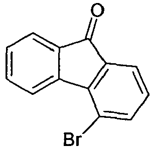 | 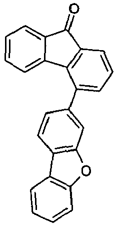 | 60% |
| **Int-6h** | 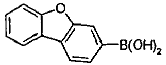 | 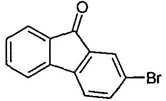 | 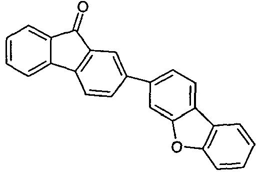 | 65% |

Analog zu der beschriebenen Synthese von Verbindung Int-7 werden die folgenden Verbindungen hergestellt:

| | Edukt 1 | Edukt 2 | Produkt | Ausbeute |
|---|---|---|---|---|
| **Int-8** | 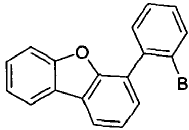 r | 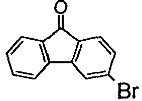 | 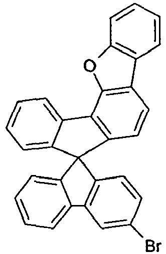 | 80% |
| | | [204 1-19-2] | | |
| **Int-9** | 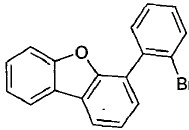 | 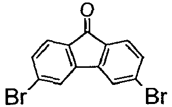 | 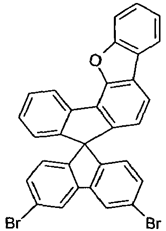 | 70% |
| | | [216312-73-1] | | |
| **Int-10** | 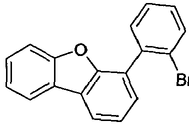 | 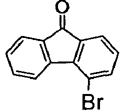 | 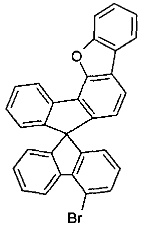 | 70% |
| | | [4269-17-4] | | |
| **Int-11** | 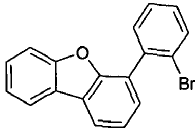 | 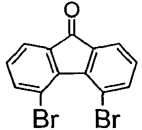 | 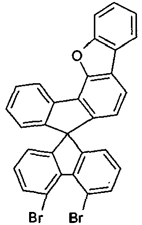 | 79% |
| | | [69414-97-7] | | |
| **Int-12** | 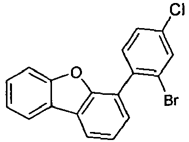 | 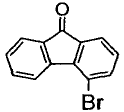 | 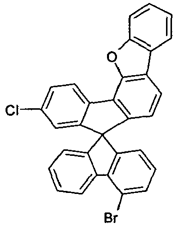 | 72% |
| | | [4269-17-4] | | |
| **Int-13** | 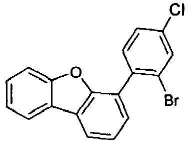 | 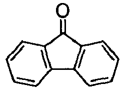 | 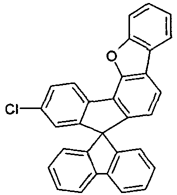 | 75% |
| | | [486-25-9] | | |
| **Int-14** | 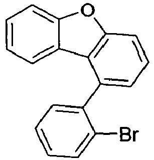 | 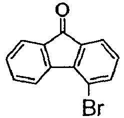 | 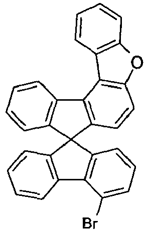 | 80% |
| | | [4269-17-4] | | |
| **Int-15** | 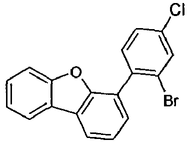 | 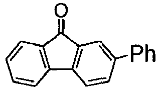 | 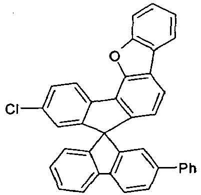 | 75% |
| | | [3096-49-9] | | |
| **Int-16** | 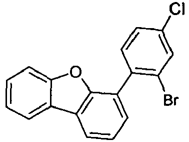 | 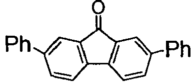 | 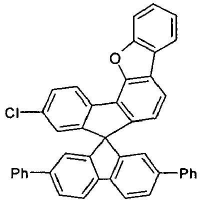 | 73% |
| | | [115033-91-5] | | |
| **Int-17** | 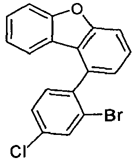 | 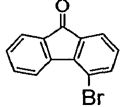 | 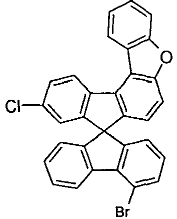 | 70% |
| | | [4269-17-4] | | |
| **Int-18** | 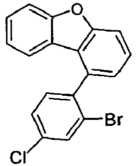 | 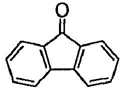 | 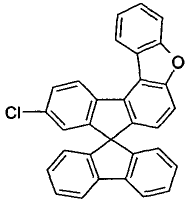 | 75% |
| | | [486-25-9] | | |
| **Int-19** | 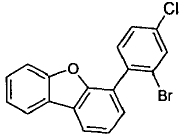 | 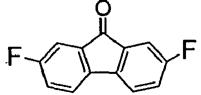 | 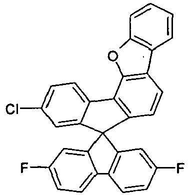 | 65% |
| | | [24313-53-9] | | |
| **Int-20** | 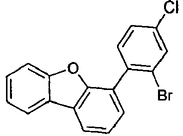 | 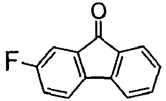 | 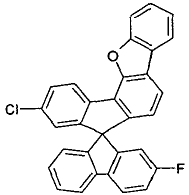 | 58% |
| | | [343-01-1] | | |
| **Int-21** | 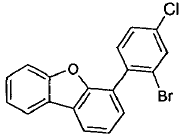 | 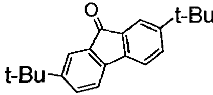 | 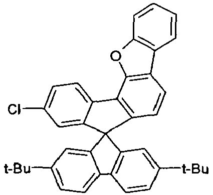 | 80% |
| | | [58775-13-6] | | |
| **Int-22** | 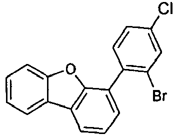 | 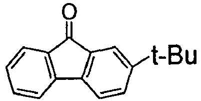 | 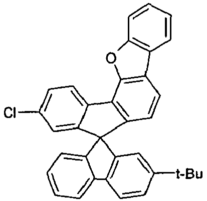 72% | 72% |
| | | [58775-11-4] | | |
| **Int-23** | 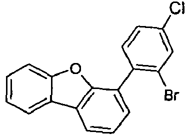 | 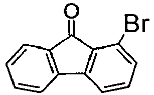 | 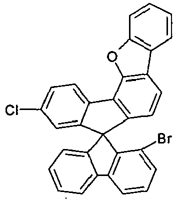 | 75% |
| | | [36804-63-4] | | |
| **Int-24** | 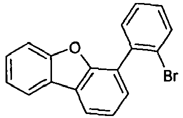 | 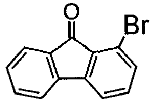 | 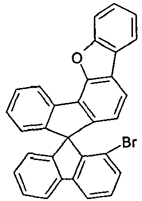 | 67% |
| | | [36804-63-4] | | |
| **Int-24a** | 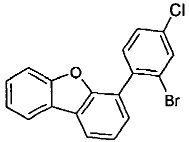 | 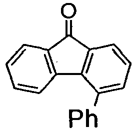 | 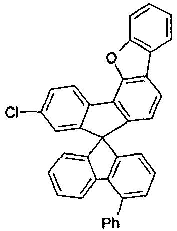 | 75% |
| | | [4269-14-1] | | |
| **Int-24b** | 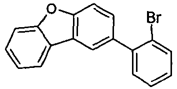 | 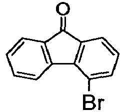 | 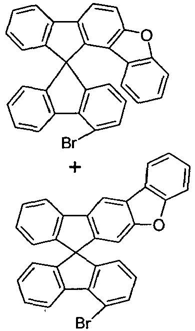 | 70% |
| **Int-24c** | 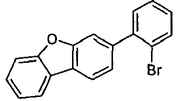 | 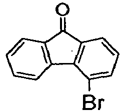 | 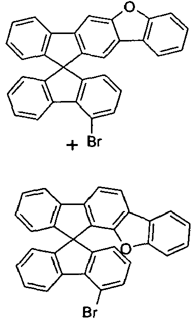 | 65% |
| **Int-24d** | 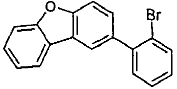 | 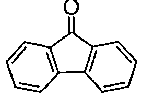 | 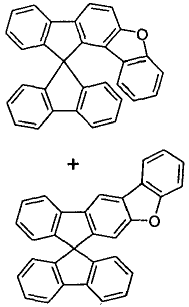 | 75% |
| **Int-24e** | 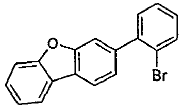 | 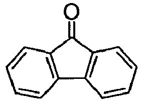 | 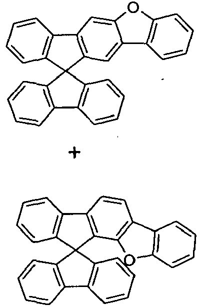 | 80% |
| **Int-24f** | 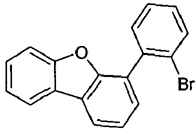 | 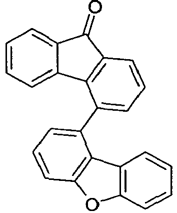 | 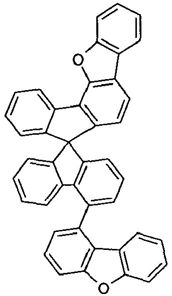 | 70% |
| **Int-24g** | 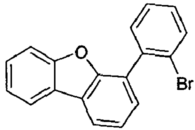 | 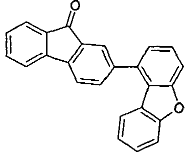 | 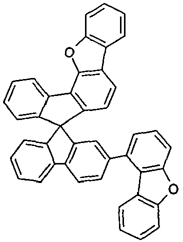 | 65% |
| **Int-24h** | 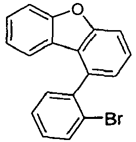 | 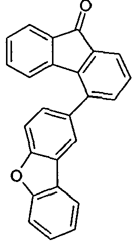 | 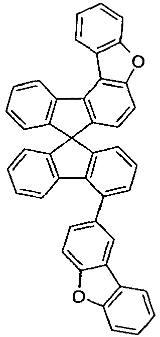 | 78% |
| **Int-24i** | 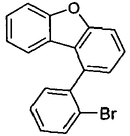 | 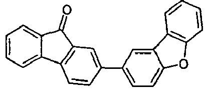 | 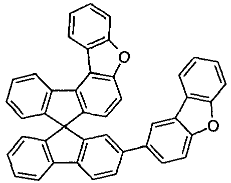 | 62% |
| **Int-24j** | 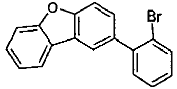 | 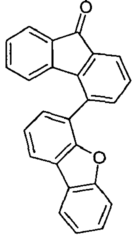 | 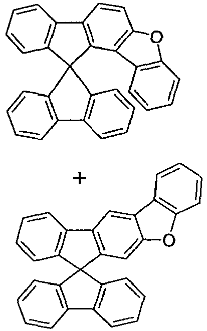 | 65% |
| **Int-24k** | 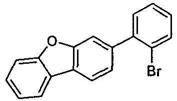 | 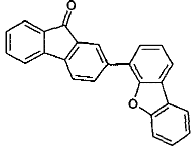 | 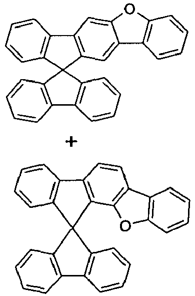 | 70% |
| **Int-24l** | 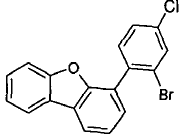 | 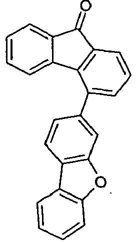 | 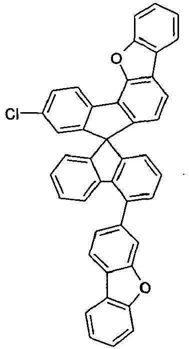 | 81% |
| **Int-24m** | 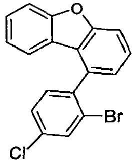 | 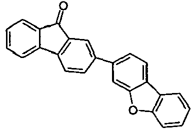 | 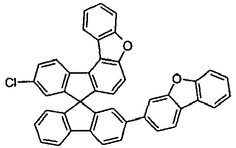 | 75% |

### Synthese der Verbindung (1-1)

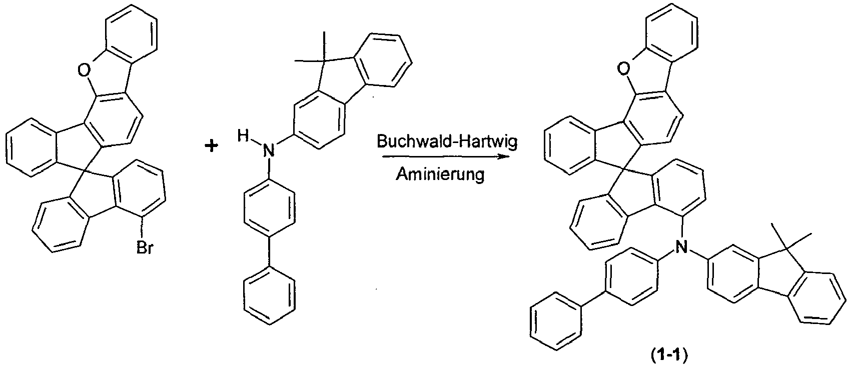

11,5 g (31,5 mmol) Biphenyl-4-yl-(9,9-dimethyl-9H-fluoren-2-yl)-amin und 14,0 g (28,8 mol) des Bromo-Spiroderivats werden in 320 mL Toluol gelöst. Die Lösung wird entgast und mit N₂ gesättigt. Danach wird sie mit 6,8 mL (2,88 mmol) einer 10%igen Tri-tert-Butylphosphin Lösung und 1,32 g (1,44 mmol) Pd₂(dba)₃ versetzt und anschließend werden 9,5 g Natrium-tert-butylat (86,5 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%(HPLC). Die Ausbeute von Verbindung (**1-1**) beträgt 16,5 g (75% der Theorie).

### Synthese der Verbindungen (1-2) bis (1-16)

Analog zu der in Beispiel 1 beschriebenen Synthese von Verbindung (**1-1**) werden auch die folgenden Verbindungen hergestellt.

| | Edukt 1 | Edukt 2 | Produkt | Ausbeute |
|---|---|---|---|---|
| **1-2** | 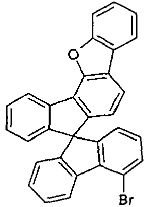 | 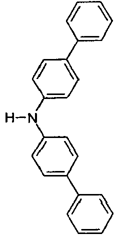 | 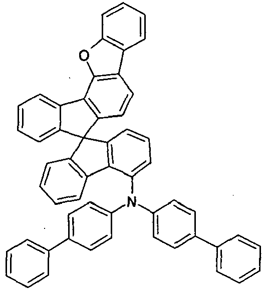 | 78 % |
| | | [102113-98-4] | | |
| **1-3** | 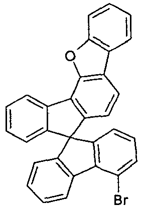 | 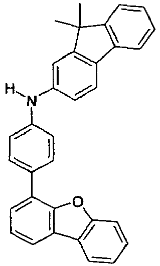 | 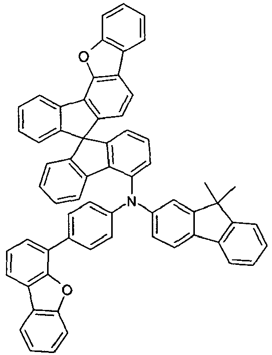 | 82% |
| **1-4** | 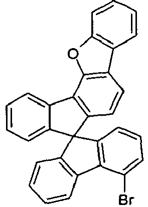 | 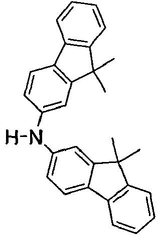 | 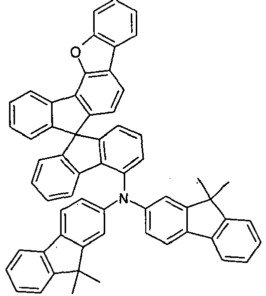 | 88% |
| | | [500717-23-7] | | |
| **1-5** | 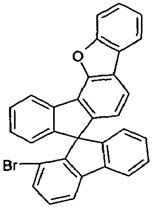 | 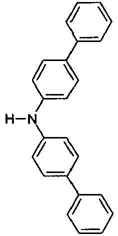 | 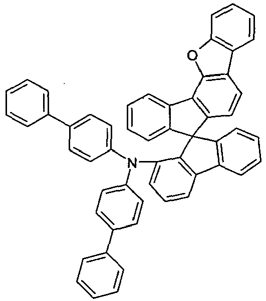 | 67% |
| | | [102113-98-4] | | |
| **1-6** | 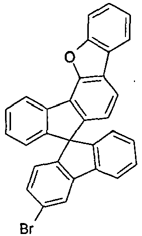 | 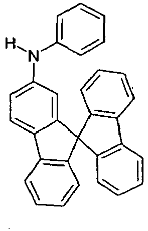 | 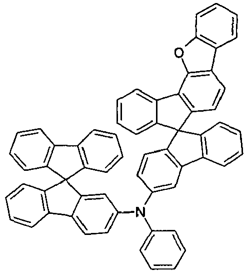 | 76% |
| **1-7** | 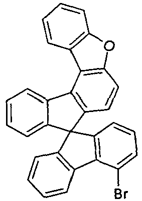 | 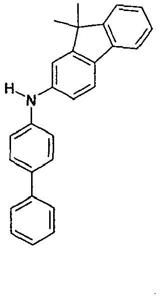 | 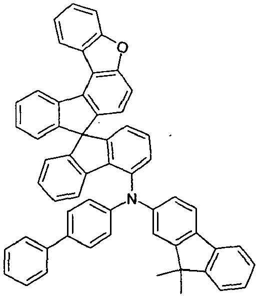 | 80% |
| **1-8** | 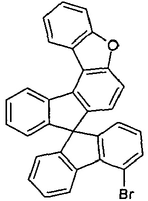 | 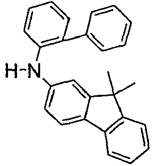 | 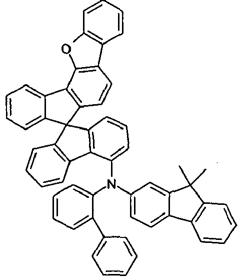 | 78% |
| | | [1198395-24-2] | | |
| **1-9** | 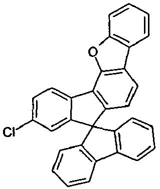 | 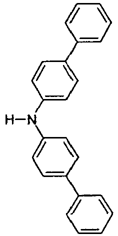 | 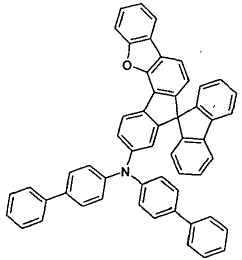 | 72 % |
| | | [102113-98-4] | | |
| **1-10** | 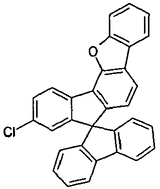 | 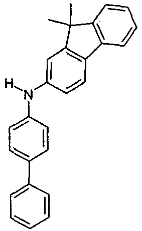 | 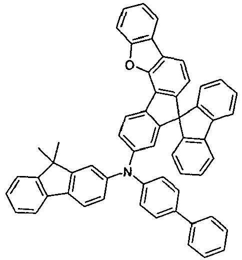 | 83% |
| **1-11** | 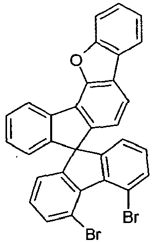 | 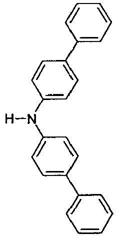 | 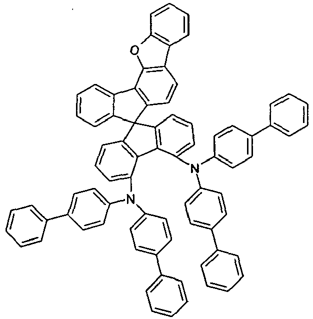 | 75% |
| | | [102113-98-4] (2 Äq.) | | |
| **1-12** | 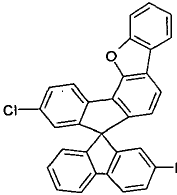 | 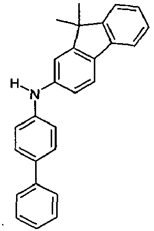 | 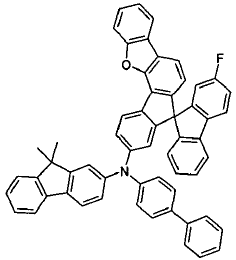 | 70% |
| **1-13** | 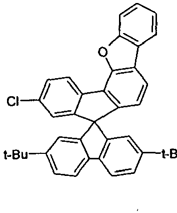 | 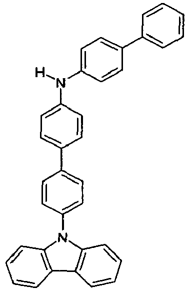 | 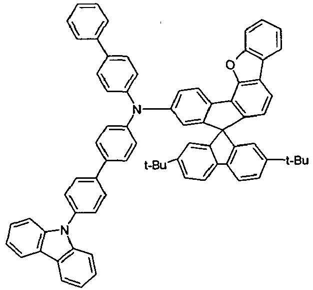 | 81% |
| **1-14** | 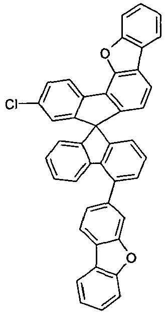 | 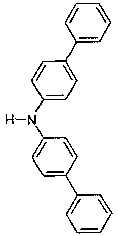 | 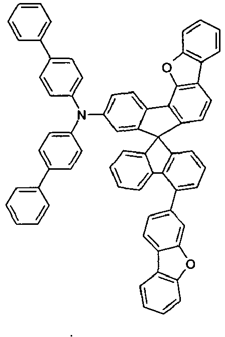 | 65% |
| **1-15** | 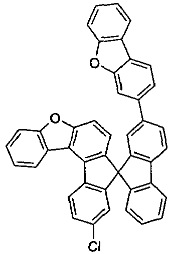 | 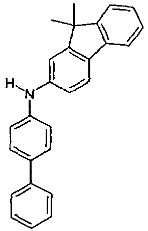 | 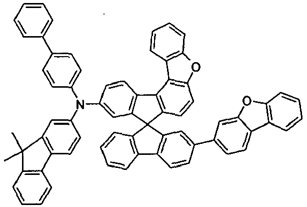 | 55% |
| **1-16** | 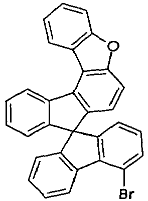 | 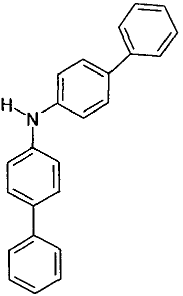 | 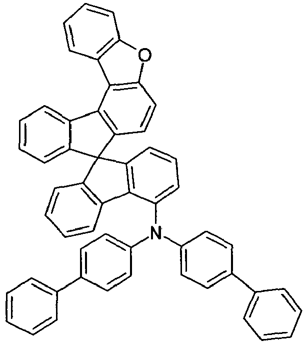 | 73% |

### A-2) Beispiel 2: Synthese der Verbindungen (2-2) bis (2-4)

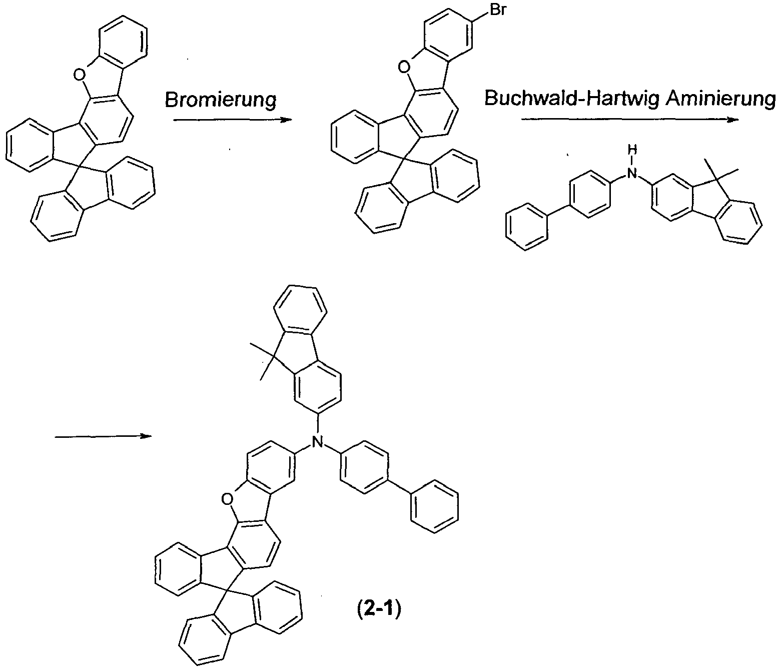

15,0 g (36,9 mmol) der Ausgangsverbindung werden in 150 mL Acetonitril gelöst und bei Raumtemperatur portionsweise mit 5,2 g (29 mmol) N-Bromsuccinimid versetzt. Nach vollständiger Umsetzung werden Wasser und Essigsäureethylester dazu gegeben, die organische Phase abgetrennt, getrocknet und eingeengt. Das Rohprodukt wird anschließend mit MeOH/Heptan (1:1) mehrfach heiß ausgerührt. Ausbeute: 14,3 g (80%) des Brom-Spiroderivats Int-25.

Analog dazu werden die folgenden bromierten Verbindungen hergestellt:

| | Edukt 1 | Bromierungs-Reagenz | Produkt | Ausbeute |
|---|---|---|---|---|
| **Int-26** | 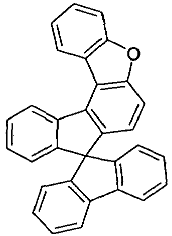 | NBS | 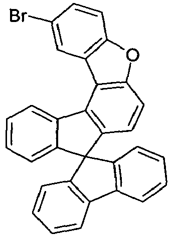 | 78% |
| **Int-27** | 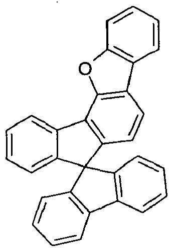 | 1) nBuLi, -78°C | 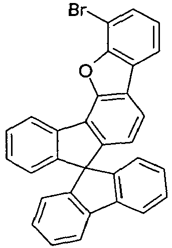 | 65% |
| | | 2) BrCH₂-CH₂Br | | |
| **Int-27a** | 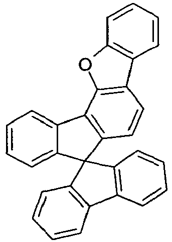 | 1) nBuLi, -78°C | 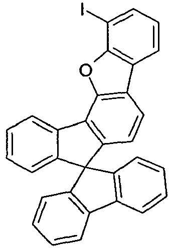 | 75% |
| | | 2) I₂ | | |

### Synthese der Verbindungen (2-2) bis (2-5)

Analog zu der in Beispiel 1 beschriebenen Synthese von Verbindung (**1-1**) werden auch die folgenden Verbindungen (**2-2**) bis (**2-5**) hergestellt.

| | Edukt 1 | Edukt 2 | Produkt | Ausbeute |
|---|---|---|---|---|
| **2-2** | 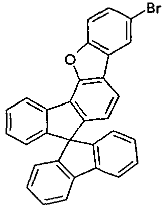 | 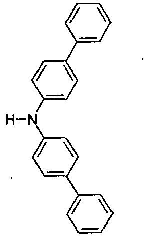 | 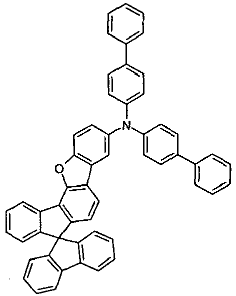 | 82% |
| | | [102113-98-4] | | |
| **2-3** | 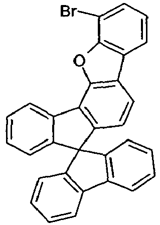 | 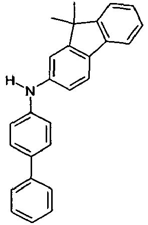 | 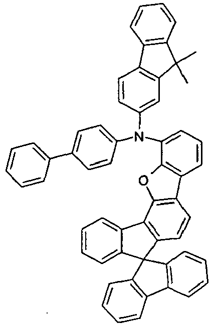 | 69% |
| **2-4** | 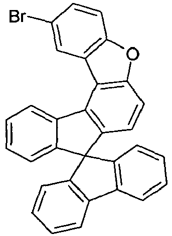 | 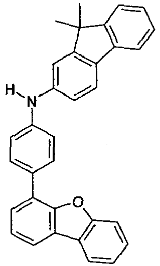 | 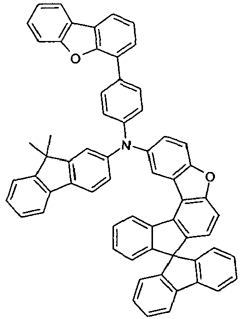 | 88% |
| **2-5** | 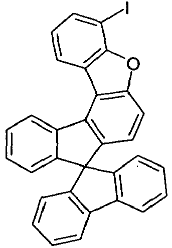 | 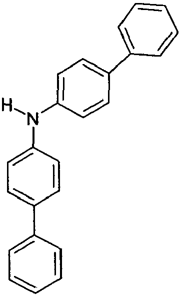 | 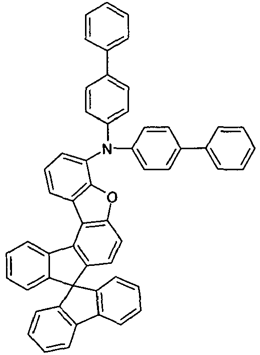 | 50% |

### A-3) Beispiel 3: Synthese der Verbindungen 3-1 bis 3-3

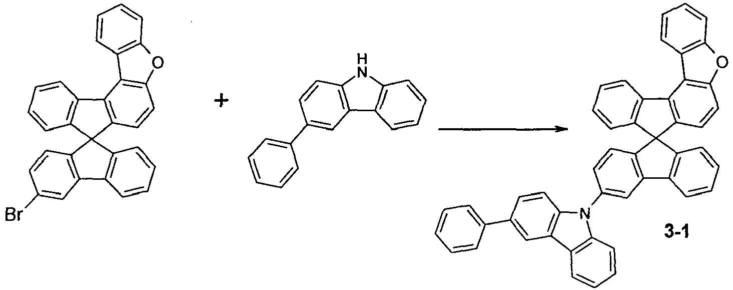

20.0 g (40,1 mmol) Brom-Derivat, 9,7 g (40,1 mmol) 3-Phenyl-9-H-carbazol und 24 g Rb₂CO₃ werden in 250 mL p-Xylol suspendiert. Zu dieser Suspension werden 0.95 g (4,2 mmol) Pd(OAc)₂ und 12.6 ml einer 1M Tri-tert-butylphosphin Lösung gegeben. Die Reaktionsmischung wird 36 h unter Rückfluss erhitzt. Nach Erkalten wird die organische Phase abgetrennt, dreimal mit 150 mL Wasser gewaschen und anschließend zur Trockne eingeengt. Der Rückstand wird mit Toluol heiß extrahiert, aus Toluol dreimal umkristallisiert und abschließend im Hochvakuum sublimiert. Man erhält 15,9 g (24,1 mmol) entsprechend 60 % der Theorie. Die Reinheit beträgt 99.9 %.

### Synthese der Verbindungen (3-2) bis (3-4)

Analog zu der in Beispiel 1 beschriebenen Synthese von Verbindung (**3-1**) werden auch die folgenden Verbindungen (**3-2**) und (**3-3**) hergestellt.

Analog werden die folgenden Verbindungen erhalten:

| | Edukt 1 | Edukt 2 | Produkt | Ausbeute |
|---|---|---|---|---|
| **3-2** | 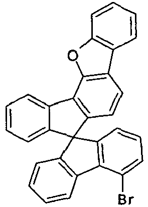 | 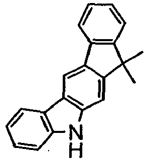 | 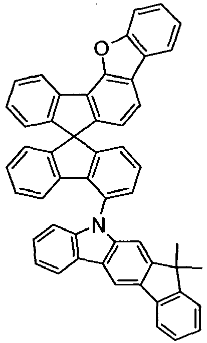 | 50% |
| | | 1257220-47-5 | | |
| **3-3** | 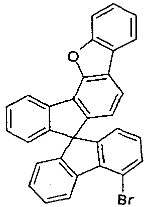 | 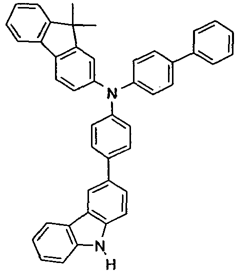 | 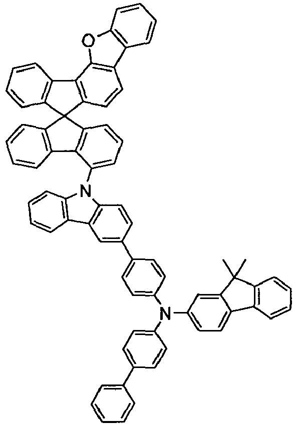 | 45% |
| | | 1427738-11-1 | | |

### A-3a) Synthese von Intermediaten für Verbindungen unter A-4)

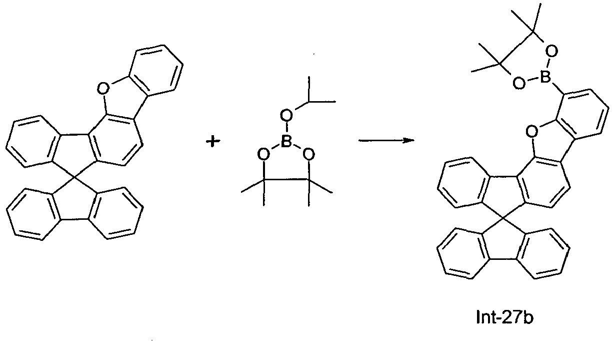

30 g (74 mmol) Dibenzospirofluoren werden in 400 mL THF bei -20°C vorgelegt. Bei dieser Temperatur werden 49 mL BuLi (2M in Hexan) zugetropft. Nach 4 Stunden werden 33 mL (148 mmol) Isopropoxytetramethyldioxaborolan zugetropft. Der Ansatz wird über Nacht bei Raumtemperatur rühren gelassen. Nach vollständiger Umsetzung werden Wasser und Essigsäureethylester dazu gegeben, die organische Phase abgetrennt, getrocknet und eingeengt. Der Rückstand wird durch Chromatographie an Kieselgel gereinigt. Ausbeute: 31 g (59 mmol), 80 % d. Th., Reinheit nach HPLC >98%.

| | Edukt 1 | Borylierungs-Reagenz | Produkt | Ausbeute |
|---|---|---|---|---|
| **Int-27c** | 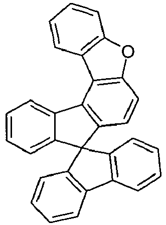 | 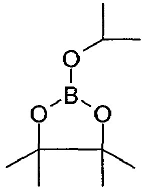 | 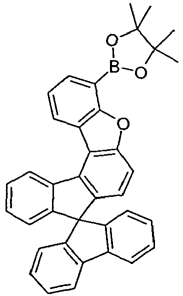 | 85% |
| **Int-27d** | 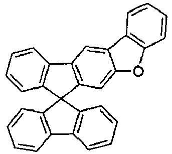 | 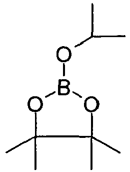 | 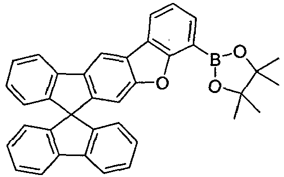 | 80% |
| **Int-27e** | 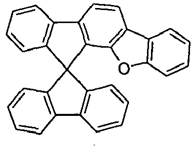 | 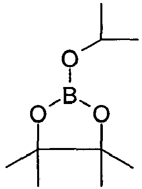 | 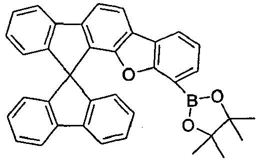 | 75% |

### A-4) Beispiel 4: Synthese der Verbindungen 4-1 bis 4-13

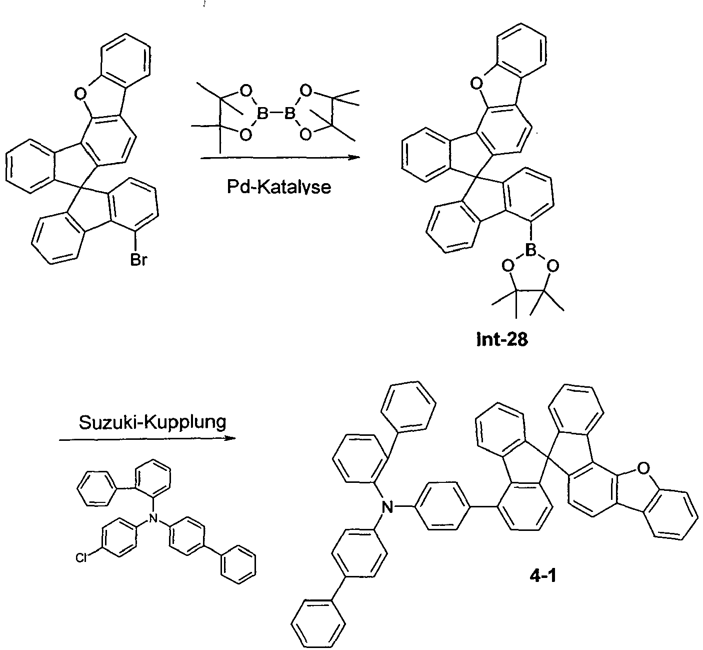

### Spirofluoren-boronester-Derivat Int-28

50 g (103 mmol) des Spirofluoren-bromderivats, 32 g (123 mmol) Bis(pinacolato)diboran und 30 g (309 mmol) Kalliumacetat werden in 800 ml Dioxan suspendiert. Zu dieser Suspension werden 2.5 g (3.09 mmol) 1,1-Bis(diphenylphosphino)ferrocen-dichloropalladium(II) Komplex mit DCM gegeben. Die Reaktionsmischung wird 16 h unter Rückfluss erhitzt. Nach Erkalten wird die organische Phase abgetrennt, dreimal mit 400 mL Wasser gewaschen und anschließend zur Trockne eingeengt. Der Rückstand wird aus Toluol umkristallisiert (52 g, 95% Ausbeute).

Analog dazu werden folgende Verbindungen hergestellt:

| | Edukt 1 | Produkt | Ausbeute |
|---|---|---|---|
| **Int-29** | 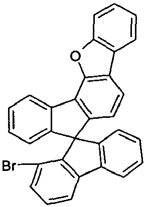 | 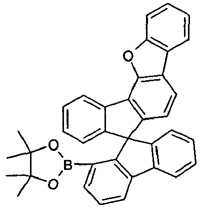 | 90% |
| **Int-30** | 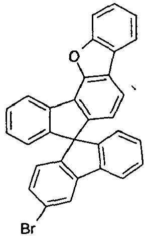 | 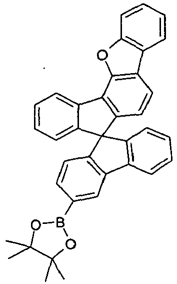 | 80% |
| **Int-31** | 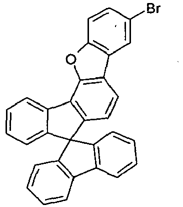 | 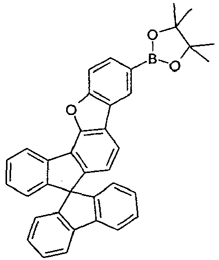 | 88% |
| **Int-32** | 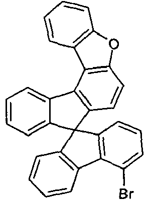 | 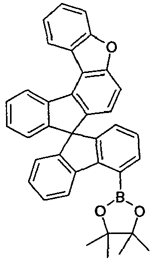 | 88% |
| **Int-33** | 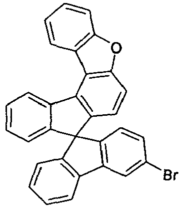 | 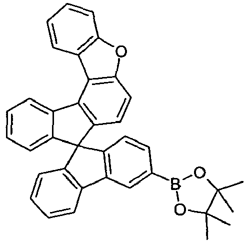 | 91% |
| **Int-34** | 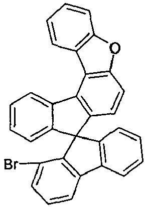 | 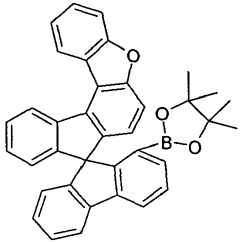 | 85% |
| **Int-34a** | 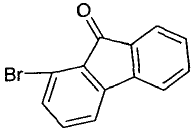 | 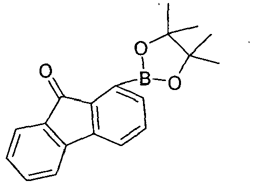 | 80% |
| **Int-34b** | 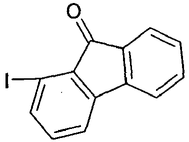 | 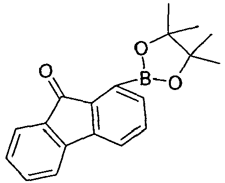 | 85% |

### Biphenyl-2-yl-biphenyl-4-yl-(4-chloro-phenyl)-amin Int-35

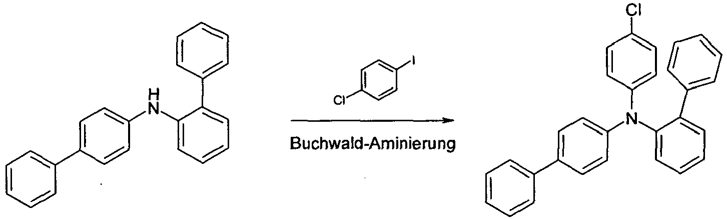

23,8 g Biphenyl-2-yl-biphenyl-4-yl-amin (74 mmol) und 21,2 g 4-Chloriodbenzol-Bromofluoren (89 mmol) werden in 500 mL Toluol gelöst. Die Lösung wird entgast und mit N₂ gesättigt. Danach wird sie mit 3 mL (3 mmol) einer Tri-tert-Butylphosphin 1M-Lösung und 0,33 g (1,48 mmol) Palladium(II)acetat versetzt und anschließend werden 10,7 g Natrium-tert-butylat (111 mmol) zugegeben. Die Reaktionsmischung wird 12 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 29 g (90% d. Th).

Analog dazu werden folgende Verbindungen hergestellt:

| | Edukt 1 | Edukt 2 | Produkt | Ausbeute |
|---|---|---|---|---|
| **Int-36** | 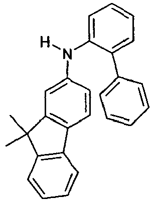 | 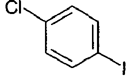 | 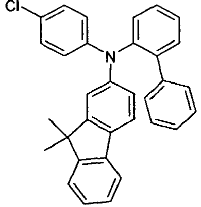 | 78 % |
| **Int-37** | 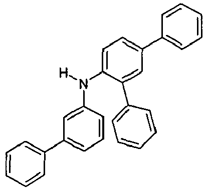 | 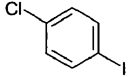 | 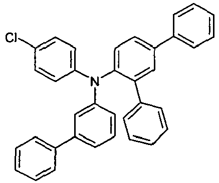 | 80% |
| **Int-38** | 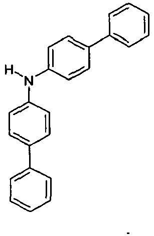 | 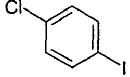 | 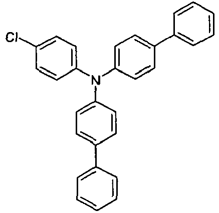 | 81% |
| **Int-39** | 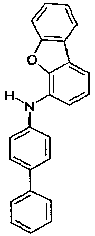 | 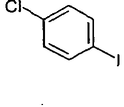 | 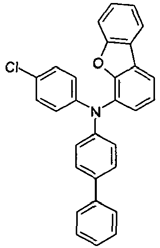 | 92% |
| **Int-40** | 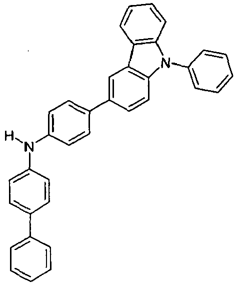 | 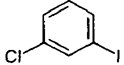 | 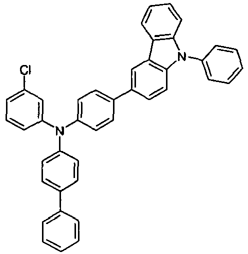 | 85% |
| **Int-41** | 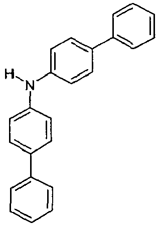 | 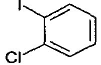 | 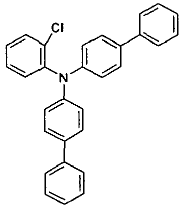 | 75% |

### Synthese der Verbindung (4-1)

24,6 g (46,3 mmol) Spirofluoren-pinacolboronester-Derivat und 20,0 g (46,3 mmol) Chlor-Derivat werden in 300 mL Dioxan und 14.1 g Caesiumfluorid (92,6 mmol) suspendiert. Zu dieser Suspension werden 4,1 g (5,56 mmol) Palladium dichlorid-bis(tricyclohexylphosphin) gegeben, und die Reaktionsmischung wird 24 h unter Rückfluss erhitzt. Nach Erkalten wird die organische Phase abgetrennt, über Kieselgel filtriert, dreimal mit 100 mL Wasser gewaschen und anschließend zur Trockne eingeengt. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 29,7 g (80% d. Th).

### Synthese der Verbindungen (4-2) bis (4-10) und Int-41a bis Int-41c

Analog zu der in Beispiel 1 beschriebenen Synthese von Verbindung (4-1) werden auch die folgenden Verbindungen hergestellt.

| | Edukt 1 | Edukt 2 | Produkt | Ausbeute |
|---|---|---|---|---|
| **4-2** | 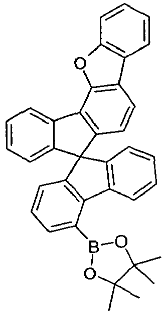 | 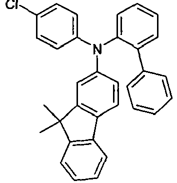 | 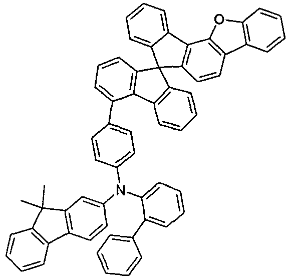 | 78% |
| **4-3** | 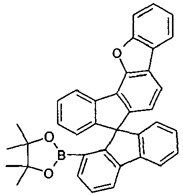 | 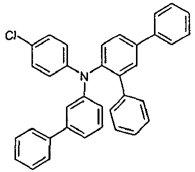 | 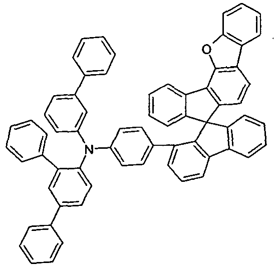 | 71% |
| **4-4** | 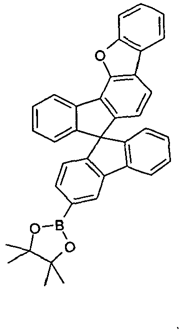 | 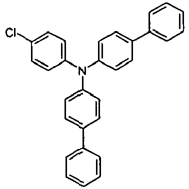 | 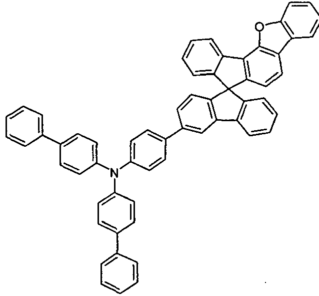 > | 82% |
| **4-5** | 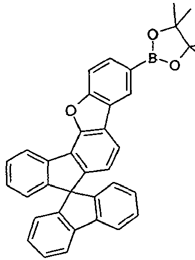 | 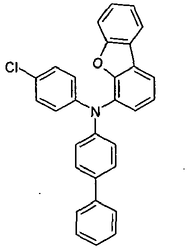 | 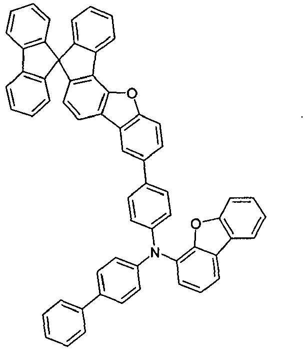 | 89% |
| **4-6** | 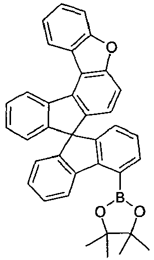 | 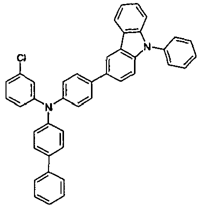 | 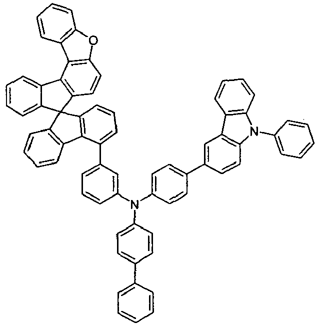 | 69% |
| **4-7** | 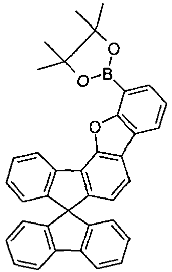 | 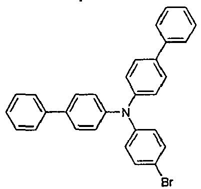 | 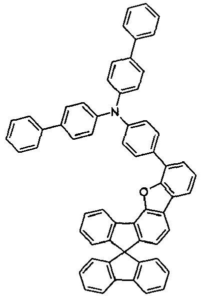 | 55% |
| **4-8** | 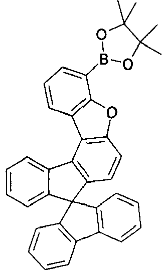 | 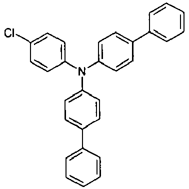 | 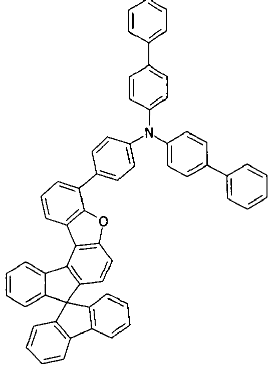 | 63% |
| **4-9 #** | 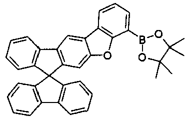 | 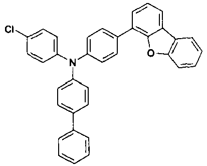 | 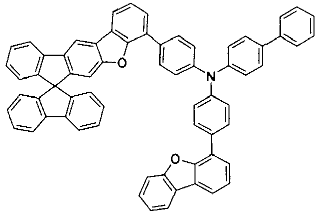 | 72% |
| **4-10 #** | 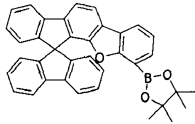 | 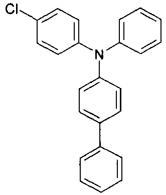 | 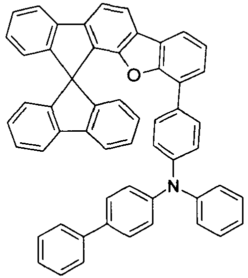 | 57% |
| **Int-41a** | 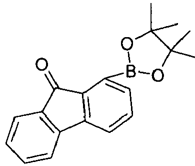 | 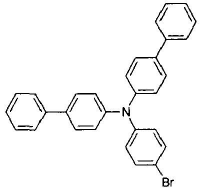 | 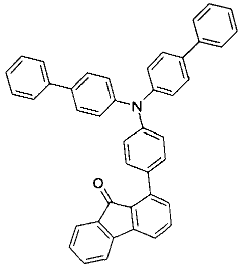 | 75% |
| **Int-41b** | 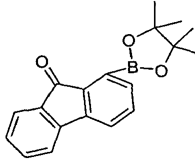 | 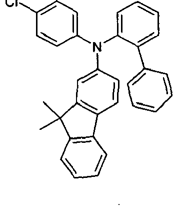 | 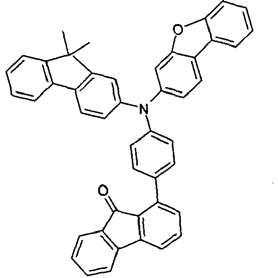 | 80% |
| **Int-41c** | 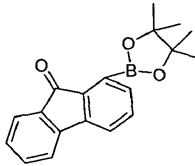 | 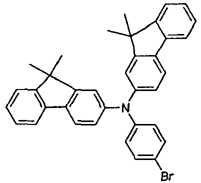 | 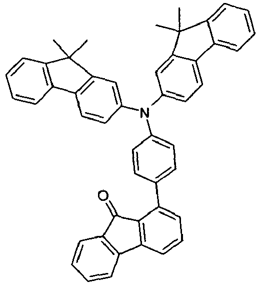 | 82% |
| **4-11** | 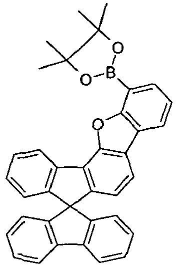 | 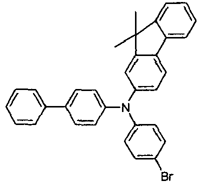 | 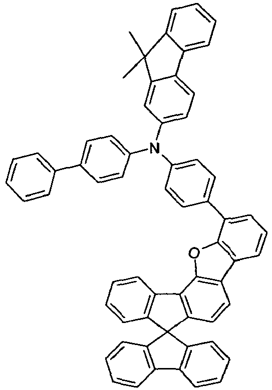 50% | 50% |

### A-5) Synthese von Verbindungen 5-1 bis 5-8

### Synthese der Intermediate Int-42 bis Int-47

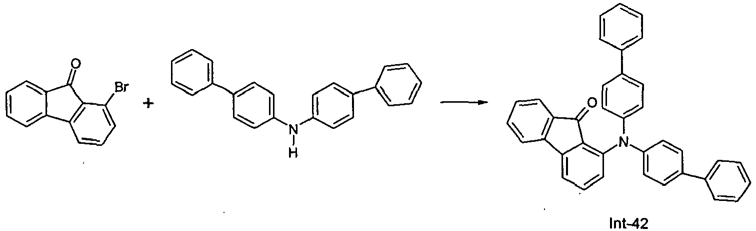

27 g (85 mmol) bis-Biphenylamin und 22,0 g (85m mol) des 1-Bromfluorenon werden in 170 mL Toluol gelöst. Die Lösung wird entgast und mit N₂ gesättigt. Danach wird sie mit 4 mL (1,7 mmol) einer 10%igen Tri-tert-Butylphosphin Lösung und 0,2 g (0,89 mmol) Pd(AcO)₂ versetzt und anschließend werden 12,2 g Natrium-tert-butylat (127 mmol) zugegeben. Die Reaktionsmischung wird 12 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Reinheit beträgt 99% (NMR). Die Ausbeute beträgt 34 g (80% der Theorie).

Analog werden die folgenden Verbindungen hergestellt:

| | Edukt 1 | Edukt 2 | Produkt | Ausbeute |
|---|---|---|---|---|
| Int-43 | 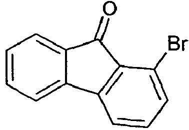 | 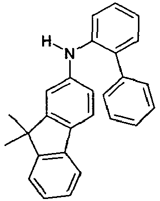 | 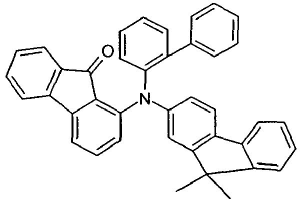 | 67% |
| Int-44 | 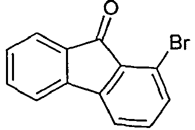 | 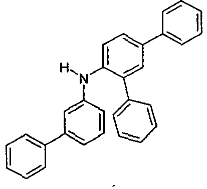 | 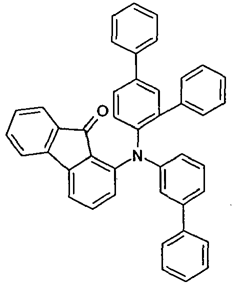 | 75% |
| Int-45 | 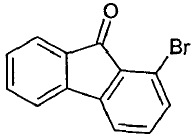 | 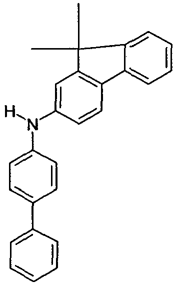 | 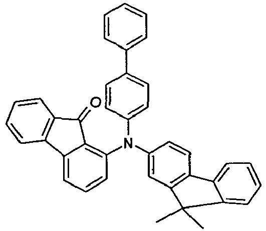 | 68% |
| Int-46 | 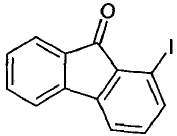 | 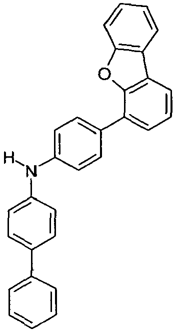 | 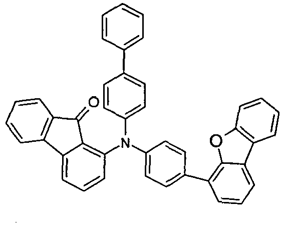 | 80% |
| Int-47 | 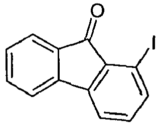 | 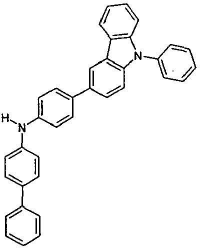 | 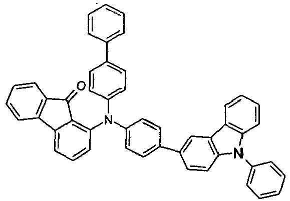 | 78% |
| Int-48 | 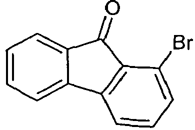 | 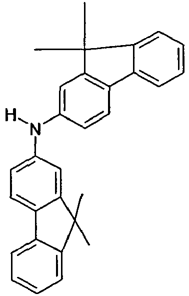 | 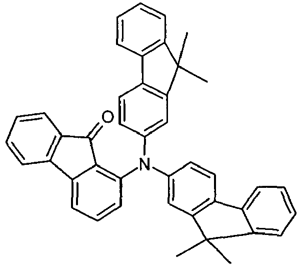 | 76% |

### Synthese der Verbindungen 5-1 bis 5-8

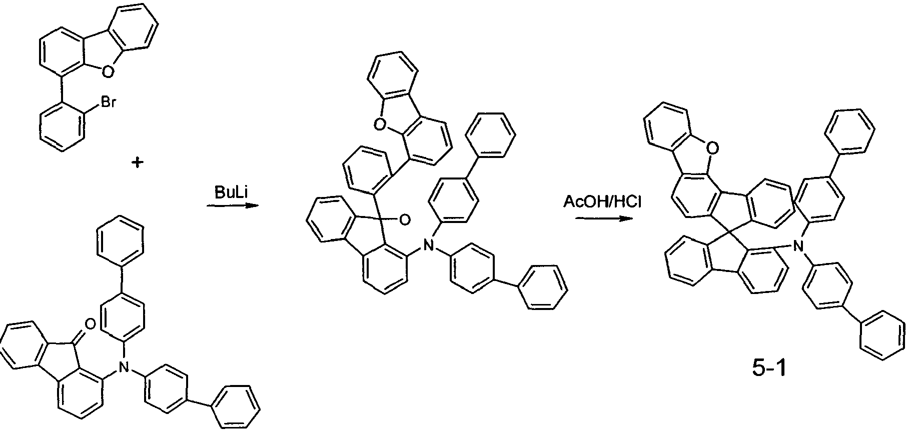

16 g (51 mmol) 4-(2-Bromo-phenyl)-dibenzofuran wird in 80 mL THF bei -78°C vorgelegt. Bei dieser Temperatur werden 13 mL BuLi (2M in Hexan) zugetropft. Nach 1 Stunde wird 24,5 g (47 mmol) Fluoren-9-on in 200 mL THF zugetropft. Der Ansatz wird über Nacht bei Raumtemperatur rühren gelassen, auf Eiswasser gegeben und mit Dichlormethan extrahiert. Die vereinigten organischen Phasen werden mit Wasser gewaschen und über Natriumsulfat getrocknet. Das Lösungsmittel wird im Vakuum entfernt und der Rückstand wird ohne weitere Aufreinigung mit 94 mL HCl und 1074 mL AcOH bei 100°C über Nacht unter Rückfluss erhitzt. Nach Erkalten saugt man vom ausgefallenen Feststoff ab, wäscht diesen einmal mit 100 ml Wasser, dreimal mit je 100 ml Ethanol, kristallisiert aus Heptan um und sublimiert abschließend im Hochvakuum. Ausbeute: 8,8 g (12 mmol), 59 %; Reinheit ca. 99,9 % nach HPLC.

Analog werden die folgenden Verbindungen hergestellt:

| | Edukt 1 | Edukt 2 | Produkt | Ausbeute |
|---|---|---|---|---|
| **5-2** | 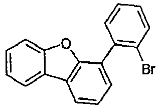 | 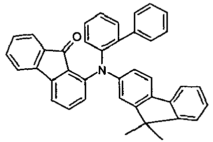 | 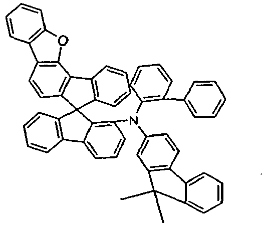 | 55% |
| **5-3** | 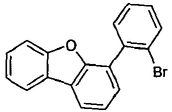 | 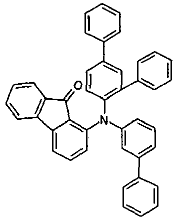 | 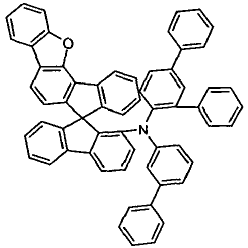 | 67% |
| **5-4** | 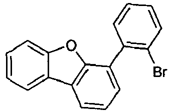 | 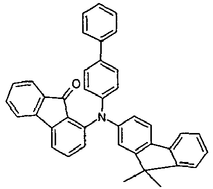 | 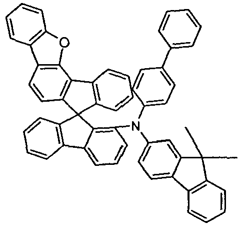 | 70% |
| **5-5** | 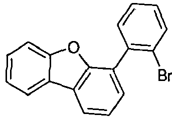 | 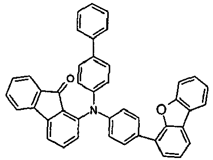 | 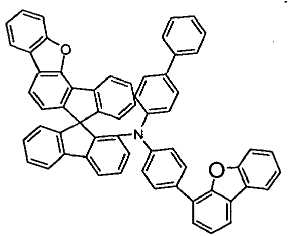 | 49% |
| **5-6** | 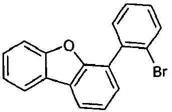 | 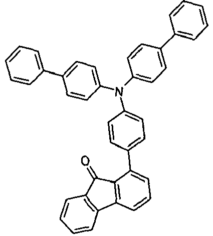 | 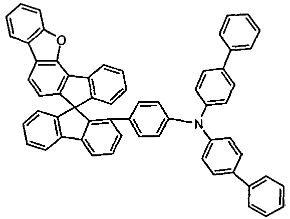 | 60% |
| **5-7** | 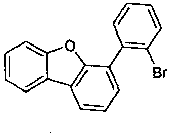 | 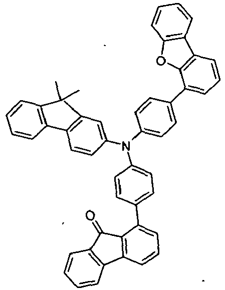 | 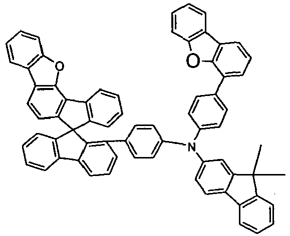 | 58% |
| **5-8** | 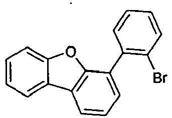 | 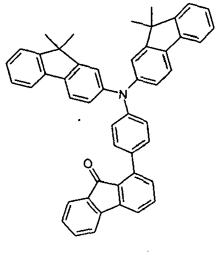 | 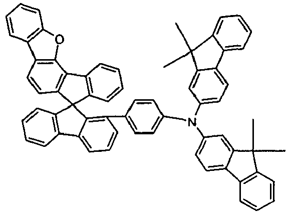 | 65% |
| **5-9** | 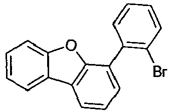 | 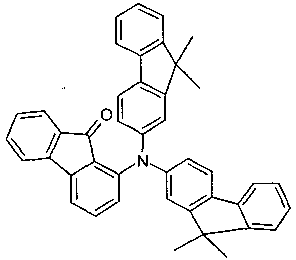 | 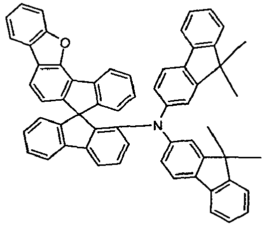 | 70% |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (z.B. Materialien) angepasst wird.

In den folgenden Device-Beispielen E1 bis E13 und E16 bis E18 (erfinderische Beispiele) und V1 bis V4 (Vergleichsbeispiele) werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht (HIL1) / Lochtransportschicht (HTL) / p-dotierte Lochtransportschicht (HIL2) / Lochtransportschicht (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet.

Die zur Herstellung der OLEDs benötigten Materialien sind in Tabelle 1 gezeigt, die verschiedenen Bauteilaufbauten in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB(5%) bedeutet hierbei, dass das Material H1 in einem Volumenanteil von 95 % und SEB in einem Anteil von 5 % in der Schicht vorliegt. Analog kann auch die Elektronentransportschicht oder die Lochinjektionsschichten aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 10 mA/cm² bezeichnet die externe Quanteneffizienz bei einer Stromdichte von 10mA/cm². LT80 @ 60 mA/cm² ist die Lebensdauer bis das OLED bei einer Starthelligkeit bei konstantem Strom von 60 mA/cm² auf 80 % der Anfangsintensität abgefallen ist.

| **Tabelle 1: Strukturen der verwendeten Materialien** | | |
|---|---|---|
| 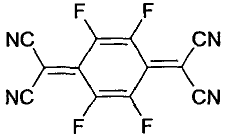 | 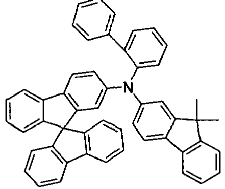 | 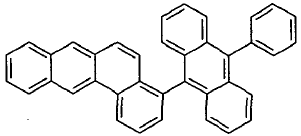 |
| F4TCNQ | HIM | H1 |
| 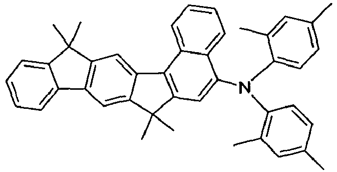 | 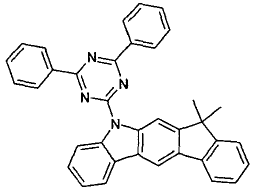 | 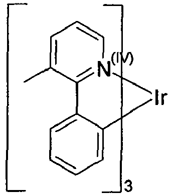 |
| SEB | H2 | TEG |
| 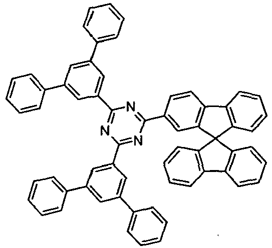 | 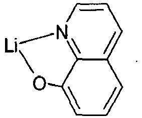 | 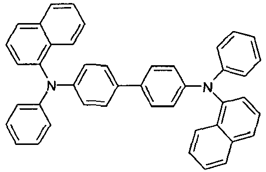 |
| ETM | LiQ | NPB |
| 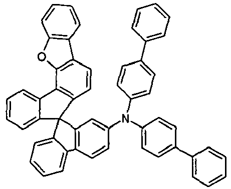 | 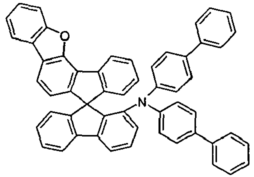 | 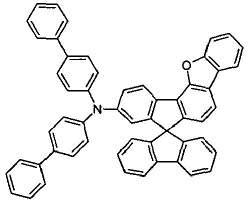 |
| HTMV1 | HTM1 | HTM2 |
| 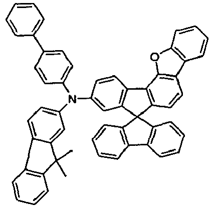 | 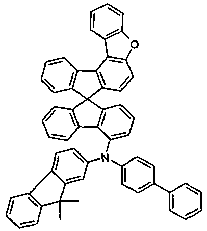 | 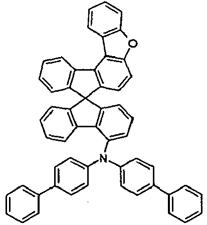 |
| HTM3 | HTM4 | HTM5 |
| 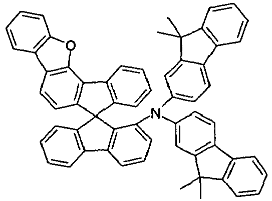 | 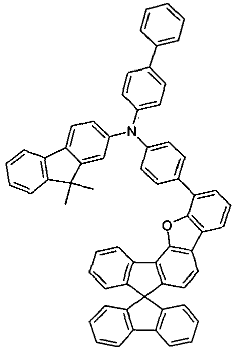 | 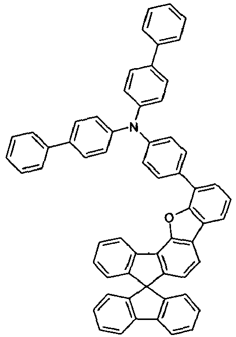 |
| HTM8 | HTM9 | HTM10 |

| **Tabelle 2: Aufbau der OLEDs** | | | | | | | |
|---|---|---|---|---|---|---|---|
| ***Exp.*** | ***HIL1*** | ***HTL*** | ***HIL2*** | ***EBL*** | ***EML*** | ***ETL*** | ***EIL*** |
| | *Dicke* / *nm* | *Dicke* / *nm* | *Dicke* / *nm* | *Dicke* / *nm* | *Dicke* / *nm* | *Dicke* / *nm* | *Dicke* / *nm* |
| *V1* | *HIM:F4TCNQ(5%) 20 nm* | *HIM 155 nm* | *NPB:F4TCNQ(5%) 20 nm* | *NPB 20 nm* | *H1:SEB(5%) 20 nm* | *ETM:LiQ(50%) 30 nm* | *LiQ 1 nm* |
| *V2* | *HIM:F4TCNQ(5%) 20 nm* | *HIM 155 nm* | *HTMV1:F4TCNQ(5%) 20 nm* | *HTMV1 20 nm* | *H1:SEB1(5%) 20 nm* | *ETM:LiQ(50%) 30 nm* | *LiQ 1 nm* |
| *E1* | *HIM:F4TCNQ(5%) 20 nm* | *HIM 155 nm* | *HTM1:F4TCNQ(5%) 20 nm* | *HTM1 20 nm* | *H1:SEB1(5%) 20 nm* | *ETM:LiQ(50%) 30 nm* | *LiQ 1 nm* |
| *E2* | *HIM:F4TCNQ(5%) 20 nm* | *HIM 155 nm* | *HTM2:F4TCNQ(5%) 20 nm* | *HTM2 20 nm* | *H1:SEB1(5%) 20 nm* | *ETM:LiQ(50%) 30 nm* | *LiQ 1 nm* |
| *E3* | *HIM:F4TCNQ(5%) 20 nm* | *HIM 155 nm* | *HTM3:F4TCNQ(5%) 20 nm* | *HTM3 20 nm* | *H1:SEB1(5%) 20 nm* | *ETM:LiQ(50%) 30 nm* | *LiQ 1 nm* |
| *E4* | *HIM:F4TCNQ(5%) 20 nm* | *HIM 155 nm* | *HTM4:F4TCNQ(5%) 20 nm* | *HTM4 20 nm* | *H1:SEB1(5%) 20 nm* | *ETM:LiQ(50%) 30 nm* | *LiQ 1 nm* |
| *E5* | *HIM:F4TCNQ(5%) 20 nm* | *HIM 155 nm* | *HTM5:F4TCNQ(5%) 20 nm* | *HTM5 20 nm* | *H1:SEB1(5%) 20 nm* | *ETM:LiQ(50%) 30 nm* | *LiQ 1 nm* |
| *E6* | *HIM:F4TCNQ(5%) 20 nm* | *HIM 155 nm* | *HTM8:F4TCNQ(5%) 20 nm* | *HTM8 20 nm* | *H1:SEB1(5%) 20 nm* | *ETM:LiQ(50%) 30 nm* | *LiQ 1 nm* |
| *E7* | *HIM:F4TCNQ(5%) 20 nm* | *HIM 155 nm* | *HTM9:F4TCNQ(5%) 20 nm* | *HTM9 20 nm* | *H1:SEB1(5%) 20 nm* | *ETM:LiQ(50%) 30 nm* | *LiQ 1 nm* |
| *E8* | *HIM:F4TCNQ(5%) 20 nm* | *HIM 155 nm* | *HTM10:F4TCNQ(5%) 20 nm* | *HTM10 20 nm* | *H1:SEB1(5%) 20 nm* | *ETM:LiQ(50%) 30 nm* | *LiQ 1 nm* |
| *V3* | *HIM:F4TCNQ(5%) 20 nm* | *HIM 210 nm* | *NPB:F4TCNQ(5%) 20 nm* | *NPB 20 nm* | *H2:TEG(10%) 30 nm* | *ETM:LiQ(50%) 40 nm* | *LiQ 1 nm* |
| *V4* | *HIM:F4TCNQ(5%) 20 nm* | *HIM 210 nm* | *HTMV1:F4TCNQ(5%) 20 nm* | *HTMV1 20 nm* | *H2:TEG(10%) 30 nm* | *ETM:LiQ(50%) 40 nm* | *LiQ 1 nm* |
| *E9* | *HIM:F4TCNQ(5%) 20 nm* | *HIM 210 nm* | *HTM1:F4TCNQ(5%) 20 nm* | *HTM1 20 nm* | *H2:TEG(10%) 30 nm* | *ETM:LiQ(50%) 40 nm* | *LiQ 1 nm* |
| *E10* | *HIM:F4TCNQ(5%) 20 nm* | *HIM 210 nm* | *HTM2:F4TCNQ(5%) 20 nm* | *HTM2 20 nm* | *H2:TEG(10%) 30 nm* | *ETM:LiQ(50%) 40 nm* | *LiQ 1 nm* |
| *E11* | *HIM:F4TCNQ(5%) 20 nm* | *HIM 210 nm* | *HTM3:F4TCNQ(5%) 20 nm* | *HTM3 20 nm* | *H2:TEG(10%) 30 nm* | *ETM:LiQ(50%) 40 nm* | *LiQ 1 nm* |
| *E12* | *HIM:F4TCNQ(5%) 20 nm* | *HIM 210 nm* | *HTM4:F4TCNQ(5%) 20 nm* | *HTM4 20 nm* | *H2:TEG(10%) 30 nm* | *ETM:LiQ(50%) 40 nm* | *LiQ 1 nm* |
| *E13* | *HIM:F4TCNQ(5%) 20 nm* | *HIM 210 nm* | *HTM5:F4TCNQ(5%) 20 nm* | *HTM5 20 nm* | *H2:TEG(10%) 30 nm* | *ETM:LiQ(50%) 40 nm* | *LiQ 1 nm* |
| *E16* | *HIM:F4TCNQ(5%) 20 nm* | *HIM 210 nm* | *HTM8:F4TCNQ(5%) 20 nm* | *HTM8 20 nm* | *H2:TEG(10%) 30 nm* | *ETM:LiQ(50%) 40 nm* | *LiQ 1 nm* |
| *E17* | *HIM:F4TCNQ(5%) 20 nm* | *HIM 210 nm* | *HTM9:F4TCNQ(5%) 20 nm* | *HTM9 20 nm* | *H2:TEG(10%) 30 nm* | *ETM:LiQ(50%) 40 nm* | *LiQ 1 nm* |
| *E18* | *HIM:F4TCNQ(5%) 20 nm* | *HIM 210 nm* | *HTM10:F4TCNQ(5%) 20 nm* | *HTM10 20 nm* | *H2:TEG(10%) 30 nm* | *ETM:LiQ(50%) 40 nm* | *LiQ 1 nm* |

Die erfindungsgemäßen Verbindungen HTM1 bis HTM5 und HTM8 bis HTM10 eignen sich sehr gut zur Verwendung als OLED-Materialien, wie die Beispiele E1 bis E13 und E16 bis E18 zeigen (E1 bis E8: Singulett-Bauteile; E9 bis E13 und E16 bis E18: Triplett-Bauteile). Mit den Verbindungen werden verbesserte Leistungsdaten der OLEDs verglichen mit den Referenzverbindungen HTMV1 und NPB erhalten (Vergleichsbeispiele V1 und V2: Singulett-Bauteile; V3 und V4: Triplett-Bauteile).

In einem Singulett-Blau-Bauteil zeigen im Vergleich zu den Referenzproben V1 und V2 (6,2 % und 7,7 %) die erfindungsgemäßen Proben E2 (7,9 %), E3 (8,6 %), E4 (7,9 %) und E5 (8,3 %) höhere Quanteneffizienzen bei 10 mA/cm². Die Lebensdauer LT80 bei 60 mA/cm² ist bei den erfindungsgemäßen Proben E1 (356 h), E2 (312 h), E4 (403 h), E6 (275 h), E7 (316 h) und E8 (408 h) auch deutlich besser als bei den Referenzproben V1 (125 h) und V2 (257 h).

In einem Triplett-Grün-Bauteil zeigen die Referenzproben V3 (11,7 %) und V4 (18,6 %) niedrigere oder gleiche Quanteneffizienzen bei 2 mA/cm² als die erfindungsgemäßen Proben E10 (18,6 %), E12 (19,8 %), E16 (19,6 %), E17 (19,4 %) und E18 (18,6 %). Auch sind die Lebensdauern (80%) bei 20 mA/cm² der erfindungsgemäßen Proben E9 (220 h), E10 (96 h), E11 (109 h), E12 (172 h), E13 (111 h), E16 (150 h), E17 (144 h) und E18 (160 h) größer als bei den Referenzproben V3 (80 h) und V4 (84 h).

## Patentansprüche

1. Verbindung gemäß einer der Formeln (I-1) bis (I-6) 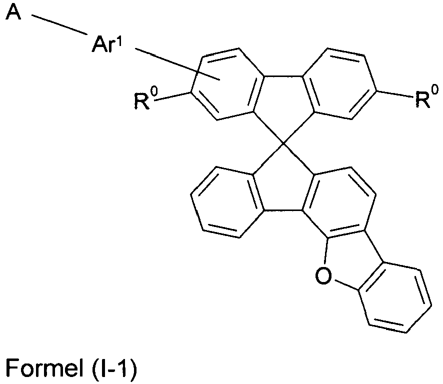 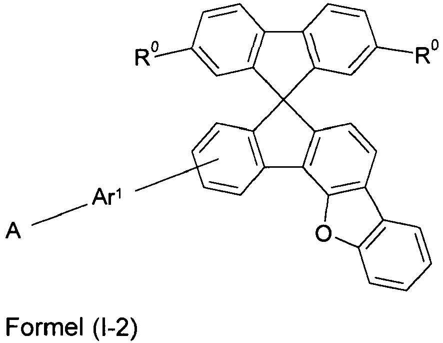 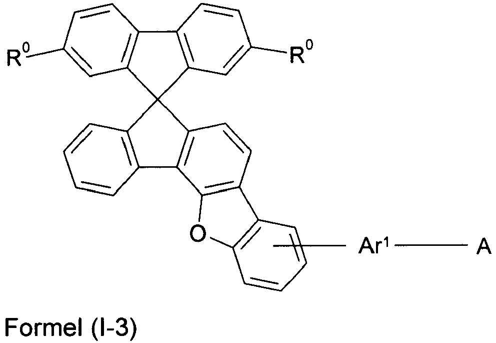 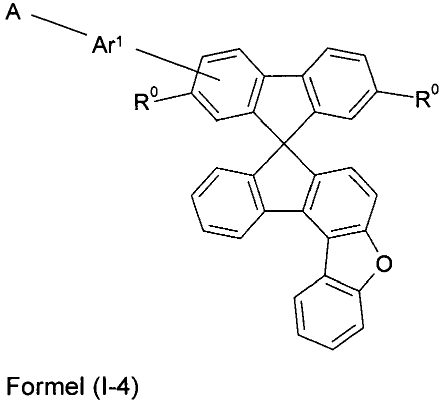 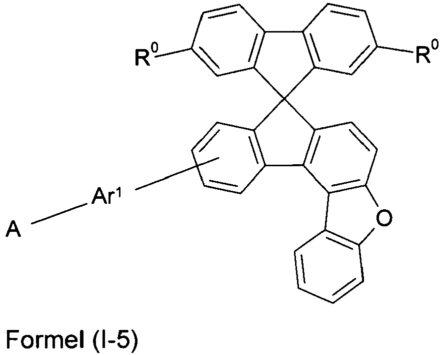 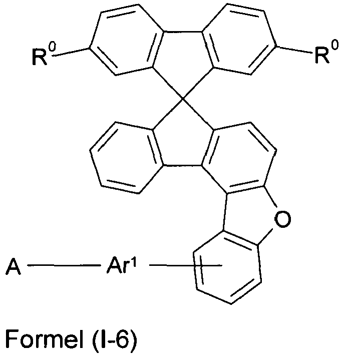
- die an einer oder mehreren unsubstituiert dargestellten Positionen an der Grundstruktur gemäß einer der Formeln (I-1) bis (I-6) mit einem Rest R¹ substituiert sein kann; und
- die die folgenden Definitionen der Variablen aufweist:
A ist bei jedem Auftreten gleich oder verschieden eine Gruppe der Formel (A1), (A2) oder (A3), die über die mit # markierte Bindung gebunden ist; 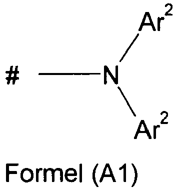 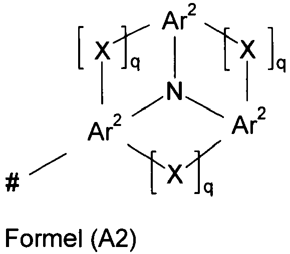 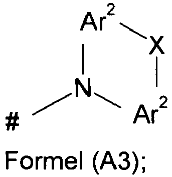
Ar¹ ist bei jedem Auftreten gleich oder verschieden eine Einfachbindung oder ein aromatisches oder heteroaromatisches Ringsystem mit 6 bis 30 aromatischen Ringatomen, das mit einem oder mehreren Resten R² substituiert sein kann;
Ar² ist bei jedem Auftreten gleich oder verschieden ein aromatisches oder heteroaromatisches Ringsystem mit 6 bis 30 aromatischen Ringatomen, das mit einem oder mehreren Resten R² substituiert sein kann;
X ist bei jedem Auftreten gleich oder verschieden eine Einfachbindung oder eine Gruppe gewählt aus BR², C(R²)₂, Si(R²)₂, C=O, O, S, S=O, SO₂, NR², PR² oder P(=O)R²;
R⁰ ist bei jedem Auftreten gleich oder verschieden H, D, F, CN, Si(R³)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch-R³C=CR³⁻, -C≡C-, Si(R³)₂, C=O, C=NR³, -C(=O)O-, -C(=O)NR³-, NR³, P(=O)(R³), -O-, -S-, SO oder SO₂ ersetzt sein können;
R¹, R² ist bei jedem Auftreten gleich oder verschieden H, D, F, C(=O)R³, CN, Si(R³)₃, N(Ar³)₂, N(R³)₂, P(=O)(R³)₂, OR³, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können, oder ein aromatisches oder heteroaromatisches Ringsystem mit 5 bis 30 aromatischen Ringatomen, das mit einem oder mehreren Resten R³ substituiert sein kann; zwei oder mehr Reste R¹ bzw. R² können miteinander verknüpft sein und einen Ring bilden;
Ar³ ist bei jedem Auftreten gleich oder verschieden ein aromatisches oder heteroaromatisches Ringsystem mit 5 bis 30 aromatischen Ringatomen, das mit einem oder mehreren Resten R³ substituiert sein kann;
R³ ist bei jedem Auftreten gleich oder verschieden H, D, F, C(=O)R⁴, CN, Si(R⁴)₃, N(Ar³)₂, N(R⁴)₂, P(=O)(R⁴)₂, OR⁴, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das mit einem oder mehreren Resten R⁴ substituiert sein kann; zwei oder mehr Reste R³ können miteinander verknüpft sein und einen Ring bilden;
R⁴ ist bei jedem Auftreten gleich oder verschieden H, D, F, CN oder ein aliphatischer, aromatischer oder heteroaromatischer organischer Rest mit 1 bis 20 C-Atomen, in dem auch ein oder mehrere H-Atome durch D, F oder CN ersetzt sein können; zwei oder mehr Substituenten R⁴ können miteinander verknüpft sein und einen Ring bilden;
q ist bei jedem Auftreten gleich oder verschieden 0 oder 1, wobei mindestens ein q in Formel (A2) gleich 1 ist.

2. Verbindung nach Anspruch 1, **dadurch gekennzeichnet, dass** A eine Gruppe der Formel (A-1) ist.

3. Verbindung nach Anspruch 1, **dadurch gekennzeichnet, dass** X bei jedem Auftreten gleich oder verschieden gewählt ist aus einer Einfachbindung oder einer Gruppe gewählt aus C(R²)₂, C=O, O, S und NR².

4. Verbindung nach Anspruch 1, **dadurch gekennzeichnet, dass** X eine Einfachbindung ist.

5. Verbindung nach einem oder mehreren der Ansprüche 1 bis 4, **dadurch gekennzeichnet, dass** R⁰ bei jedem Auftreten gleich oder verschieden H, D, F, eine geradkettige Alkyl- oder Alkoxygruppe mit 1 bis 10 C-Atomen oder eine verzweigte oder cyclische Alkyl- oder Alkoxygruppe mit 3 bis 10 C-Atomen ist, wobei die oben genannten Gruppen jeweils mit einem oder mehreren Resten R³ substituiert sein können.

6. Verbindung nach einem oder mehreren der Ansprüche 1 bis 5, **dadurch gekennzeichnet, dass** R¹ und R² bei jedem Auftreten gleich oder verschieden sind H, D, F, CN, eine geradkettige Alkylgruppe mit 1 bis 10 C-Atomen oder eine verzweigte oder cyclische Alkylgruppe mit 3 bis 10 C-Atomen, wobei die oben genannten Gruppen jeweils mit einem oder mehreren Resten R³ substituiert sein können, oder ein aromatisches oder heteroaromatisches Ringsystem mit 6 bis 25 aromatischen Ringatomen, das jeweils mit einem oder mehreren Resten R³ substituiert sein kann.

7. Verbindung gemäß einer der Formeln (II-5) bis (II-6) 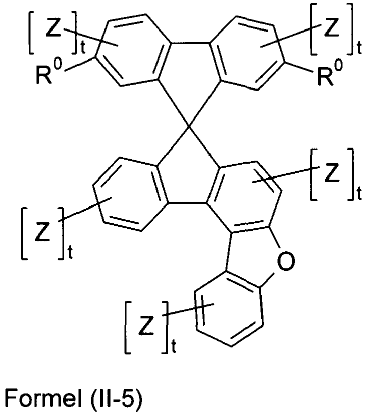 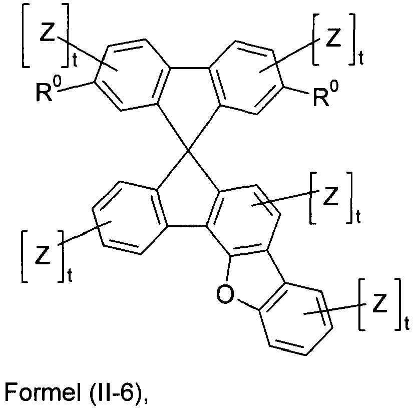 welche an einem oder mehreren freien Positionen mit einem Rest R¹ substituiert sein können, der gemäß Anspruch 1 oder 6 definiert ist, und wobei R⁰ gemäß Anspruch 1 oder 5 definiert ist und die weiteren Variablen wie folgt definiert sind:
Z ist bei jedem Auftreten gleich oder verschieden gewählt aus F, Cl, Br, I, B(OR³)₂, OSO₂R³, S(=O)R³ und S(=O)₂R³;
t ist bei jedem Auftreten gleich oder verschieden 0 oder 1, wobei mindestens ein Index t pro Formel gleich 1 ist.

8. Verfahren zur Herstellung einer Verbindung nach einem oder mehreren der Ansprüche 1 bis 6, **dadurch gekennzeichnet, dass** zunächst das Spirobifluoren-Grundgerüst hergestellt wird, und in einem späteren Schritt über eine metallorganische Kupplungsreaktion eine Arylamino- oder eine Carbazolgruppe oder eine Aryl- oder Heteroarylgruppe, die mit einer Arylamino- oder Carbazolgruppe substituiert ist, eingeführt wird.

9. Oligomere, Polymere oder Dendrimere enthaltend eine oder mehrere Verbindungen nach einem oder mehreren der Ansprüche 1 bis 6, wobei die Bindung(en) zum Polymer, Oligomer oder Dendrimer an beliebigen, in den Formeln (I-1) bis I(-6) mit R⁰, R¹ oder R² substituierten Positionen lokalisiert sein können.

10. Formulierung, enthaltend mindestens eine Verbindung nach einem oder mehreren der Ansprüche 1 bis 6 sowie mindestens ein Lösungsmittel.

11. Verwendung einer Verbindung nach einem oder mehreren der Ansprüche 1 bis 6 in einer elektronischen Vorrichtung.

12. Elektronische Vorrichtung, enthaltend mindestens eine Verbindung nach einem oder mehreren der Ansprüche 1 bis 6.

13. Elektronische Vorrichtung nach Anspruch 12, **dadurch gekennzeichnet, dass** sie ausgewählt ist aus der Gruppe bestehend aus organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, organischen lichtemittierenden elektrochemischen Zellen, organischen Laserdioden und organischen Elektrolumineszenzvorrichtungen, enthaltend mindestens eine Verbindung nach einem oder mehreren der Ansprüche 1 bis 6.

14. Organische Elektrolumineszenzvorrichtung nach Anspruch 13, **dadurch gekennzeichnet, dass** die Verbindung nach einem oder mehreren der Ansprüche 1 bis 6 als Lochtransportmaterial oder als Matrixmaterial der emittierenden Schicht vorliegt.

## Claims

1. Compound of one of the formulae (I-1) to (I-6) 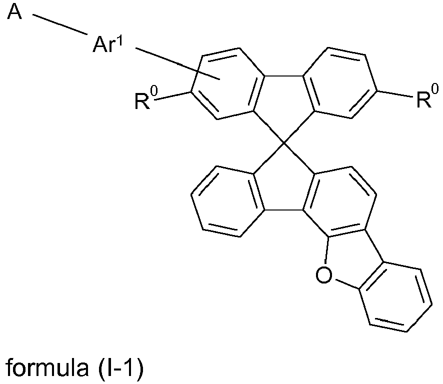 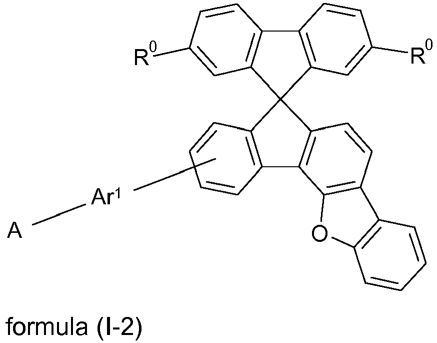 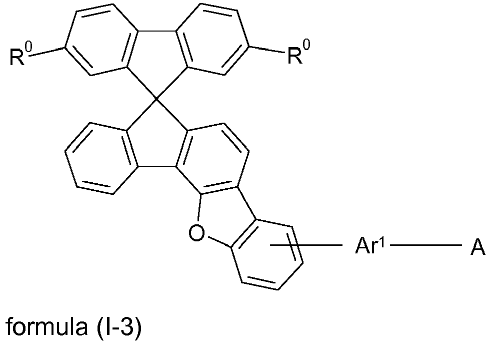 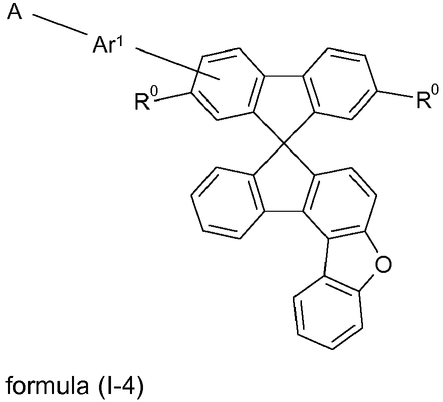 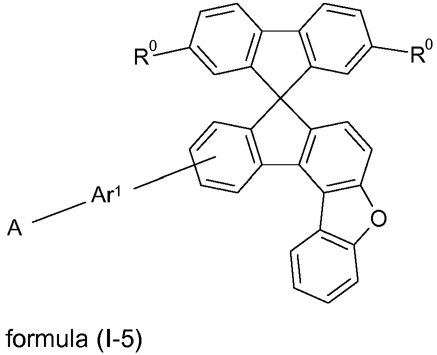 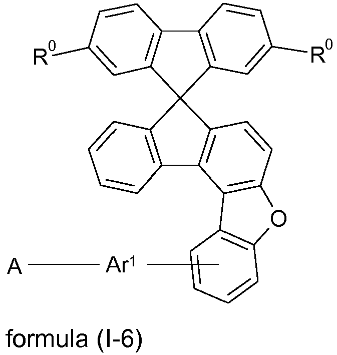
- which may be substituted by a radical R¹ at one or more positions shown as unsubstituted on the basic structure of one of the formulae (I-1) to (I-6); and
- which has the following definitions of the variables:
A is on each occurrence, identically or differently, a group of the formula (A1), (A2) or (A3), which is bonded via the bond marked with #; 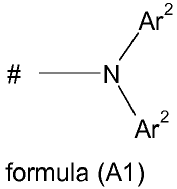 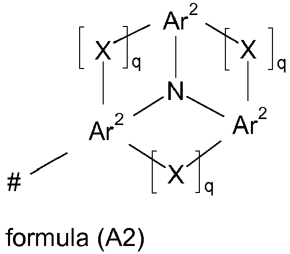 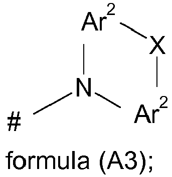
Ar¹ is on each occurrence, identically or differently, a single bond or an aromatic or heteroaromatic ring system having 6 to 30 aromatic ring atoms, which may be substituted by one or more radicals R²;
Ar² is on each occurrence, identically or differently, an aromatic or heteroaromatic ring system having 6 to 30 aromatic ring atoms, which may be substituted by one or more radicals R²;
X is on each occurrence, identically or differently, a single bond or a group selected from BR², C(R²)₂, Si(R²)₂, C=O, O, S, S=O, SO₂, NR², PR² or P(=O)R²;
R⁰ is on each occurrence, identically or differently, H, D, F, CN, Si(R³)₃, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂;
R¹, R² are on each occurrence, identically or differently, H, D, F, C(=O)R³, CN, Si(R³)₃, N(Ar³)₂, N(R³)₂, P(=O)(R³)₂, OR³, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂, or an aromatic or heteroaromatic ring system having 5 to 30 aromatic ring atoms, which may be substituted by one or more radicals R³; two or more radicals R¹ or R² may be linked to one another and form a ring;
Ar³ is on each occurrence, identically or differently, an aromatic or heteroaromatic ring system having 5 to 30 aromatic ring atoms, which may be substituted by one or more radicals R³;
R³ is on each occurrence, identically or differently, H, D, F, C(=O)R⁴, CN, Si(R⁴)₃, N(Ar³)₂, N(R⁴)₂, P(=O)(R⁴)₂, OR⁴, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂, or an aromatic or heteroaromatic ring system having 5 to 30 aromatic ring atoms, which may be substituted by one or more radicals R⁴; two or more radicals R³ may be linked to one another and form a ring;
R⁴ is on each occurrence, identically or differently, H, D, F, CN or an aliphatic, aromatic or heteroaromatic organic radical having 1 to 20 C atoms, in which, in addition, one or more H atoms may be replaced by D, F or CN; two or more substituents R⁴ may be linked to one another and form a ring;
q is on each occurrence, identically or differently, 0 or 1, where at least one q in formula (A2) is equal to 1.

2. Compound according to Claim 1, **characterised in that** A is a group of the formula (A-1) ist.

3. Compound according to Claim 1, **characterised in that** X is selected on each occurrence, identically or differently, from a single bond or a group selected from C(R²)₂, C=O, O, S and NR².

4. Compound according to Claim 1, **characterised in that** X is a single bond.

5. Compound according to one or more of Claims 1 to 4, **characterised in that** R⁰ is on each occurrence, identically or differently, H, D, F, a straight-chain alkyl or alkoxy group having 1 to 10 C atoms or a branched or cyclic alkyl or alkoxy group having 3 to 10 C atoms, where the above-mentioned groups may each be substituted by one or more radicals R³.

6. Compound according to one or more of Claims 1 to 5, **characterised in that** R¹ and R² are on each occurrence, identically or differently, H, D, F, CN, a straight-chain alkyl group having 1 to 10 C atoms or a branched or cyclic alkyl group having 3 to 10 C atoms, where the above-mentioned groups may each be substituted by one or more radicals R³, or an aromatic or heteroaromatic ring system having 6 to 25 aromatic ring atoms, which may in each case be substituted by one or more radicals R³.

7. Compound of one of the formulae (II-5) to (II-6) 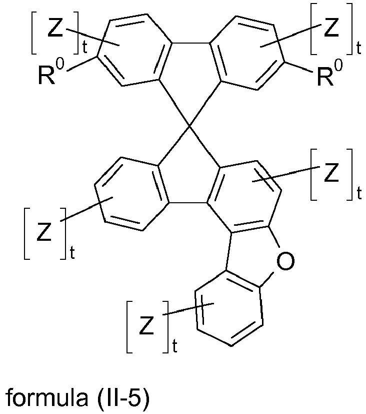 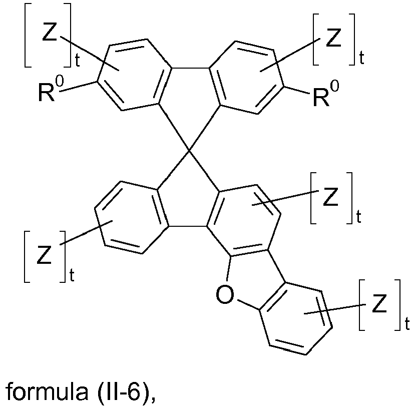 which may be substituted at one or more free positions by a radical R¹, which is defined in accordance with Claim 1 or 6, and where R⁰ is defined in accordance with Claim 1 or 5 and the other variables are defined as follows:
Z is selected on each occurrence, identically or differently, from F, Cl, Br, I, B(OR³)₂, OSO₂R³, S(=O)R³ and S(=O)₂R³;
t is on each occurrence, identically or differently, 0 or 1, where at least one index t per formula is equal to 1.

8. Process for the preparation of a compound according to one or more of Claims 1 to 6, **characterised in that** firstly the spirobifluorene basic structure is prepared, and, in a later step, an arylamino or carbazole group or an aryl or heteroaryl group which is substituted by an arylamino or carbazole group is introduced via an organometallic coupling reaction.

9. Oligomers, polymers or dendrimers containing one or more compounds according to one or more of Claims 1 to 6, where the bond(s) to the polymer, oligomer or dendrimer may be localised at any desired positions in the formulae (I-1) to (I-6) that are substituted by R⁰, R¹ or R².

10. Formulation comprising at least one compound according to one or more of Claims 1 to 6 and at least one solvent.

11. Use of a compound according to one or more of Claims 1 to 6 in an electronic device.

12. Electronic device comprising at least one compound according to one or more of Claims 1 to 6.

13. Electronic device according to Claim 12, **characterised in that** it is selected from the group consisting of organic integrated circuits, organic field-effect transistors, organic thin-film transistors, organic light-emitting transistors, organic solar cells, organic optical detectors, organic photoreceptors, organic field-quench devices, organic light-emitting electrochemical cells, organic laser diodes and organic electroluminescent devices comprising at least one compound according to one or more of Claims 1 to 6.

14. Organic electroluminescent device according to Claim 13, **characterised in that** the compound according to one or more of Claims 1 to 6 is present as hole-transport material or as matrix material of the emitting layer.

## Revendications

1. Composé de l'une des formules (I-1) à (I-6) 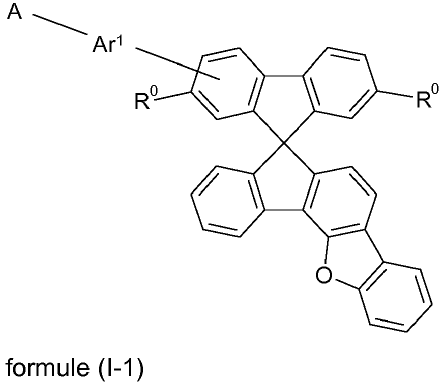 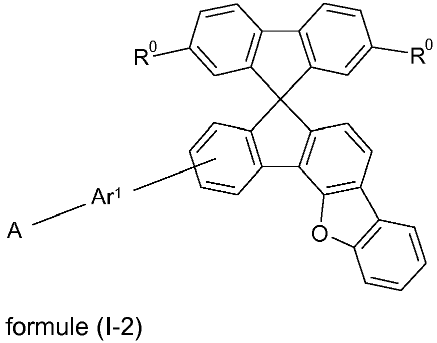 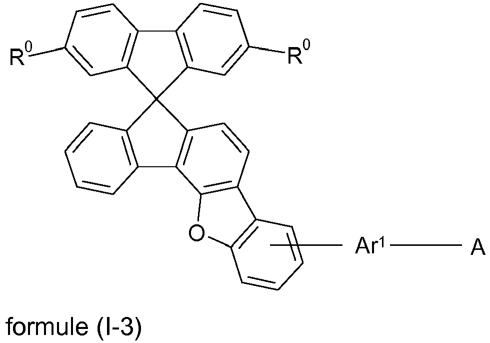 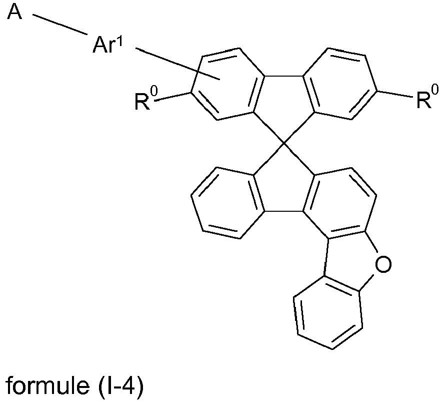 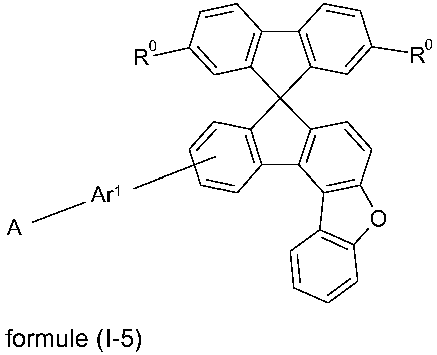 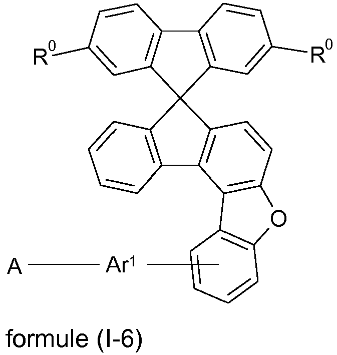
- lequel peut être substitué par un radical R¹ au niveau d'une ou de plusieurs position(s) qui est/sont représentée(s) comme étant non substituée(s) au niveau de la structure de base de l'une des formules (I-1) à (I-6) ; et
- lequel présente les définitions qui suivent pour les variables :
A est pour chaque occurrence, de manière identique ou différente, un groupe de la formule (A1), (A2) ou (A3), lequel est lié via le lien qui est indiqué à l'aide de # ; 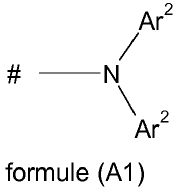 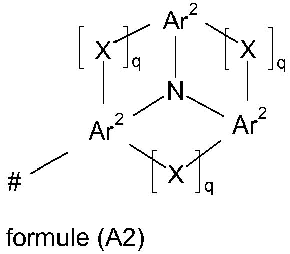 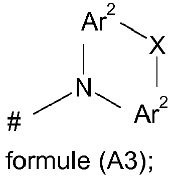
Ar¹ est pour chaque occurrence, de manière identique ou différente, une liaison simple ou un système de cycle aromatique ou hétéroaromatique qui comporte de 6 à 30 atomes de cycle aromatique, lequel peut être substitué par un radical ou par plusieurs radicaux R²;
Ar² est pour chaque occurrence, de manière identique ou différente, un système de cycle aromatique ou hétéroaromatique qui comporte de 6 à 30 atomes de cycle aromatique, lequel peut être substitué par un radical ou par plusieurs radicaux R²;
X est pour chaque occurrence, de manière identique ou différente, une liaison simple ou un groupe qui est sélectionné parmi BR², C(R²)₂, Si(R²)₂, C=O, O, S, S=O, SO₂, NR², PR² ou P(=O)R² ;
R⁰ est pour chaque occurrence, de manière identique ou différente, H, D, F, CN, Si(R³)₃, un groupe alkyle ou alcoxy en chaîne droite qui comporte de 1 à 20 atome(s) de C ou un groupe alkyle ou alcoxy ramifié qui comporte de 3 à 20 atomes de C ou un groupe alkényle ou alkynyle qui comporte de 2 à 20 atomes de C, dans lequel les groupes qui ont été mentionnés ci-avant peuvent chacun être substitués par un radical ou par plusieurs radicaux R³ et dans lequel un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂ ;
R¹, R² sont pour chaque occurrence, de manière identique ou différente, H, D, F, C(=O)R³, CN, Si(R³)₃, N(Ar³)₂, N(R³)₂, P(=O)(R³)₂, OR³, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, dans lequel les groupes qui ont été mentionnés ci-avant peuvent chacun être substitués par un radical ou par plusieurs radicaux R³ et dans lequel un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂, ou un système de cycle aromatique ou hétéroaromatique qui comporte de 5 à 30 atomes de cycle aromatique, lequel peut être substitué par un radical ou par plusieurs radicaux R³; deux radicaux R¹ ou R² ou plus peuvent être liés l'un à l'autre ou les uns aux autres et peuvent former un cycle ;
Ar³ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R³ ;
R³ est pour chaque occurrence, de manière identique ou différente, H, D, F, C(=O)R⁴, CN, Si(R⁴)₃, N(Ar³)₂, N(R⁴)₂, P(=O)(R⁴)₂, OR⁴, S(=O)R⁴, S(=O)₂R⁴, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, dans lequel les groupes qui ont été mentionnés ci-avant peuvent chacun être substitués par un radical ou par plusieurs radicaux R⁴ et dans lequel un ou plusieurs groupe(s) CH₂ dans les groupes qui ont été mentionnés ci-avant peut/peuvent être remplacé(s) par -R⁴C=CR⁴-, -C≡C-, Si(R⁴)₂, C=O, C=NR⁴, -C(=O)O-, -C(=O)NR⁴- , NR⁴, P(=O)(R⁴), -O-, -S-, SO ou SO₂, ou un système de cycle aromatique ou hétéroaromatique qui comporte de 5 à 30 atomes de cycle aromatique, lequel peut être substitué par un radical ou par plusieurs radicaux R⁴; deux radicaux R³ ou plus peuvent être liés l'un à l'autre ou les uns aux autres et peuvent former un cycle ;
R⁴ est pour chaque occurrence, de manière identique ou différente, H, D, F, CN ou un radical organique aliphatique, aromatique ou hétéroaromatique qui comporte de 1 à 20 atome(s) de C, dans lequel, en outre, un ou plusieurs atome(s) de H peut/peuvent être remplacé(s) par D, F ou CN ; deux substituants R⁴ ou plus peuvent être liés l'un à l'autre ou les uns aux autres et former un cycle ;
q est pour chaque occurrence, de manière identique ou différente, 0 ou 1, où au moins un q dans la formule (A2) est égal à 1.

2. Composé selon la revendication 1, **caractérisé en ce que** A est un groupe de la formule (A-1).

3. Composé selon la revendication 1, **caractérisé en ce que** X est sélectionné pour chaque occurrence, de manière identique ou différente, parmi une liaison simple ou un groupe qui est sélectionné parmi C(R²)₂, C=O, O, S et NR².

4. Composé selon la revendication 1, **caractérisé en ce que** X est une liaison simple.

5. Composé selon une ou plusieurs des revendications 1 à 4, **caractérisé en ce que** R⁰ est pour chaque occurrence, de manière identique ou différente, H, D, F, un groupe alkyle ou alcoxy en chaîne droite qui comporte de 1 à 10 atome(s) de C ou un groupe alkyle ou alcoxy ramifié ou cyclique qui comporte de 3 à 10 atomes de C, dans lequel les groupes qui ont été mentionnés ci-avant peuvent chacun être substitués par un radical ou par plusieurs radicaux R³.

6. Composé selon une ou plusieurs des revendications 1 à 5, **caractérisé en ce que** R¹ et R² sont pour chaque occurrence, de manière identique ou différente, H, D, F, CN, un groupe alkyle en chaîne droite qui comporte de 1 à 10 atome(s) de C ou un groupe alkyle ramifié ou cyclique qui comporte de 3 à 10 atomes de C, dans lequel les groupe qui ont été mentionnés ci-avant peuvent chacun être substitués par un radical ou par plusieurs radicaux R³, ou un système de cycle aromatique ou hétéroaromatique qui comporte de 6 à 25 atomes de cycle aromatique, lequel peut dans chaque cas être substitué par un radical ou par plusieurs radicaux R³.

7. Composé de l'une des formules (II-5) à (II-6) : 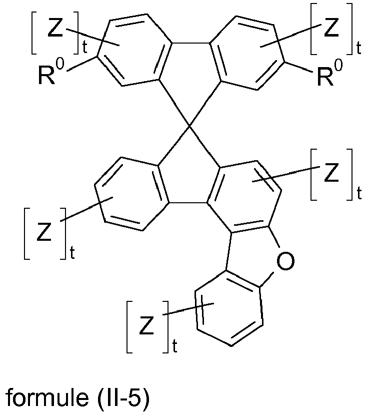 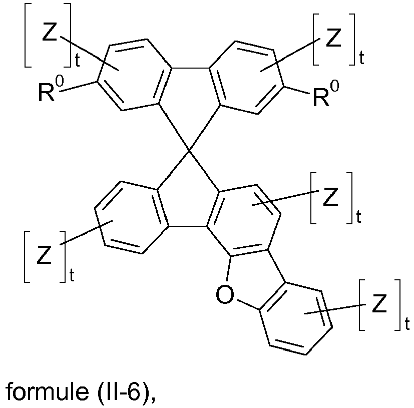 lequel peut être substitué au niveau d'une ou de plusieurs position(s) libre(s) par un radical R¹, lequel est défini conformément à la revendication 1 ou 6, et dans lequel R⁰ est défini conformément à la revendication 1 ou 5 et les autres variables sont définies comme suit :
Z est sélectionné pour chaque occurrence, de manière identique ou différente, parmi F, Cl, Br, I, B(OR³)₂, OSO₂R³, S(=O)R³ et S(=O)₂R³ ;
t est pour chaque occurrence, de manière identique ou différente, 0 ou 1, dans lequel au moins un indice t par formule est égal à 1.

8. Procédé pour la préparation d'un composé selon une ou plusieurs des revendications 1 à 6, **caractérisé en ce que**, en premier lieu, la structure de base spirobifluorène est préparée, et, au niveau d'une étape ultérieure, un groupe arylamino ou carbazole ou un groupe aryle ou hétéroaryle qui est substitué par un groupe arylamino ou carbazole est introduit via une réaction de couplage organométallique.

9. Oligomères, polymères ou dendrimères contenant un ou plusieurs composés selon une ou plusieurs des revendications 1 à 6, dans lesquels la/les liaison(s) sur le polymère, sur l'oligomère ou sur le dendrimère peut/peuvent être localisée(s) au niveau de n'importe quelles positions souhaitées dans les formules (I-1) à (I-6) qui sont substituées par R⁰, R¹ ou R².

10. Formulation comprenant au moins un composé selon une ou plusieurs des revendications 1 à 6 et au moins un solvant.

11. Utilisation d'un composé selon une ou plusieurs des revendications 1 à 6 dans un dispositif électronique.

12. Dispositif électronique comprenant au moins un composé selon une ou plusieurs des revendications 1 à 6.

13. Dispositif électronique selon la revendication 12, **caractérisé en ce qu'**il est sélectionné parmi le groupe qui est constitué par les circuits intégrés organiques, les transistors à effet de champ organiques, les transistors à film mince organiques, les transistors à émission de lumière/(électro)luminescents organiques, les cellules solaires organiques, les détecteurs optiques organiques, les photorécepteurs organiques, les dispositifs à extinction de champ organiques, les cellules électrochimiques à émission de lumière/(électro)luminescentes organiques, les diodes laser organiques et les dispositifs à émission de lumière/(électro)luminescents organiques qui comprennent au moins un composé conformément à une ou plusieurs des revendications 1 à 6.

14. Dispositif électroluminescent organique selon la revendication 13, **caractérisé en ce que** le composé selon une ou plusieurs des revendications 1 à 6 est présent en tant que matériau de transport de trous ou en tant que matériau de matrice de la couche d'émission.
